# FASCICULE DE BREVET EUROPEEN

(11) **EP 2 406 263 B1**
(45) Date de publication et mention de la délivrance du brevet: **25.09.2013**
(21) Numéro de dépôt: 10710681.7
(22) Date de dépôt: 11.03.2010
(51) Int. Cl.: C07D 487/04, A61K 31/53, A61P 35/00

(54) **DERIVÉS DE PYRAZOLO[1,5-A]-1,3,5-TRIAZINES, LEUR PREPARATION ET LEUR APPLICATION EN THÉRAPEUTIQUE**
PYRAZOLO[1,5-A]-1,3,5-TRIAZINDERIVATE, HERSTELLUNG DAVON UND THERAPEUTISCHE VERWENDUNG DAVON
PYRAZOLO[1,5-A]-1,3,5-TRIAZINE DERIVATIVES, PREPARATION THEREOF, AND THERAPEUTIC USE THEREOF

(30) Priorité: 11.03.2009 FR 0951521; 11.03.2009 US 159246 P
(43) Date de publication de la demande: 18.01.2012
(73) Titulaire: Centre National de la Recherche Scientifique (C.N.R.S.), 75794 Paris Cedex 16 (FR); Université Paris Descartes, 75270 Paris Cedex 06 (FR); Université Claude Bernard Lyon I, 69100 Villeurbanne (FR); INSERM - Institut National de la Santé et de la Recherche Médicale, 75013 Paris (FR)
(72) Inventeur: MEIJER, Laurent, F-29680 Roscoff (FR); GALONS, Hervé, F-75014 Paris (FR); JOSEPH, Benoït, F-69100 Villeurbanne (FR); POPOWYCZ, Florence, F-69100 Villeurbanne (FR); OUMATA, Nassima, F-75017 Paris (FR)
(74) Mandataire: Le Coupanec, Pascale A.M.P.
(86) Numéro de dépôt international: PCT/IB2010/051063
(87) Numéro de publication internationale: WO 2010/103486

(56) Documents cités:
- US-A1- 2005 187 219
- POPOWYCZ, FLORENCE ET AL: "Pyrazolo[1,5-a]-1,3,5-triazine as a Purine Bioisostere: Access to Potent Cyclin-Dependent Kinase Inhibitor (R)-Roscovitine Analogue" JOURNAL OF MEDICINAL CHEMISTRY , 52(3), 655-663 CODEN: JMCMAR; ISSN: 0022-2623, 2009, XP002548292 cité dans la demande

## Description

L'invention se rapporte à des dérivés de pyrazolo[1,5-*a*]-1,3,5-triazines, à leur préparation et à leur application en thérapeutique.

Un certain nombre de documents décrivent des composés dérivés de pyrazolo[1,5-a]-1,3,5-triazines.

Ainsi, du document WO 2005/082908 sont connus des composés pyrazolotriazines en tant qu'inhibiteurs de kinase.

Du document WO 2002/50079 sont connus des composés inhibiteurs de kinases dépendantes des cyclines (CDKs) et de la Glycogène Synthase Kinase-3 (GSK-3).

Enfin, le document WO 2004/011464 décrit entre autres des composés inhibiteurs des phosphodiestérases PDE2 et PDE4.

Par ailleurs, Bettayeb et al., « N-&-N, a new class of cell death-inducing kinase inhibitors derived from the purine roscovitine », Mol. Cancer Ther. 2008, 7, 2713 et « Pyrazolo[1,5-a]-1,3,5-triazine as a purine bioisostere : access to potent cyclin-dependent kinase inhibitor (R)-roscovitine analogue », J. Med. Chem. 2009, 52, 655, décrivent le potentiel antitumoral du composé nommé N-&-N1:

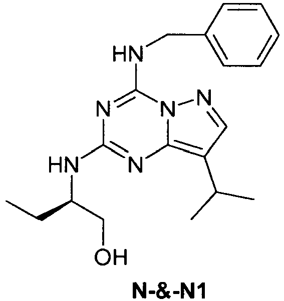

L'invention a pour objet des composés répondant à la formule (I) ci-après : dans laquelle : 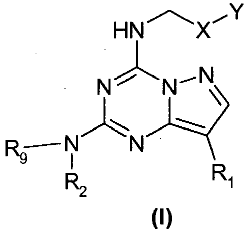 R₁ représente un groupe (C₁-C₆)alkyle ou un groupe (C₃-C₆)cycloalkyle,
R₂ représente un groupe (C₁-C₆)alkyle, un groupe (C₁-C₆)cycloalkyle, un groupe (C₁-C₆)alkényle, un groupe (C₁-C₆)fluoroalkyle, un groupe (C₁-C₃)fluoroalcoxy ou un groupe (C₁-C₆)alcoxy(C₁-C₆)alkyle, substitué (i) par un à trois groupes hydroxy, ou (ii) par un groupe NRₐR_{b}, où Rₐ et R_{b} représentent indépendamment un atome d'hydrogène ou un groupe (C₁-C₃)alkyle,
ou bien R₂ représente un groupe pyrrolidinylméthyle substitué par un à trois groupes hydroxy,
ledit groupe R₂ étant éventuellement substitué par un groupe -OCOR₃, dans lequel R₃ représente un dérivé d'acide aminé naturel ou non naturel ou un groupe pipéridyle de formule (**B**) dans laquelle : 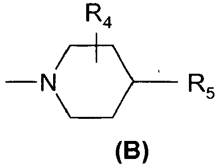 R₄ représente un hydrogène, un atome d'halogène, un groupe (C₁-C₃)alkyle, un groupe hydroxy(C₁-C₃)alkyle ou un groupe -NRₐR_{b}, où Rₐ et R_{b} sont tels que définis précédemment et R₅ représente un hydrogène, un groupe (C₁-C₃)alkyle, un groupe -N(Me)₂, un groupe pipéridyle ou un groupe morpholinyle,
R₉ prend la même signification que R₂ et peut également représenter un atome d'hydrogène,
alternativement R₂ et R₉ forment ensemble, avec l'atome d'azote qui les porte, un hétérocycle choisi parmi les groupes pyrrolidinyle, pipéridinyle, pipérazinyle, et pipéridinylpipéridinyle, ces hétérocycles pouvant être substitués par un à trois groupes choisis parmi hydroxy, (C₁-C₆)alkyle ou (C₁-C₆)alcoxy(C₁-C₆)alkyle, ces deux derniers groupes étant substitués (i) par un à trois groupes hydroxy, ou (ii) par un groupe NRₐR_{b}, où Rₐ et R_{b} représentent indépendamment un atome d'hydrogène ou un groupe (C₁-C₃)alkyle,
X et Y représentent indépendamment un groupe phényle ou un groupe hétéroaryle, lesdits groupes hétéroaryle et phényle pouvant être substitués par un ou deux groupes indépendamment choisis parmi un groupe (C₁-C₂)alkyle, un groupe (C₁-C₂)alcoxy, un atome d'halogène, un groupe (C₁-C₂)fluoroalkyle, un groupe (C₁-C₂)fluoroalcoxy, un groupe hydroxy, un groupe -COOH, un groupe -CONHR₆ et un groupe -NRₐR_{b}, où Rₐ et R_{b} sont tels que définis précédemment,
R₆ est un hydrogène ou un groupe (C₁-C₃)alkyle,
ledit groupe hétéroaryle étant choisi parmi un groupe thiényle, un groupe pyridyle, un groupe pyrimidinyle, un groupe thiazolyle, un groupe pyrrolyle et un groupe furanyle,
ainsi que ses sels pharmaceutiquement acceptables.

Ledit groupe dérivé d'acide aminé naturel ou non naturel peut dériver des 20 acides aminés naturels. Parmi lesdits acides aminés naturels, on peut en particulier citer la L-valine, la L-leucine, la L-sérine, la L-thréonine, la L-asparagine et l'acide aspartique.

Parmi les dérivés d'acide aminé non naturels, on peut citer les dérivés acylés des acides aminés naturels, à savoir comportant à la place du groupe -COOH terminal un groupe (C₁-C₃)alkylcarbonyle ou un groupe (C₁-C₄)alcoxycarbonyle, notamment un groupe méthylcarbonyle donnant un dérivé acétylé ou encore un groupe tert-butoxycarbonyle donnant un dérivé Boc.

Des dérivés d'acides aminés de configuration D peuvent aussi être utilisés.

Selon un aspect de l'invention, le groupe hétéroaryle est choisi parmi un groupe thiényle, un groupe furanyle et un groupe pyridyle.

Selon un aspect particulier de l'invention, R₃ représente une des formules (**a-1**) à (a-5) suivantes : 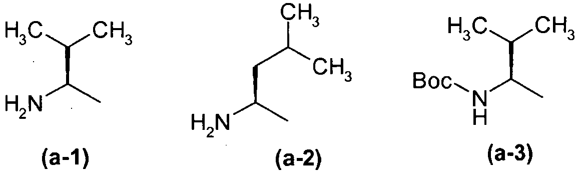 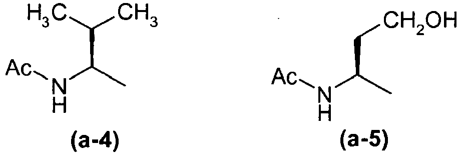 où Boc représente un groupe tert-butoxycarbonyle et Ac un groupe acétyle, ainsi que leurs énantiomères.

Les composés de formule (**I**) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (**I**) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (**I**) font également partie de l'invention.

Parmi les acides pharmaceutiquement acceptables on peut notamment citer l'acide chlorhydrique, l'acide bromhydrique, l'acide trifluoroacétique, l'acide fumarique, l'acide tartrique, l'acide citrique, l'acide ascorbique et l'acide maléique.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- (Cₜ-C_{z}) où t et z peuvent prendre les valeurs de 1 à 6, une chaîne carbonée pouvant avoir de t à z atomes de carbone, par exemple (C₁-C₃) une chaîne carbonée qui peut avoir de 1 à 3 atomes de carbone ;
- « halogène » un atome choisi parmi le fluor, le chlore, le brome et l'iode, notamment parmi le fluor, le chlore, le brome ;
- un groupe alkyle : un groupe aliphatique saturé linéaire ou ramifié. A titre d'exemples, on peut citer les groupes méthyle, éthyle, propyle, isopropyle, butyle, isobutyle, tert-butyle, pentyle, etc ;
- un groupe alkényle : un groupe aliphatique linéaire ou ramifié comprenant une ou deux insaturations. A titre d'exemple, on peut citer les groupes éthényle, propényle, isopropényle, butényle, isobutényle, tert-butényle, pentényle, etc.,
- un groupe cycloalkyle : un groupe alkyle cyclique. A titre d'exemples, on peut citer les groupes cyclopropyle, cyclobutyle, cyclopentyle, cyclohexyle, etc ;
- un groupe alcoxy : un groupe -O-alkyle où le groupe alkyle est tel que précédemment défini ;
- un groupe phényle, à la fois le groupe phényle (monovalent) et le groupe divalent correspondant phénylène ;
- un groupe hétéroaryle, à la fois le groupe hétéroaryle (monovalent) et le groupe divalent correspondant hétéroarylène, ledit groupe hétéroaryle représentant un groupe mono ou bicyclique, aromatique ou non, comprenant un ou deux hétéroatomes tels que l'azote, l'oxygène ou le soufre ;
- un groupe fluoroalkyle et un groupe fluoroalcoxy, respectivement un groupe alkyle et un groupe alcoxy, tels que définis précédemment, substitués par au moins un atome de fluor. A titre d'exemples, on peut citer les groupes perfluoroalkyles, tels le trifluorométhyle ou le perfluoropropyle.

Selon un mode de réalisation particulier, le groupe R₁ représente un groupe éthyle ou un groupe isopropyle.

Selon un mode de réalisation particulier, les groupes X et Y ne sont pas substitués.

Selon un autre mode de réalisation particulier, lorsque l'un au moins des groupes X et Y est substitué, il comporte un ou deux groupes de substitution et ledit groupe de substitution est avantageusement indépendamment choisi parmi un groupe (C₁-C₂)alkyle, un groupe (C₁-C₂)fluoroalkyle, un groupe (C₁-C₂)alcoxy, un groupe (C₁-C₂)fluoroalcoxy, un atome d'halogène, un groupe hydroxy et un groupe -COOH.

Toujours selon cet autre mode de réalisation, lorsque Y est un groupe hétéroaryle et est monosubstitué, la substitution se situe avantageusement en position 3 ou 5.

Selon un autre mode de réalisation particulier, le groupe R₂ représente l'une des formules suivantes, le groupe R₉ étant alors un atome d'hydrogène :

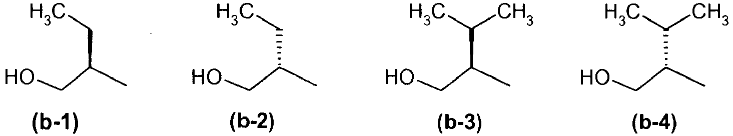

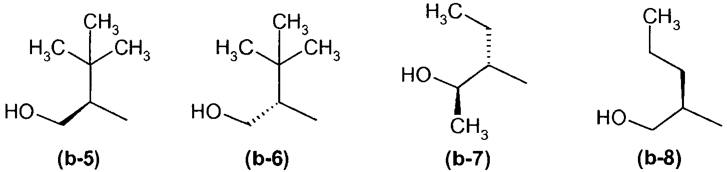

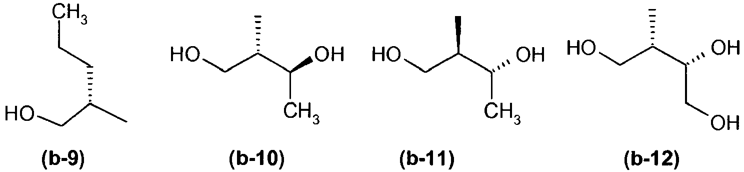

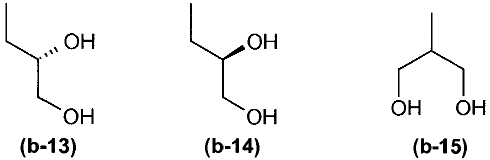

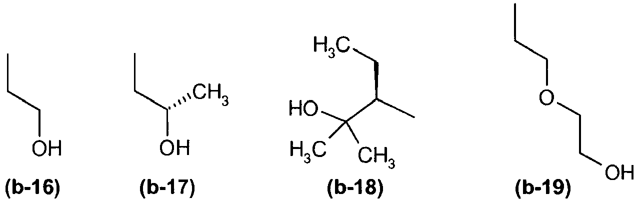

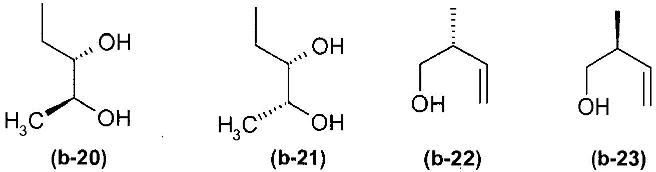

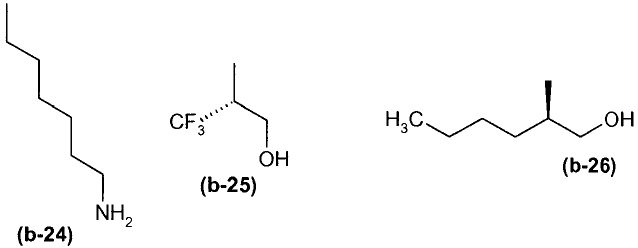

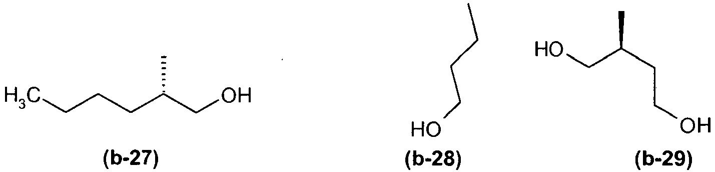

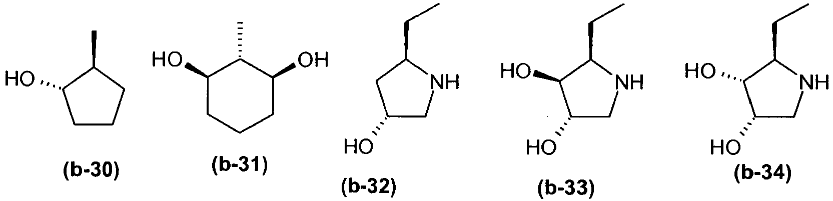

Selon un mode de réalisation encore plus particulier, le groupe R₂ représente l'une des formules (**b-1**), (**b-2**), (**b-10**), (**b-11**), (**b-13**), (**b-14**) ou (**b-15**) telles que définies ci-dessus.

Selon un mode de réalisation particulier, lorsque R₂ et R₉ forment ensemble un hétérocycle, le groupe pyrrolidinyle est avantageusement un groupe pyrrolidin-1-yle, le groupe pipéridinyle est avantageusement un groupe pipéridin-1-yle, le groupe pipérazinyle est avantageusement un groupe pipérazin-1-yle et le groupe pipéridinylpipéridinyle est avantageusement un groupe pipéridin-4-ylpipéridin-1-yle.

Selon encore un autre mode de réalisation particulier le groupement NR₂R₉ représente l'une des formules suivantes :

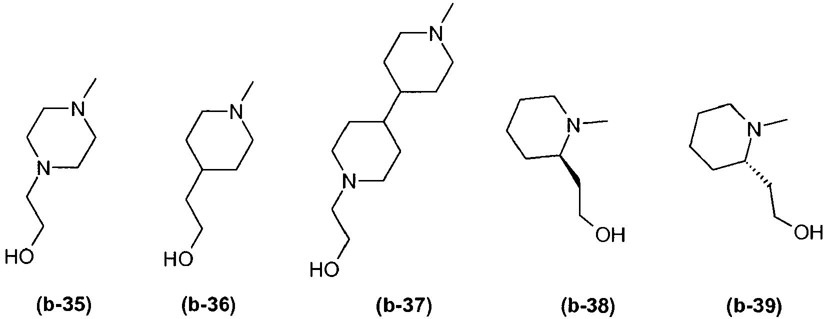

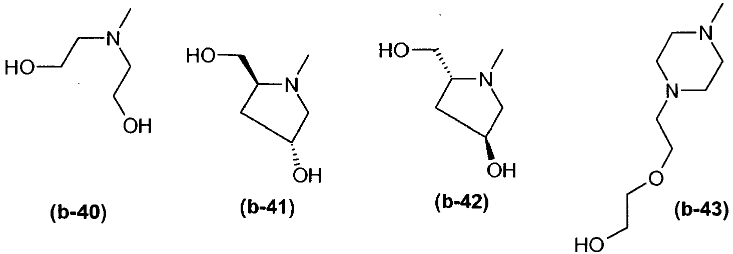

Selon encore un autre mode de réalisation particulier, le groupement NR₂R₉ présente l'une des formules (**b-35**) à (**b-43**), à l'exclusion des groupements (**b-38**) et (**b-39**).

Selon un autre mode de réalisation encore plus particulier, l'invention est relative à des composés de formule (**I**) telle que définie ci-dessus, dans laquelle R₁ représente un groupe isopropyle ; R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy et avantageusement représente l'une des formules (**b-1**), (**b-2**), (**b-10**), (**b-11**), (**b-13**), (**b-14**) ou (b-15) telles que définies ci-dessus ; et X et Y sont tels que définis ci-dessus et ne sont pas substitués et sont avantageusement indépendamment un groupe phényle, un groupe pyridyle ou un groupe thiényle, étant entendu que X et Y ne sont pas simultanément un groupe thiényle ou un groupe pyridyle.

Selon encore un autre mode de réalisation particulier, le groupe pipéridyle de formule (**B**) est un groupe :

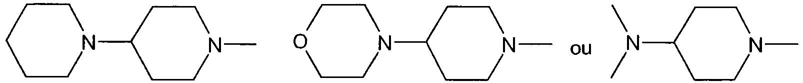

Parmi les composés objets de l'invention, on peut citer un premier groupe de composés de formule (**Ia**), couverts par la formule (**I**) : 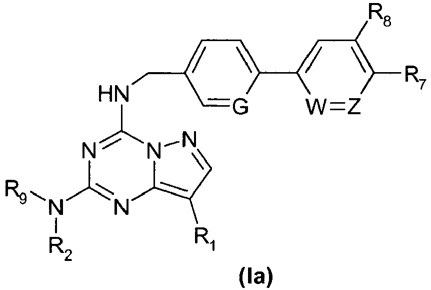 dans laquelle :
R₁ est tel que défini précédemment et est avantageusement un groupe isopropyle,
R₂ et R₉ représentent indépendamment un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy avec R₉ pouvant représenter un atome d'hydrogène et R₂ représente avantageusement l'une des formules (**b-1**), (b-2), (**b-10**), (**b-11**), (**b-13**), (**b-14**) ou (**b-15**) telles que définies ci-dessus, tandis que R₉ est un atome d'hydrogène,
ou bien R₂ et R₉ forment ensemble, avec l'atome d'azote qui les porte, un groupe pipéridin-1-yle ou pipéridin-4-ylpipéridin-1-yle, ledit groupe étant substitué par un à trois groupes (C₁-C₆)alkyle substitué par un groupe hydroxy et R₂ et R₉ pris ensemble représentent avantageusement l'une des formules (**b-37**), (**b-38**), (**b-39**) ou (**b-40**) telles que définies ci-dessus,
R₇ et R₈ représentent indépendamment un atome d'hydrogène, un groupe (C₁-C₂)alkyle, un groupe (C₁-C₂)fluoroalkyle, un groupe (C₁-C₂)alcoxy, un groupe (C₁-C₂)fluoroalcoxy, un atome d'halogène, un groupe hydroxy ou un groupe -COOH,
G représente -CH=, ou -N=, et
lorsque G représente -CH=, les groupes W et Z représentent soit simultanément -CH=, soit l'un représente -N= et l'autre représente -CH=,
lorsque G représente -N=, alors W et Z représentent -CH=.

Parmi les composés objets de l'invention, on peut citer un deuxième groupe de composés de formule (**Ib**), couverts par la formule (**I**) : 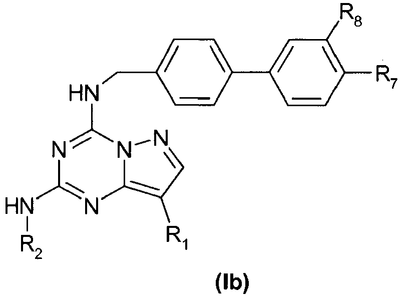 dans laquelle :
R₁ est tel que défini précédemment et est avantageusement un groupe isopropyle,
R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy, et avantageusement représente l'une des formules (**b-1**), (**b-2**), (**b-10**), (**b-11**), (**b-13**), (**b-14**) ou (**b-15**) telles que définies ci-dessus, et
R₇ et R₈ représentent indépendamment un atome d'hydrogène, un groupe (C₁-C₂)alkyle, un groupe (C₁-C₂)fluoroalkyle, un groupe (C₁-C₂)alcoxy, un groupe (C₁-C₂)fluoroalcoxy, un atome d'halogène, un groupe hydroxy ou un groupe -COOH.

Parmi les composés objets de l'invention, on peut citer un troisième groupe de composés de formule (**Ic**), couverts par la formule (**I**) : 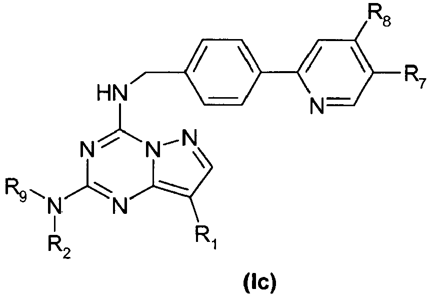 dans laquelle :
R₁ est tel que défini précédemment et est avantageusement un groupe éthyle ou isopropyle,
soit R₉ est un atome d'hydrogène et R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy, et avantageusement représente l'une des formules (**b-1**), (**b-2**), (**b-10**), (**b-11**), (**b-13**), (**b-14**) ou (**b-15**) telles que définies ci-dessus,
soit R₂ et R₉ forment ensemble, avec l'atome d'azote qui les porte, un groupe pipéridin-1-yle ou pipéridin-4-ylpipéridin-1-yle, ledit groupe étant substitué par un à trois groupes (C₁-C₆)alkyle substitué par un groupe hydroxy et R₂ et R₉ pris ensemble représentent avantageusement l'une des formules (**b-37**), (**b-38**), (**b-39**) ou (**b-40**) telles que définies ci-dessus, et
R₇ et R₈ représentent indépendamment un atome d'hydrogène, un groupe (C₁-C₂)alkyle, un groupe (C₁-C₂)fluoroalkyle, un groupe (C₁-C₂)alcoxy, un groupe (C₁-C₂)fluoroalcoxy, un atome d'halogène, un groupe hydroxy ou un groupe -COOH.

Parmi les composés objets de l'invention, on peut citer un troisième groupe de composés de formule (**Id**), couverts par la formule (**I**) : 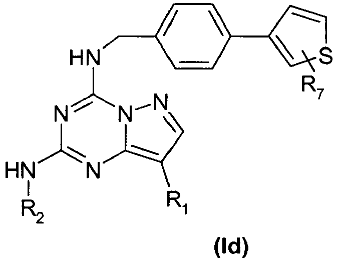 dans laquelle :
R₁ est tel que défini précédemment et est avantageusement un groupe isopropyle,
R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy, et avantageusement représente l'une des formules (**b-1**), (**b-2**), (**b-10**), **(b-11**), (**b-13**), (**b-14**) ou (**b-15**) telles que définies ci-dessus, et
R₇ représente un atome d'hydrogène, un groupe (C₁-C₂)alkyle, un groupe (C₁-C₂)fluoroalkyle, un groupe (C₁-C₂)alcoxy, un groupe (C₁-C₂)fluoroalcoxy, un atome d'halogène, un groupe hydroxy ou un groupe -COOH.

Parmi les composés objets de l'invention, on peut citer un quatrième groupe de composés de formule (Ie), couverts par la formule (I) : 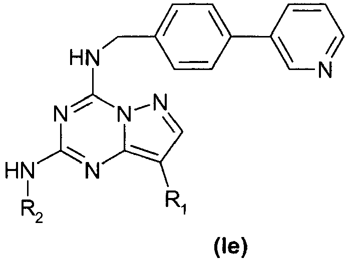 dans laquelle :
R₁ est tel que défini précédemment et avantageusement un groupe isopropyle,
R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy, et avantageusement représente l'une des formules (**b-1**), (**b-2**), (**b-10**), (**b-11**), (**b-13**), (**b-14**) ou (**b-15**) telles que définies ci-dessus.

Parmi les composés objets de l'invention, on peut citer un cinquième groupe de composés de formule (If), couverts par la formule (I) : 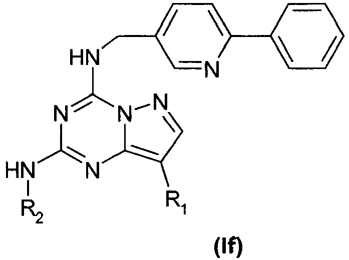 dans laquelle :
R₁ est tel que défini précédemment et avantageusement un groupe isopropyle,
R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy, et avantageusement représente l'une des formules (**b-1**), (**b-2**), (**b-10**), (**b-11**), (**b-13**), (**b-14**) ou (**b-15**) telles que définies ci-dessus.

Les sels pharmaceutiquement acceptables des composés de formule (**Ia**), (**Ib**), (**Ic**), (**Id**), (**Ie**) et (**If**) font également partie de l'invention.

Parmi les composés de formule (**I**) de l'invention, on peut notamment citer les composés suivants :
- (*R*) 2-(1-hydroxybut-2-ylamino)-8-isopropyl-4-(4-phénylbenzylamino)pyrazolo[1,5-*a*]-1,3,5-triazine (**1**)
- (*R*) 2-(1-hydroxybut-2-ylamino)-8-isopropyl-4-[4-(pyridin-2-yl)benzylamino]pyrazolo[1,5-*a*]-1,3,5-triazine (**2**)
- Sel de fumarate du (*R*) 2-(1-hydroxybut-2-ylamino)-8-isopropyl-4-[4-(pyridin-2-yl)benzylamino]pyrazolo[1,5-*a*]-1,3,5-triazine (**3**)
- (*S*) 2-(1-hydroxybut-2-ylamino)-8-isopropyl-4-[4-(pyridin-2-yl)benzylamino]pyrazolo[1,5-*a*]-1,3,5-triazine **(4)**
- Sel de fumarate du (*S*) 2-(1-hydroxybut-2-ylamino)-8-isopropyl-4-[4-(pyridin-2-yl)benzylamino]pyrazolo[1,5-*a*]-1,3,5-triazine **(5)**
- (*R*) 2-(1-hydroxybut-2-ylamino)-8-isopropyl-4-[4-(thiophén-3-yl)benzylamino]pyrazolo[1,5-*a*]-1,3,5-triazine **(6)**
- (*S*) 2-(1-hydroxybut-2-ylamino)-8-isopropyl-4-[4-(thiophén-3-yl)benzylamino]pyrazolo[1,5-*a*]-1,3,5-triazine **(7)**
- (*S*) 2-(1,2-dihydroxypropan-3-ylamino)-8-isopropyl-4-[4-(pyridin-2-yl)benzylamino]pyrazolo[1,5-*a*]-1,3,5-triazine **(8)**
- Sel de fumarate du (S) 2-(1,2-dihydroxypropan-3-ylamino)-8-isopropyl-4-[4-(pyridin-2-yl)benzylamino]pyrazolo[1,5-*a*]-1,3,5-triazine **(9)**
- (*R*) 2-(1,2-dihydroxypropan-3-ylamino)-8-isopropyl-4-[4-(pyridin-2-yl)benzylamino]pyrazolo[1,5-*a*]-1,3,5-triazine **(10)**
- Sel de fumarate du (*R*) 2-(1,2-dihydroxypropan-3-ylamino)-8-isopropyl-4-[4-(pyridin-2-yl)benzylamino]pyrazolo[1,5-*a*]-1,3,5-triazine **(11)**
- (*2R,3R*) 2-(1,3-dihydroxybut-2-ylamino)-8-isopropyl-4-[4-(pyridin-2-yl)benzylamino]pyrazolo[1,5-*a*]-1,3,5-triazine **(12)**
- sel de fumarate du (2*R*,3*R*) 2-(1,3-dihydroxybut-2-ylamino)-8-isopropyl-4-[4-(pyridin-2-yl)benzylamino]pyrazolo[1,5-*a*]-1,3,5-triazine **(13)**
- (2*S*,3*S*) 2-(1,3-dihydroxybut-2-ylamino)-8-isopropyl-4-[4-(pyridin-2-yl)benzylamino]pyrazolo[1,5-*a*]-1,3,5-triazine **(14)**
- Sel de fumarate du (2*S*,3*S*) 2-(1,3-dihydroxybut-2-ylamino)-8-isopropyl-4-[4-(pyridin-2-yl)benzylamino]pyrazolo[1,5-*a*]-1,3,5-triazine **(15)**
- (2*R*,3*R*) 2-(1,3-dihydroxybut-2-ylamino)-8-isopropyl-4-[4-(thiophén-3-yl)benzylamino]pyrazolo[1,5-*a*]-1,3,5-triazine **(16)**
- 2-(1,3-dihydroxyprop-2-ylamino)-8-isopropyl-4-[4-(pyridin-2-yl)benzylamino]pyrazolo[1,5-*a*]-1,3,5-triazine **(17)**
- Sel de fumarate du 2-(1,3-dihydroxyprop-2-ylamino)-8-isopropyl-4-[4-(pyridin-2-yl)benzylamino]pyrazolo[1,5-*a*]-1,3,5-triazine **(18),**
- 2-(1,3-dihydroxyprop-2-ylamino)-8-isopropyl-4-[4-(pyridin-3-yl)benzylamino]pyrazolo[1,5-*a*]-1,3,5-triazine **(19)**
- (*S*) 2-(-1-hydroxybut-2-ylamino)-8-isopropyl-4-[4-(pyridin-3-yl)benzylamino]pyrazolo[1,5-*a*]-1,3,5-triazine **(20)**
- (2*S*,3*S*) 2-(1,3-dihydroxybut-2-ylamino)-8-isopropyl-4-[4-phényl(pyridin-3-yl)méthylamino]pyrazolo[1,5-*a*]-1,3,5-triazine **(21)**
- (*S*) 2-(-1-hydroxybut-2-ylamino)-8-isopropyl-4-[4-phényl(pyridin-3-yl)méthylamino]pyrazolo[1,5-*a*]-1,3,5-triazine **(22)**
- (2*R*,3*R*) 2-(1,3-dihydroxybut-2-ylamino)-8-isopropyl-4-[4-phényl(pyridin-3-yl)méthylamino]pyrazolo[1,5-*a*]-1,3,5-triazine **(23)**
- 2-(1,3-dihydroxyprop-2-ylamino)-8-isopropyl-4-[4-phényl(pyridin-3-yl)méthylamino]pyrazolo[1,5-*a*]-1,3,5-triazine **(24)**
- (*R*) 2-(1-hydroxy-4-méthylpent-2-ylamino)-8-isopropyl-4-[4-phényl(pyridin-3-yl)méthylamino]pyrazolo[1,5-*a*]-1,3,5-triazine **(25)**
- (*S*) 2-(1-hydroxy-4-méthylpent-2-ylamino)-8-isopropyl-4-[4-phényl(pyridin-3-yl)méthylamino]pyrazolo[1,5-*a*]-1,3,5-triazine **(26)**
- (*S*) 2-(1-hydroxy-3,3-diméthylbut-2-ylamino)-8-isopropyl-4-[4-(pyridin-2-yl)benzylamino]pyrazolo[1,5-*a*]-1,3,5-triazine **(27)**
- (*S*) 2-(1-hydroxy-3-méthylbut-2-ylamino)-8-isopropyl-4-[4-(pyridin-2-yl)benzylamino]pyrazolo[1,5-*a*]-1,3,5-triazine **(28)**
- 2-[4-[1-[8-isopropyl-4-[[4-(pyridin-2-yl)phényl]méthylamino]pyrazolo[1,5-a]-1,3,5-triazin-2-yl]pipéridin-4-yl]pipéridin-1-yl]éthanol **(29)**
- 2-[4-[8-isopropyl-4-[[4-(pyridin-2-yl)phényl]méthylamino]pyrazolo[1,5-a]-1,3,5-triazin-2-yl]pipérazin-1-yl]éthanol **(30)**
- (*R*) 2-(1,2-dihydroxypropan-3-ylamino)-8-éthyl-4-[4-(pyridin-2-yl)benzylamino]pyrazolo[1,5-*a*]-1,3,5-triazine **(31)**
- [(2*R*)-3-[[8-ethyl-4-[[4-(2-pyridyl)phenyl]methyl-amino]pyrazolo[1,5-a][1,3,5]triazin-2-yl]amino]-2-hydroxy-propyl] (2R)-2-Bocamino-3-methyl-butanoate **(32)**
- [(2*R*)-3-[[8-ethyl-4-[[4-(2-pyridyl)phenyl]methylamino]pyrazolo[1,5-a][1,3,5]triazin-2-yl]amino]-2-hydroxy-propyl] (2R)-2-amino-3-methyl-butanoate **(33)** à l'état de base ou de sel notamment de dichlorhydrate
- (*S*) 2-(1,2-dihydroxypropan-3-ylamino)-8-éthyl-4-[4-(pyridin-2-yl)benzylamino]pyrazolo[1,5-*a*]-1,3,5-triazine **(34)**
- (*S*) 8-ethyl-2-(-1-hydroxybut-2-ylamino)-4-[4-(pyridin-2-yl)benzylamino]pyrazolo[1,5-*a*]-1,3,5-triazine **(35)**
- (*R*) 8-ethyl-2-(-1-hydroxybut-2-ylamino)-4-[4-(pyridin-2-yl)benzylamino]pyrazolo[1,5-*a*]-1,3,5-triazine **(36)**
- 2-(1,3-dihydroxyprop-2-ylamino)-8-éthyl-4-[4-(pyridin-2-yl)benzylamino]pyrazolo[1,5-*a*]-1,3,5-triazine **(37)**
- 2-[(2*S*)-1-[8-éthyl-4-[[4-(2-pyridyl)phényl]methylamino]pyrazolo[1,5-a][1,3,5]triazin-2-yl]-2-pipéridyl]éthanol **(38)**
- 2-[[8-éthyl-4-[[4-(2-pyridyl)phényl]méthylamino]-pyrazolo[1,5-a][1,3,5]triazin-2-yl]-(2-hydroxyéthyl)amino]éthanol **(39)**
- (2*R*,3*R*)-2-[[8-éthyl-4-[[4-(2-pyridyl)phényl]méthylamino]pyrazolo[1,5-a][1,3,5]triazin-2-yl]amino]butane-1,3-diol **(40)**
- (2*S*,3*S*)-2-[[8-éthyl-4-[[4-(2-pyridyl)phényl]méthylamino]pyrazolo[1,5-a][1,3,5]triazin-2-yl]amino]butane-1,3-diol **(41)**
ainsi que leurs éventuels sels d'acides pharmaceutiquement acceptables.

Les composés suivants font également partie de l'invention. 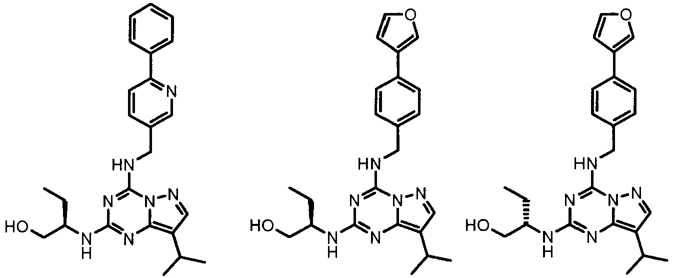 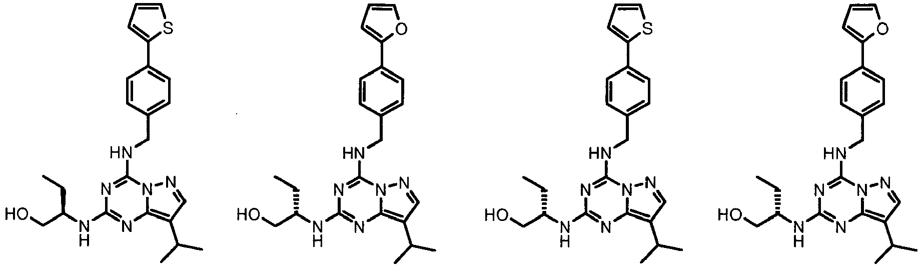 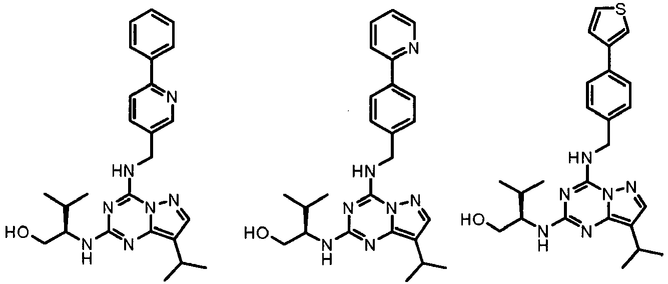 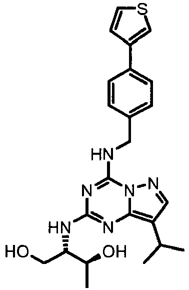 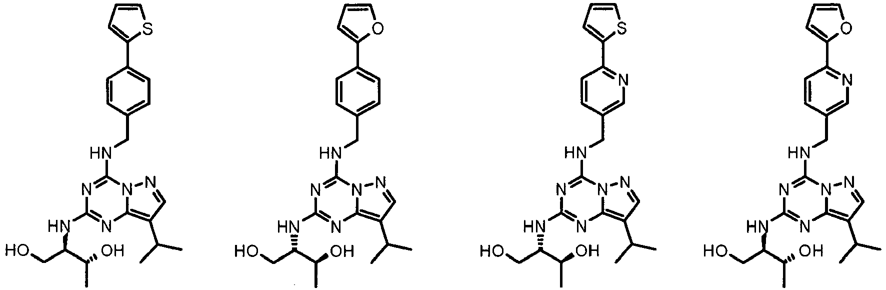 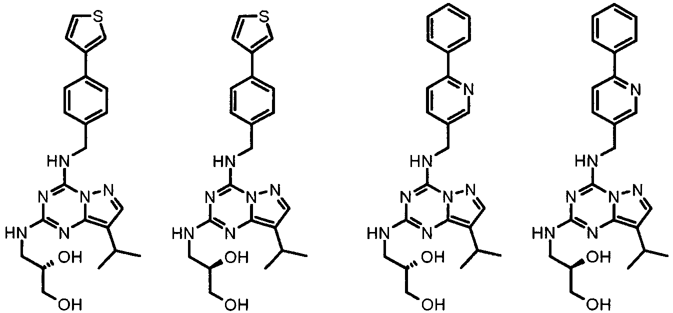 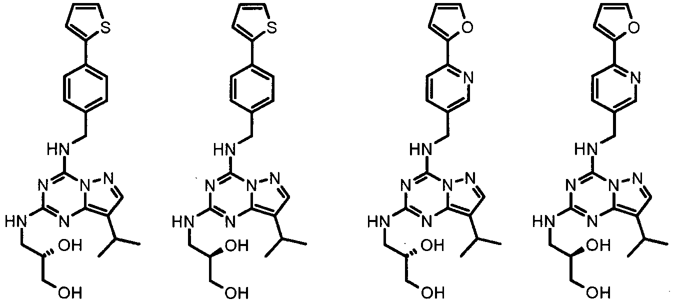 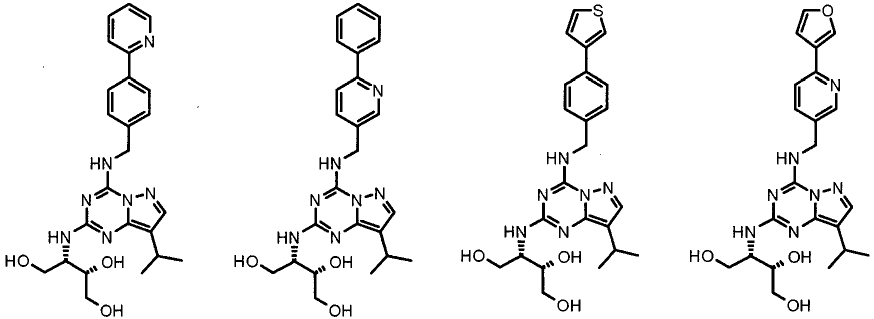 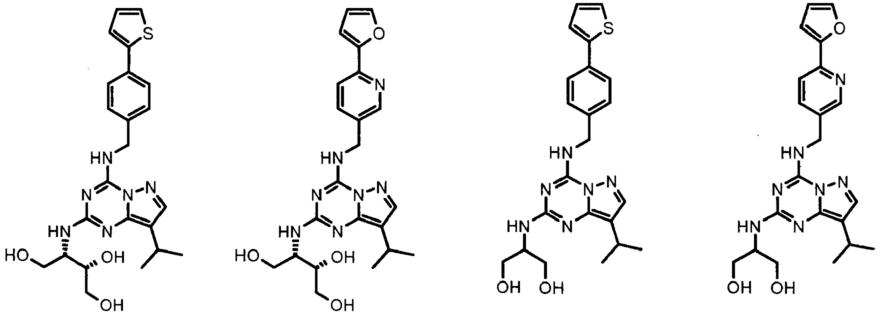 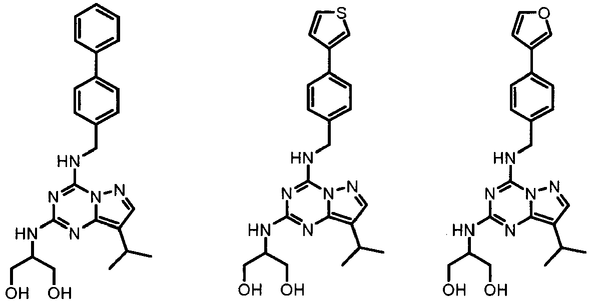 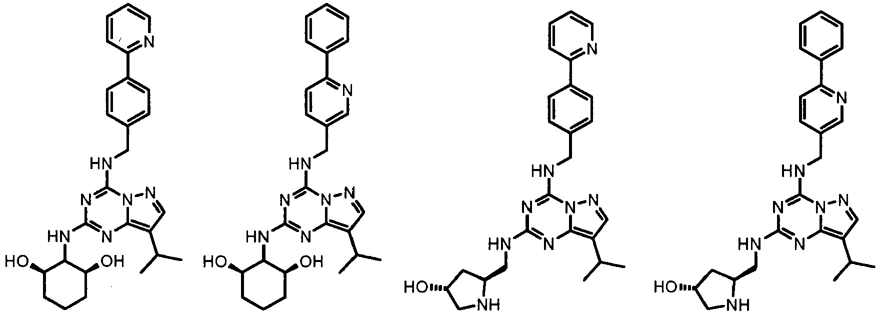 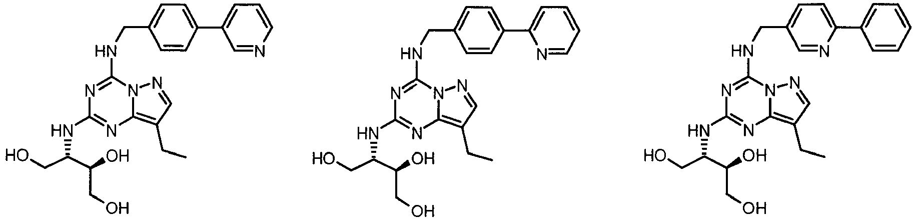 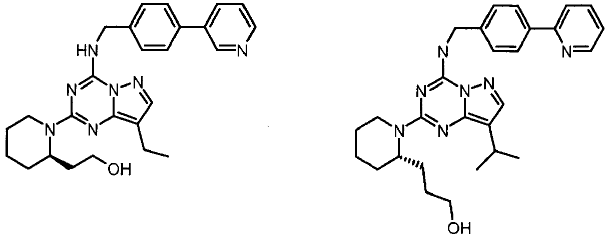 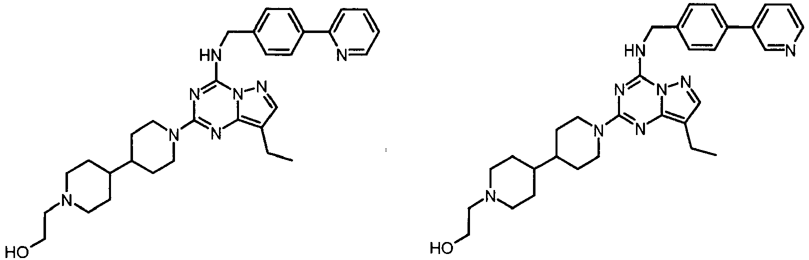 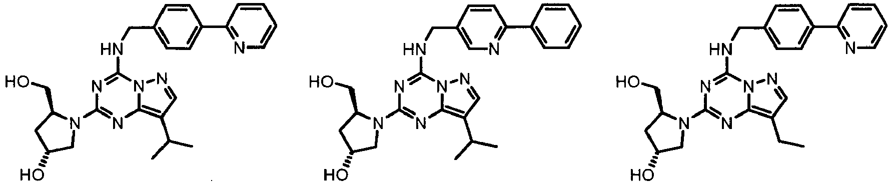 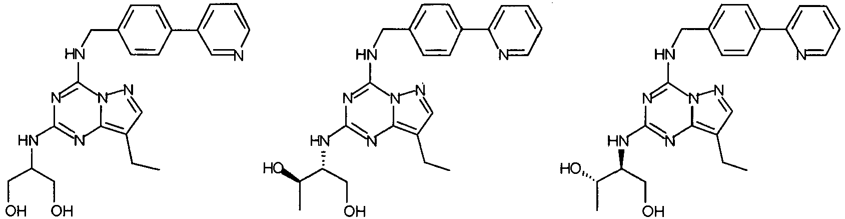 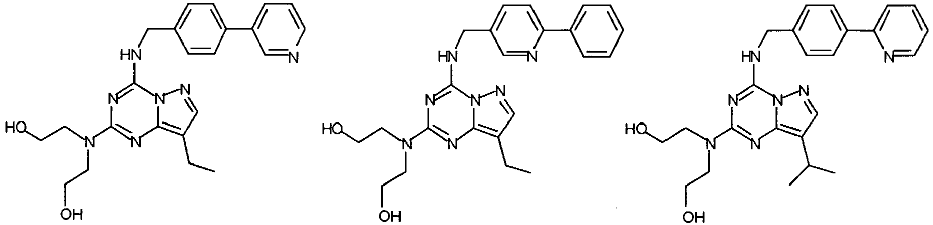
- et leurs sels d'acides pharmaceutiquement acceptables.

L'invention a également pour objet un procédé de préparation des composés de formule (**I**)**.**

Les composés de l'invention peuvent être préparés selon différentes méthodes décrites selon le Schéma 1 décrit ci-dessous :

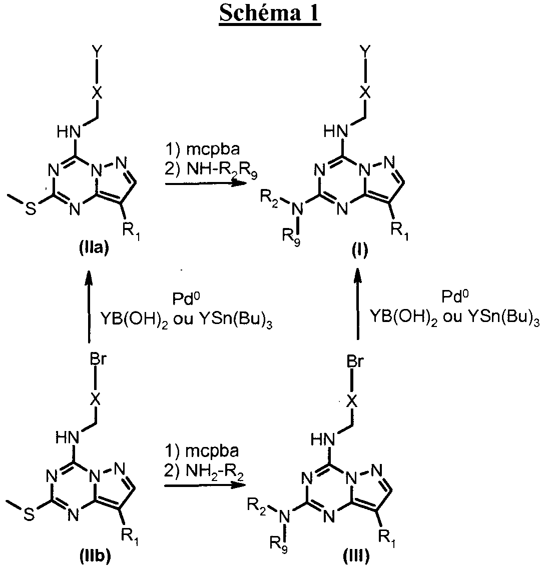

Selon le schéma 1, le composé de formule (**IIa**), où X, Y et R₁ sont tels que définis précédemment, est soumis à une réaction d'oxydation en présence d'acide méta-chloroperbenzoïque (acide méta-chloroperbenzoïque) par exemple dans le dichlorométhane à 0°C, pour conduire à la sulfone correspondante. Cette dernière est engagée directement dans une réaction de substitution nucléophile aromatique (SN_{AR}) en présence d'une amine primaire de formule NH-R₂R₉, où R₂ et R₉ sont tels que définis précédemment, à une température variant de 100 à 180°C, par exemple à 140°C, pour conduire au composé de formule (**I**). Alternativement, on peut également préparer les composés de type (**I**) à partir des composés de formule (**IIb**). Ces composés (**IIb**) où X et R₁ sont tels que définis précédemment, sont initialement engagés dans une réaction de couplage catalysée par un métal comme par exemple le palladium (réaction de Suzuki-Miyaura ou de Stille) ou le nickel, pour donner les intermédiaires (**IIa**)**.** Ces mêmes composés (**IIb**) peuvent être oxydés en présence d'acide méta-chloroperbenzoïque dans le dichlorométhane à 0°C pour conduire à la sulfone qui est déplacée par une amine primaire (SN_{AR}) pour donner les composés de formule (**III**)**.** Ces derniers, soumis à une réaction de couplage catalysée par un métal comme par exemple le palladium ou le nickel, conduisent aux composés de formule (**I**)**.**

Ainsi, la présente invention concerne selon un de ses aspects un procédé de préparation d'un composé de formule (**I**) conforme à l'invention, caractérisé en ce que l'on fait réagir un composé de formule (**IIa**) qui suit : 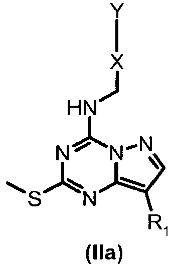 dans laquelle X, Y et R₁ sont tels que définis précédemment, avec de l'acide méta-chloroperbenzoïque dans une réaction d'oxydation et en ce qu'on engage la sulfone obtenue directement dans une réaction de substitution nucléophile en présence d'une amine primaire de formule NH₂-R₂, où R₂ est tel que défini précédemment, à une température variant de 100 à 180°C, par exemple à 140°C, pour conduire au composé de formule (**I**), ou bien en ce que l'on fait réagir un composé de formule (**IIb**) 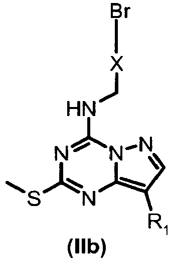 dans laquelle X et R₁ sont tels que définis précédemment, avec un métal comme par exemple le palladium ou le nickel, pour donner un composé de formule (**IIa**) tel que défini ci-dessus, ou alternativement, en ce que l'on fait réagir un composé de formule (**IIb**) tel que défini ci-dessus, avec de l'acide méta-chloroperbenzoïque dans une réaction d'oxydation, la sulfone obtenue étant directement engagée dans une réaction de substitution nucléophile en présence d'une amine primaire de formule NH-R₂R₉, où R₂ et R₉ sont tels que définis précédemment, pour donner un composé de formule (**III**) : 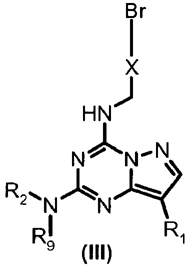 dans laquelle X₁, R₁ et R₂ sont tels que définis précédemment, que l'on soumet à une réaction de couplage catalysée par un métal comme par exemple le palladium ou le nickel, pour conduire à un composé de formule (**I**).

On décrit ci-après les méthodes de préparation des dérivés de formule (**IIa**) ou (IIb) qui sont utilisés pour la préparation des composés de formule (**I**) de l'invention.

Les dérivés de formule (**IIa**) ou (**IIb**) peuvent être préparés à partir des composés (**IV**) par la méthode décrite dans le document « pyrazolo[1,5-a]-1,3,5-triazine as a purine bioisostere: access to potent cyclin-dependent kinase inhibitor (R)-roscovitine analogue », J. Med. Chem. 2009, 52, 655.

De façon générale, on fait réagir les 4-(*N*-méthyl-*N*-phénylamino)-2-thiomëthylpyrazolo[1,5-*a*]-1,3,5-triazines substituées en position 8 (**IV**), où R₁ est tel que défini précédemment avec les aryl- ou hétéroarylméthylèneamines (**V**), où X et Y sont tels que définis précédemment, à une température variant de 100 à 180 °C, par exemple à 140 °C pour conduire aux composés de formule (**IIa**) ou (**IIb**) selon le Schéma 2 décrits ci-dessous :

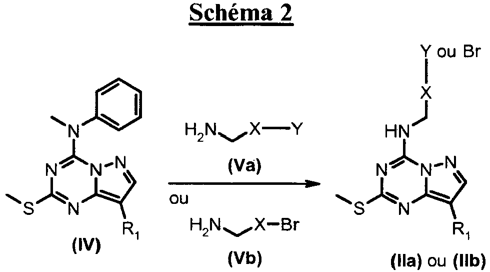

Les composés de formule (**IV**), lorsqu'ils ne sont pas disponibles commercialement, peuvent être préparés à partir des procédés décrits dans la littérature, par exemple dans la publication « pyrazolo[1,5-*a*]-1,3,5-triazine as a purine bioisostere : access to potent cyclin-dependent kinase inhibitor (R)-roscovitine analogue », citée plus haut.

Les composés de formule (**V**), lorsqu'ils ne sont pas disponibles commercialement, peuvent être préparés à partir des procédés décrits dans la littérature, par exemple dans la publication WO 2003/022805.

Dans les schémas généraux de synthèse 1 et 2, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'homme du métier.

L'invention, selon un autre de ses aspects, a également pour objet les composés de formule (II) 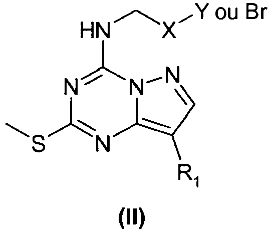 dans laquelle :
X, Y et R₁ sont tels que définis précédemment.

Ces composés sont utiles comme intermédiaires de synthèse des composés de formule (**I**). Des exemples de composés de formule (**II**) sont donnés dans le tableau 1 ci-après.

Les exemples qui suivent décrivent également la préparation de composés de formule (**I**) conformes à l'invention. Ces exemples ne sont pas limitatifs et ne font qu'illustrer l'invention. Les numéros des composés exemplifiés renvoient à ceux donnés dans le tableau 2, qui illustre les structures chimiques et les propriétés physiques de quelques composés selon l'invention.

Dans les préparations et exemples qui suivent :
- PF = Point de Fusion (en degré Celsius) tel que mesuré sur un appareil Büchi B545 avec un gradient de température de 1°C par minute.
- RMN = résonance magnétique nucléaire réalisée avec un spectromètre Bruker 300 MHz ou 400 MHz. Les solvants employés sont le DMSO-*d₆* et le CDCl₃ et les déplacements chimiques sont exprimés par rapport au TMS. Les abréviations utilisées sont :
- s = singulet,
- d = doublet,
- dd = doublet dédoublé,
- t = triplet,
- m = multiplet,
- sél = singulet élargi.

### I. Préparation des INTERMEDIAIRES

### Préparation I.1

### 8-Isopropyl-2-(méthylsulfanyl)-4-(4-phénylbenzylamino)pyrazolo[1,5-a]-1,3,5-triazine (IIa.1).

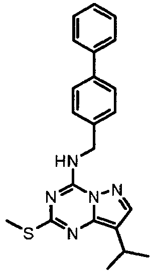

Dans un tube scellé, une solution de 8-isopropyl-4-(*N*-méthyl-*N*-phénylamino)-2-(méthylsulfanyl)pyrazolo[1,5-*a*]-1,3,5-triazine (114 mg, 0,36 mmol) et de 4-phénylbenzylamine (100 mg, 0,55 mmol) est chauffé à 140°C pendant 24 h. Après refroidissement, le solvant est évaporé. Le composé brut recueilli est purifié par chromatographie flash (EP/Et₂O 9:1) pour donner **IIa.1** (95 mg, 67%). PF = 134-135 °C (MeOH). ¹H RMN (300 MHz, CDCl₃): δ 7,75 (s, 1H, Hₐᵣₒₘ), 7,59-7,56 (m, 4H, Hₐᵣₒₘ), 7,45-7,34 (m, 5H, Hₐᵣₒₘ), 6,93 (sél, 1H, NH), 4.85 (d, 2H, *J* = 5,7 Hz, CH₂), 3,18 (hept, 1H, *J* = 7,2 Hz, CH), 2,58 (s, 3H, CH₃), 1,33 (d, 6H, *J =* 7,2 Hz, 2 CH₃). SM (ESI): *m*/*z* 390 (MH⁺).

### Préparation I.2

### 8-Isopropyl-2-(méthylsulfanyl)-4-[4-(pyridin-2-yl)benzylamino]pyrazolo[1,5-a]-1,3,5-triazine (IIa.2)

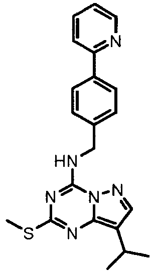

Selon les mêmes conditions conduisant à la préparation du composé **IIa.1,** le composé **IIa.2** est préparé à partir de 8-isopropyl-4-(*N*-méthyl-*N*-phénylamino)-2-(méthylsulfanyl)pyrazolo[1,5-*a*]-1,3,5-triazine et de 4-(pyridin-2-yl)benzylamine. Rdt = 60%. PF = 136-138°C (EtOH). ¹H RMN (300 MHz, CDCl₃): δ 8,71 (d, 1H, *J* = 4,7 Hz, Hₐᵣₒₘ), 8,00 (d, 2H, *J* = 8,1 Hz, Hₐᵣₒₘ), 7,82-7,72 (m, 3H, Hₐᵣₒₘ), 7.49 (d, 2H, *J* = 8,1 Hz, Hₐᵣₒₘ), 7,26 (dd, 1H, *J* = 4,7, 7,2 Hz, Hₐᵣₒₘ), 6,76 (sél, 1H, NH), 4,86 (d, 2H, *J* = 6,0 Hz, CH₂), 3,16 (hept, 1H, *J =* 7,0 Hz, CH), 2,57 (s, 3H, CH₃), 1,33 (d, 6H, *J =* 7,0 Hz, 2 CH₃). SM (ESI): *m*/*z* 391 (MH⁺).

### Préparation I.3

### 8-Isopropyl-2-(méthylsulfanyl)-4-[4-(thiophén-3-yl)benzylamino]pyrazolo[1,5-a]-1,3,5-triazine (IIa.3)

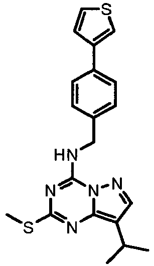

Selon les mêmes conditions conduisant à la préparation du composé **IIa.1**, le composé **IIa.3** est préparé à partir de 8-isopropyl-4-(*N*-méthyl-*N*-phénylamino)-2-(méthylsulfanyl)pyrazolo[1,5-*a*]-1,3,5-triazine et de 4-(thiophén-3-yl)benzylamine. Rdt = 74%. PF = 141-143°C (EtOH). ¹H RMN (300 MHz, CDCl₃): δ 7,73 (s, 1H, Hₐᵣₒₘ), 7,57 (d, 2H, *J* = 8,3 Hz, Hₐᵣₒₘ), 7,45-7,36 (m, 5H, Hₐᵣₒₘ), 6,78 (sél, 1H, NH), 4,81 (d, 2H, *J* = 6,0 Hz, CH₂), 3,16 (hept, 1H, *J =* 7,0 Hz, CH), 2,58 (s, 3H, CH₃), 1,33 (d, 6H, *J* = 7,0 Hz, 2 CH₃). SM (ESI): *m*/*z* 396 (MH⁺).

### Préparation I.4

### 4-(4-Bromobenzylamino)-8-isopropyl-2-(méthylsulfanyl)pyrazolo[1,5-a]-1,3,5-triazine (IIb.4)

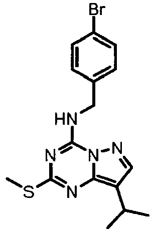

Selon les mêmes conditions conduisant à la préparation du composé **IIa.2,** le composé **IIb.4** est préparé à partir de 8-isopropyl-4-(*N*-méthyl-*N*-phénylamino)-2-(méthylsulfanyl)pyrazolo[1,5-*a*]-1,3,5-triazine et de 4-bromobenzylamine. Rdt = 75%. PF = 171-172°C (EtOH). ¹H RMN (300 MHz, CDCl₃): δ 7,74 (s, 1H, Hₐᵣₒₘ), 7,47 (d, 2H, *J* = 8,3 Hz, Hₐᵣₒₘ), 7,24 (d, 2H, *J* = 8,3 Hz, Hₐᵣₒₘ), 6,76 (sél, 1H, NH), 4,75 (d, 2H, *J* = 6,0 Hz, CH₂), 3,15 (hept, 1H, *J* = 6,8 Hz, CH), 2,56 (s, 3H, CH₃), 1,33 (d, 6H, *J* = 6,8 Hz, 2 CH₃). SM (ESI): *m*/*z* 394 (Br⁸¹, MH⁺), 392 (Br⁷⁹, MH⁺).

### Préparation 1.5

### (R) 2-(-1-Hydroxybut-2-ylamino)-4-(4-bromobenzylamino)-8-isopropylpyrazolo [1,5-a]-1,3,5-triazine (III.5)

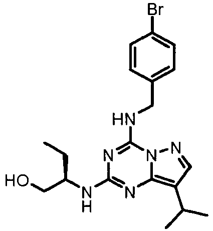

Une solution de **IIb.4** (200 mg, 0,51 mmol) dans le dichlorométhane (CH₂Cl₂) (6 mL) est agitée à 0 °C. L'acide méta-chloroperbenzoïque 70-75% (126 mg, 0,51 mmol) est ajouté, puis la solution est agitée pendant 1 h. La même quantité d'acide est additionnée une seconde fois. La solution finale est agitée pendant 2 h à 0°C. Après ajout d'une solution de NaHCO₃, l'extraction liquide-liquide est réalisée. La phase organique isolée est lavée par une solution de NaCl, séchée sur MgSO₄, puis évaporée sous pression réduite. La sulfone est obtenue avec un rendement quantitatif, puis engagée dans l'étape suivante sans autre purification. Une solution de sulfone (216 mg, 0,51 mmol) et de (*R*)-(-)-2-aminobutanol commercial (247 µL, 2,62 mmol) est chauffée à 140°C pendant 24 h. Après refroidissement, le solvant est évaporé. Le composé brut recueilli est purifié par chromatographie flash (EP/AcOEt 9:1 à 6:4) pour donner **III.5** (103 mg, 47%). Huile. ¹H RMN (300 MHz, CDCl₃): δ 7,57 (s, 1H, Hₐᵣₒₘ), 7,42 (d, 2H, *J* = 8,3 Hz, Hₐᵣₒₘ), 7,15 (d, 2H, *J* = 8,3 Hz, Hₐᵣₒₘ), 7,10 (sél, 1H, NH), 5,15 (sél, 1H, NH), 4,61 (d, 2H, *J* = 6,0 Hz, CH₂), 3,93-3,61 (m, 3H, CH + CH₂), 3,00 (hept, 1H, *J* = 6,8 Hz, CH), 1,70-1,52 (m, 2H, CH₂), 1,25 (d, 6H, *J =* 6,8 Hz, 2 CH₃), 1,00 (t, 3H, *J =* 7,4 Hz, CH₃). SM (ESI): *m*/*z* 435 (Br⁸¹, MH⁺), 433 (Br⁷⁹, MH⁺).

### Préparation I.6

### 8-Isopropyl-2-méthylsulfanyl-4-[4-(pyridin-3-yl)benzylamino]pyrazolo[1,5-a]-1,3,5-triazine (IIa.4)

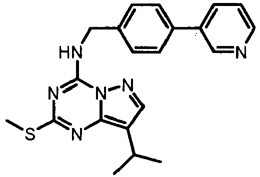

Selon les mêmes conditions conduisant à l'obtention du composé **IIa.2,** le composé **IIa.4** est obtenu à partir de la 8-isopropyl-4-(*N*-méthyl-*N*-phénylamino)-2-(méthylsulfanyl)pyrazolo[1,5-*a*]-1,3,5-triazine et de 4-(pyridin-3-yl)benzylamine. Rdt = 65%. ¹H RMN (400 MHz, CDCl₃): δ 8,83 (s, 1H, Hₐᵣₒₘ), 8,60 (d, 1H, Hₐᵣₒₘ), 7,88 (d, 1H, Hₐᵣₒₘ), 7,76(s, 1H, Hₐᵣₒₘ), 7.58 (d, 2H, Hₐᵣₒₘ), 7,51 (d, 2H, Hₐᵣₒₘ), 7,42 (m, 1H, Hₐᵣₒₘ), 6.78 (t, 1H, NH); 4.87 (d, 2H, *J* = 6,0 Hz, CH₂), 3,17 (hept, 1H, *J* = 6,8 Hz, CH), 2,55 (s, 3H, CH₃); 1,35 (d, 6H, *J* = 6,8 Hz, 2 CH₃). SM (ESI): *m*/*z* 391 (MH⁺).

### Préparation 1.7

### 8-Isopropyl-2-méthylsulfanyl-4-[4-phényl(pyridin-3yl)méthylamino]pyrazolo [1,5-a]-1,3,5-triazin-4-amine (IIa.5)

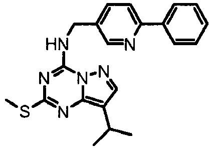

Selon les mêmes conditions conduisant à l'obtention du composé **II.a2,** le composé **IIa.5** est obtenu à partir de la 8-isopropyl-4-(*N*-méthyl-*N*-phénylamino)-2-(méthylsulfanyl)pyrazolo[1,5-*a*]-1,3,5-triazine et de (6-phénylpyridin-3-yl)méthanamine. ¹H RMN(400 MHz, CDCl₃): δ 8,71(s, 1H, Hₐᵣₒₘ), 7,98 (m, 3H, Hₐᵣₒₘ), 7,75 (m, 3H, Hₐᵣₒₘ), 7,45(m, 2H, Hₐᵣₒₘ), 6,85(t, 1H, NH), 4,85 (d, 2H, *J* = 6,0 Hz, CH₂), 3,15 (hept, 1H, *J* = 6,8 Hz, CH), 1,70(s, 3H, CH₃), (d, 6H, *J* = 6,8 Hz, 2 CH₃). SM (ESI): *m*/*z* 391 (MH⁺).

**Tableau 1**

| **Préparations n°** | **R₁** | **R₂** | **X** | **Y** | **PF (°C) et/ou masse (m/z)** |
|---|---|---|---|---|---|
| **IIa.1** | isoPr | -SMe | 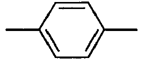 | 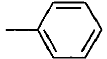 | PF = 134-135 MH⁺ = 390 |
| **IIa.2** | isoPr | -SMe | 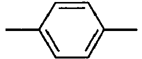 | 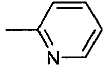 | PF = 136-138 MH⁺ = 391 |
| **IIa.3** | isoPr | -SMe | 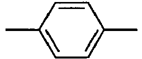 | 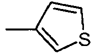 | PF = 141-143 MH⁺ = 396 |
| **IIb.4** | isoPr | -SMe | 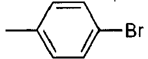 | / | PF = 171-172 MH⁺ = 394 |
| **III.5** | isoPr | 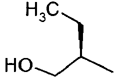 | 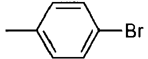 | / | MH⁺ = 435 |
| **IIIa.4** | isoPr | -SMe | 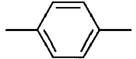 | 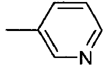 | MH+ = 391 |
| **IIIa.5** | isoPr | -SMe | 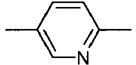 | 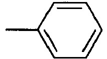 | MH+ = 391 |

Les exemples qui suivent illustrent l'invention sans la limiter.

### II. Préparation des composés de formule (I)

### Exemple 1: (R) 2-(-1-Hydroxybut-2-ylamino)-8-isopropyl-4-phénylbenzylamino)pyrazolo[1,5-a]-1,3,5-triazine (1)

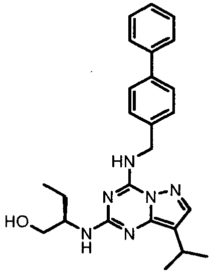

Une solution de **IIa.1** (160 mg, 0,41 mmol) dans le CH₂Cl₂ (4 mL) est agitée à 0 °C. L'acide méta-chloroperbenzoïque 70-75% (100 mg, 0,41 mmol) est ajouté, puis la solution est agitée pendant 1 h. La même quantité d'acide est additionnée une seconde fois. La solution finale est agitée pendant 2 h à 0°C. Après ajout d'une solution de NaHCO₃, l'extraction liquide-liquide est réalisée. La phase organique isolée est lavée par une solution de NaCl, séchée sur MgSO₄, puis évaporée sous pression réduite. La sulfone est obtenue avec un rendement quantitatif, puis engagée dans l'étape suivante sans autre purification. Une solution de 8-isopropyl-2-(méthylsulfonyl)-4-[*N*-(4-phényl)benzylamino]pyrazolo[1,5-*a*]-1,3,5-triazine (173 mg, 0,41 mmol) et de (*R*)-(-)-2-aminobutanol commercial (193 µL, 2,03 mmol) est chauffé à 140°C pendant 24 h. Après refroidissement, le solvant est évaporé. Le composé brut recueilli est purifié par chromatographie flash (EP/AcOEt 8:2, puis 1:1) pour donner 1 (75 mg, 43%). Huile. ¹H RMN (300 MHz, CDCl₃): δ 7,63 (s, 1H, Hₐᵣₒₘ), 7,59-7,56 (m, 4H, Hₐᵣₒₘ), 7,47-7,33 (m, 5H, Hₐᵣₒₘ), 6.74 (sél, 1H, NH), 4,78-4,75 (m, 2H, CH₂), 3,94-3,96 (sél, 1H, CH), 3,82 (d, *1 H, J* = 10,8 Hz, CH₂), 3,68 (dd, 1 H, *J* = 7,3, 10,8 Hz, CH₂), 3,02 (hept, 1H, *J* = 6,6 Hz, CH), 1,70-1,52 (m, 2H, CH₂), 1,28 (d, 6H, *J* = 6,8 Hz, 2 CH₃), 1,03 (t, 3H, *J* = 7,4 Hz, CH₃). SM (ESI): *m*/*z* 431 (MH⁺).

### Exemple 2: (R) 2-(-1-Hydroxybut-2-ylamino)-8-isopropyl-4-[4-(pyridin-2-yl)benzylamino]pyrazolo[1,5-a]-1,3,5-triazine (2)

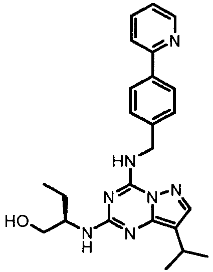

Une solution de **IIa.2** (400 mg, 1,02 mmol) dans le CH₂Cl₂ (12 mL) est agitée à 0 °C. L'acide méta-chloroperbenzoïque 70-75 % (250 mg, 1,02 mmol) est ajouté, puis la soltuion est agitée pendant 1 h. La même quantité d'acide est additionnée une seconde fois. La solution finale est agitée pendant 2 h à 0 °C. Après ajout d'une solution de NaHCO₃, l'extraction liquide-liquide est réalisée. La phase organique isolée est lavée par une solution de NaCl, séchée sur MgSO₄ puis évaporée sous pression réduite. La sulfone est obtenue avec un rendement quantitatif, puis engagée dans l'étape suivante sans autre purification. Une solution de sulfone (430 mg, 1,02 mmol) et de (R)-(-)-2-aminobutanol commercial (482 µL, 5,09 mmol) est chauffée à 140 °C pendant 12 h. Après refroidissement, le solvant est évaporé. Le composé brut recueilli est purifié par chromatographie flash (EP/AcOEt 9:1 à 6:4) pour donner 2 (200 mg, 45 %). Mousse. ¹H RMN (300 MHz, CDCl₃): δ 8,69 (d, 1H, *J* = 4,1 Hz, Hₐᵣₒₘ), 7,96 (d, 2H, *J* = 7,9 Hz, Hₐᵣₒₘ), 7,78-7,69 (m, 2H, Hₐᵣₒₘ), 7,62 (s, 1H, Hₐᵣₒₘ), 7,42 (d, 2H, *J* = 7,9 Hz, Hₐᵣₒₘ), 7,26-7,21 (m, 1H, Hₐᵣₒₘ), 6,91 (sél, 1H, NH), 5,10 (s, 1H, H échangeable), 4,75 (d, 2H, *J* = 6,0 Hz, CH₂), 3,93 (sél, 1H, CH), 3,83-3,62 (m, 2H, CH₂), 3,02 (hept, 1H, *J* = 6,8 Hz, CH), 1,70-1,52 (m, 2H, CH₂), 1,27 (d, 6H, *J* = 6,8 Hz, 2 CH₃), 1,02 (t, 3H, *J* = 7,4 Hz, CH₃). SM (ESI): m/z 432 (MH⁺).

### Exemple 3 : (R) 2-(-1-Hydroxybut-2-ylamino)-8-isopropyl-4-[4-(pyridin-2-yl)benzylamino]pyrazolo[1,5-a]-1,3,5-triazine (2)

Dans un tube scellé, le composé **III.5** (103 mg, 0,24 mmol) est additionné à une solution dégasée de tri-n-butyl(pyridin-2-yl)stannane (177 mg, 0,48 mmol) et de Pd(PPh₃)₄ (23 mg, 0,02 mmol) dans le toluène (10 mL). La solution finale dégasée est chauffée à 110°C pendant une nuit. Après refroidissement et dilution avec une solution de NaHCO₃, la solution est extraite avec le CH₂Cl₂ (3 x 12 mL). La phase organique isolée est lavée par une solution de NaCl. Après séchage sur MgSO₄, le solvant est évaporé sous pression réduite. Le résidu est purifié par chromatographie flash (EP/AcOEt 9:1 à 6:4) pour donner 2 (35 mg, 34 %). Mousse. ¹H RMN (300 MHz, CDCl₃): δ 8,69 (d, 1H, *J* = 4,1 Hz, H_{a.rom}), 7,96 (d, 2H, *J* = 7,9 Hz, Hₐᵣₒₘ), 7,78-7,69 (m, 2H, Hₐᵣₒₘ), 7,62 (s, 1H, Hₐᵣₒₘ), 7,42. (d, 2H, *J* = 7,9 Hz, Hₐᵣₒₘ), 7,26-7,21 (m, 1H, Hₐᵣₒₘ), 6,91 (sél, 1H, NH), 5,10 (s, 1H, H échangeable), 4,75 (d, 2H, *J* = 6,0 Hz, CH₂), 3,93 (sél, 1H, CH), 3,83-3,62 (m, 2H, CH₂), 3,02 (hept, 1H, *J*= 6,8 Hz, CH), 1,70-1,52 (m, 2H, CH₂), 1,27 (d, 6H, *J* = 6,8 Hz, 2 CH₃), 1,02 (t, 3H, *J* = 7,4 Hz, CH₃). SM (ESI): *m*/*z* 432 (MH⁺).

### Exemple 4 : Fumarate du (R) 2-(-1-hydroxybut-2-ylamino)-8-isopropyl-4-[4-(pyridin-2-yl)benzylamino]pyrazolo[1,5-a]-1,3,5-triazine (3)

Le produit **2** est traité par de l'acide fumarique dans une solution EtOH/Et₂O. Le sel de l'acide fumarique **3** cristallise dans le milieu réactionnel à 0°C. PF = 175-177 °C. ¹H RMN (300 MHz, DMSO-*d₆*): δ 13,12 (sél, 2H, OH), 8,70 (sél, 1H, NH), 8,64 (d, 1H, *J* = 4,1 Hz, Hₐᵣₒₘ), 8,03 (d, 2H, *J* = 8,3 Hz, Hₐᵣₒₘ), 7,92 (d, 1H, *J* = 8,0 Hz, Hₐᵣₒₘ), 7,86 (t, 1H, J = 8,0 Hz, Hₐᵣₒₘ), 7,70 (s, 1H, Hₐᵣₒₘ), 7,48 (d large, 2H, *J* = 8,3 Hz, Hₐᵣₒₘ), 7,35-7,31 (m, 1H, Hₐᵣₒₘ), 6,62 (s, 2H, =CH), 6,51 (sél, 1H, NH), 4,67 (sél, 2H, CH₂), 4,51 (sél, 1H, OH), 3,82 (sél, 1H, CH), 3,45-3,32 (m, 2H, CH₂), 2,90 (hept, 1H, *J* = 6,8 Hz, CH), 1,65-1,35 (m, 2H, CH₂), 1,23 (d, 6H, *J* = 6,8 Hz, 2 CH₃), 0,84 (t, 3H, *J* = 7,4 Hz, CH₃).

### Exemple 5 : (S) 2-(-1-Hydroxybut-2-ylamino)-8-isopropyl-4-[4-(pyridin-2-yl)benzylamino]pyrazolo[1,5-a]-1,3,5-triazine (4)

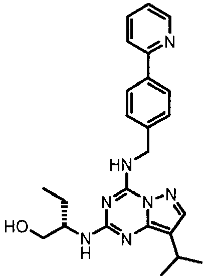

Selon les mêmes conditions conduisant à la préparation du composé **2,** le composé **4** est préparé à partir de **IIa.2** par réaction d'oxydation de l'atome de soufre puis introduction du (S)-(+)-2-aminobutanol commercial. Rdt = 48%. Mousse. ¹H RMN (300 MHz, CDCl₃): δ 8,69 (d, 1H, *J* = 4,1 Hz, Hₐᵣₒₘ), 7,96 (d, 2H, *J* = 7,9 Hz, Hₐᵣₒₘ), 7,78-7,69 (m, 2H, Hₐᵣₒₘ), 7,62 (s, 1H, Hₐᵣₒₘ), 7,42 (d, 2H, *J* = 7,9 Hz, Hₐᵣₒₘ), 7,26-7,21 (m, 1H, Hₐᵣₒₘ), 6,91 (sél, 1H, NH), 5,10 (s, 1H, H échangeable), 4,75 (d, 2H, *J* = 6,0 Hz, CH₂), 3,93 (sél, 1H, CH), 3,83-3,62 (m, 2H, CH₂), 3,02 (hept, 1H, *J* = 6,8 Hz, CH), 1,70-1,52 (m, 2H, CH₂), 1,27 (d, 6H, *J* = 6,8 Hz, 2 CH₃), 1,02 (t, 3H, *J=* 7,4 Hz, CH₃). SM (ESI): *m*/*z* 432 (MH⁺).

### Exemple 6 : Fumarate du (S) 2-(-1-hydroxybut-2-ylamino)-8-isopropyl-4-[4-(pyridin-2-yl)benzylamino]pyrazolo[1,5-a]-1,3,5-triazine (5)

Le produit **4** est traité par de l'acide fumarique dans une solution EtOH/Et₂O. Le sel de l'acide fumarique **5** cristallise dans le milieu réactionnel. PF = 175-177 °C. ¹H RMN (300 MHz, DMSO-*d₆*): δ 13,12 (sél, 2H, OH), 8,71 (sél, 1H, NH), 8,64 (d, 1H, *J* = 4,1 Hz, Hₐᵣₒₘ), 8,03 (d, 2H, *J* = 8,3 Hz, Hₐᵣₒₘ), 7,92 (d, 1H, *J* = 8,0 Hz, Hₐᵣₒₘ), 7,86 (t, 1H, J = 8,0 Hz, Hₐᵣₒₘ), 7,70 (s, 1H, Hₐᵣₒₘ), 7,48 (d, 2H, *J* = 8,3 Hz, Hₐᵣₒₘ), 7,35-7,31 (m, 1H, Hₐᵣₒₘ), 6,62 (s, 2H, =CH), 6,51 (sél, 1H, NH), 4,67 (sél, 2H, CH₂), 4,51 (sél, 1H. OH), 3,82 (sél, 1H, CH), 3,45-3,32 (m, 2H, CH₂), 2,90 (hept, 1H, *J* = 6,8 Hz, CH), 1,65-1,35 (m, 2H, CH₂), 1,23 (d, 6H, *J* = 6,8 Hz, 2 CH₃), 0,84 (t, 3H, *J* = 7,4 Hz, CH₃).

### Exemple 7 : (R) 2-(-1-Hydroxybut-2-ylamino)-8-isopropyl-4-[4-(thiophén-3-yl)benzylamino]pyrazolo[1,5-a]-1,3,5-triazine (6)

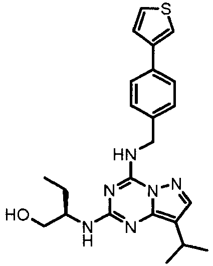

Selon les mêmes conditions conduisant à la préparation du composé **2**, le composé **6** est préparé à partir de **IIa.3** par réaction d'oxydation de l'atome de soufre puis introduction du (*R*)-(-)-2-aminobutanol commercial. Rdt = 50 %. Huile. ¹H RMN (300 MHz, CDCl₃): δ (s, 1H, Hₐᵣₒₘ), 7,57 (d, 2H, *J* = 8,3 Hz, Hₐᵣₒₘ), 7,44 (sél, 1H, Hₐᵣₒₘ), 7,40-7,34 (m, 4H, Hₐᵣₒₘ), 6,83 (sél, 1H, NH), 5,10 (sél, 1H, H échangeable), 4,72 (d, 2H, *J* = 6,0 Hz, CH₂), 4,00-3,92 (m, 1H, CH), 3,83 (d, 1H, *J* = 10,7 Hz, CH₂), 3,66 (dd, 1H, *J* = 7,3, 10,7 Hz, CH₂), 3,02 (hept, 1H, *J* = 6,8 Hz, CH), 1,70-1,52 (m, 2H, CH₂), 1,27 (d, 6H, *J* = 6,8 Hz, 2 CH₃), 1,03 (t, 3H, *J* = 7,4 Hz, CH₃). SM (ESI): *m*/*z* 437 (MH⁺).

### Exemple 8 : (R) 2-(-1-Hydroxybut-2-ylamino)-8-isopropyl-4-[4-(thiophén-3-yl)benzylamino]pyrazolo[1,5-a]-1,3,5-triazine (6)

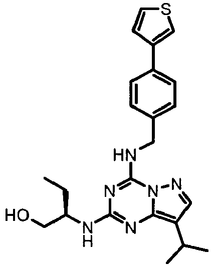

Dans un tube scellé, le composé **III**.**5** (180 mg, 0,41 mmol) est additionné à une solution dégasée de l'acide thiophén-3-ylboronique (79 mg, 0,62 mmol), de Pd(PPh₃)₄ (47 mg, 0,04 mmol) et de NaHCO₃ 2 M (2 mL) dans le toluène (10 mL). La solution finale est chauffée à 140 °C pendant une nuit. Après refroidissement et dilution avec de l'H₂O, la solution est extraite avec le CH₂Cl₂ (3 x 12 mL). La phase organique isolée est lavée par une solution de NaCl. Après séchage sur MgSO₄, le solvant est évaporé sous pression réduite. Le résidu est purifié par chromatographie flash (EP/AcOEt 9:1 à 6:4) pour donner **6** (90 mg, 50 %). Huile. ¹H RMN (300 MHz, CDCl₃): δ 7,61 (s, 1H, Hₐᵣₒₘ), 7,57 (d, 2H, *J* = 8,3 Hz, Hₐᵣₒₘ), 7,44 (sél, 1H, Hₐᵣₒₘ), 7,40-7,34 (m, 4H, Hₐᵣₒₘ), 6,80 (sél, 1H, NH), 5,11 (sél, 1 H, H échangeable), 4,72 (d, 2H, *J* = 6,0 Hz, CH₂), 4,00-3,92 (m, 1H, CH), 3,83 (d, 1H, *J* = 10,7 Hz, CH₂), 3,66 (dd, 1H, *J* = 7,3, 10,7 Hz, CH₂), 3,02 (hept, 1H, *J* = 6,8 Hz, CH), 1,70-1,52 (m, 2H, CH₂), 1,27 (d, 6H, *J* = 6,8 Hz, 2 CH₃), 1,03 (t, 3H, *J* = 7,4 Hz, CH₃). SM (ESI): *m*/*z* 437 (MH⁺).

### Exemple 9 : (S) 2-(-1-Hydroxybut-2-ylamino)-8-isopropyl-4-[4-(thiophén-3-yl)benzylamino]pyrazolo[1,5-a]-1,3,5-triazine (7)

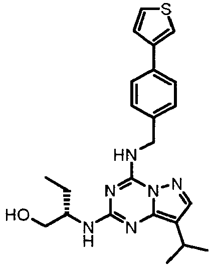

Selon les mêmes conditions conduisant à la préparation du composé 2, le composé **6** est préparé à partir de **IIa.3** par réaction d'oxydation de l'atome de soufre puis introduction du (*S*)-(+)-2-aminobutanol commercial. Rdt = 49 %. Huile. ¹H RMN (300 MHz, CDCl₃): δ 7,61 (s, 1H, Hₐᵣₒₘ), 7,57 (d, 2H, *J* = 8,3 Hz, Hₐᵣₒₘ), 7,44 (sél, 1H, Hₐᵣₒₘ), 7,40-7,34 (m, 4H, Hₐᵣₒₘ), 6,81 (sél, 1H, NH), 5,08 (sél, 1H, NH), 4,72 (d, 2H, *J* = 6,0 Hz, CH₂), 4,00-3,92 (m, 1H, CH), 3,83 (d, 1H, *J* = 10,7 Hz, CH₂), 3,66 (dd, 1H, *J* = 7,3, 10,7 Hz, CH₂), 3,02 (hept, 1H, *J* = 6,8 Hz, CH), 1,70-1,52 (m, 2H, CH₂), 1,27 (d, 6H, *J* = 6,8 Hz, 2 CH₃), 1,03 (t, 3H, *J* = 7,4 Hz, CH₃). SM (ESI): *m*/*z* 437 (MH⁺).

### Exemple 10: (S) 2-(1,2-Dihydroxypropan-3-ylamino)-8-isopropyl-4-[4-(thiophén-3-yl)benzylamino]pyrazolo[1,5-a]-1,3,5-triazine (8)

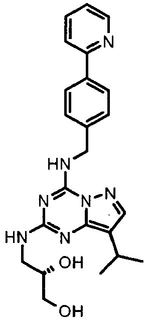

Selon les mêmes conditions conduisant à la préparation du composé **2**, le composé **8** est préparé à partir de **IIa.2** par réaction d'oxydation de l'atome de soufre puis introduction du (*S*)-3-amino-1,2-propanediol commercial. Rdt = 20%. Huile. ¹H RMN (300 MHz, CDCl₃): δ 8,69 (d, 1H, *J* = 4,5 Hz, Hₐᵣₒₘ), 7,98 (d, 2H, *J* = 8,3 Hz, Hₐᵣₒₘ), 7,79-7,70 (m, 2H, Hₐᵣₒₘ), 7,66 (s, 1H, Hₐᵣₒₘ), 7,45 (d, 2H, *J* = 8,3 Hz, Hₐᵣₒₘ), 7,27-7,22 (m, 1H, Hₐᵣₒₘ), 6,81 (sél, 1H, NH), 4,78 (d, 2H, *J* = 5,8 Hz, CH₂), 3,83-3,79 (m, 1H, CH), 3,65-3-3,55 (m, 4H, 2 CH₂), 3,03 (hept, 1H, *J* = 7,0 Hz, CH), 1,28 (d, 6H, *J* = 7,0 Hz, 2 CH₃). SM (ESI): *m*/*z* 434 (MH⁺).

### Exemple 11: Fumarate du (S) 2-(1,2-dihydroxypropan-3-ylamino)-8-isopropyl-4-[4-(pyridin-2-yl)benzylamino]pyrazolo[1,5-a]-1,3,5-triazine (9)

Le produit **8** est traité par de l'acide fumarique dans une solution EtOH/Et₂O. Le sel de l'acide fumarique **9** cristallise dans le milieu réactionnel. PF = 168-170 °C. ¹H RMN (300 MHz, CDCl₃ + 1 goutte DMSO-*d*₆): δ 8,64 (d, 1H, *J* = 3,8 Hz, Hₐᵣₒₘ), 7,93 (d, 2H, *J* = 8,1 Hz, Hₐᵣₒₘ), 7,74-7,65 (m, 2H, Hₐᵣₒₘ), 7,60 (s, 1H, Hₐᵣₒₘ), 7,42 (d, 2H, *J* = 8,1 Hz, Hₐᵣₒₘ), 7,26-7,18 (m, 1H, Hₐᵣₒₘ), 6,76 (s, 2H, =CH), 4,74 (d, 2H, *J* = 5,9 Hz, CH₂), 3,80-3,75 (m, 1H, CH), 3,56-3,45 (m, 4H, 2 CH₂), 2,99 (hept, 1H, *J* = 6,8 Hz, CH), 1,23 (d, 6H, *J* = 6,8 Hz, 2 CH₃).

### Exemple 12 : (R) 2-(1,2-Dihydroxypropan-3-ylamino)-8-isopropyl-4-[4-(pyridin-2-yl)benzylamino]pyrazolo[1,5-a]-1,3,5-triazine (10)

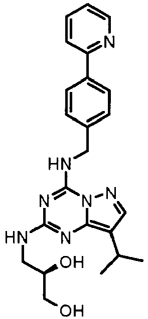

Selon les mêmes conditions conduisant à la préparation du composé **2**, le composé **10** est préparé à partir de **IIa.2** par réaction d'oxydation de l'atome de soufre puis introduction du (*R*)-3-amino-1,2-propanediol commercial. Rdt = 35%. Huile. ¹H RMN (300 MHz, CDCl₃): δ 8,69 (d, 1H, *J* = 4,5 Hz, Hₐᵣₒₘ), 7,98 (d, 2H, *J* = 8,3 Hz, Hₐᵣₒₘ), 7,79-7,70 (m, 2H, Hₐᵣₒₘ), 7,66 (s, 1H, Hₐᵣₒₘ), 7,45 (d, 2H, *J* = 8,3 Hz, Hₐᵣₒₘ), 7,27,28-7,22 (m, 1H, Hₐᵣₒₘ), 6,80 (sél, 1H, NH), 4,78 (d, 2H, *J* = 5,8 Hz, CH₂), 3,83-3,79 (m, 1H, CH), 3,65-3-3,55 (m, 4H, 2 CH₂), 3,03 (hept, 1H, J = 7,0 Hz, CH), 1,28 (d, 6H, J = 7,0 Hz, 2 CH₃). SM (ESI): *m*/*z* 434 (MH⁺).

### Exemple 13: Fumarate du (R) 2-(1,2-Dihydroxypropan-3-ylamino)-8-isopropyl-4-[4-(pyridin-2-yl)benzylamino]pyrazolo[1,5-a]-1,3,5-triazine (11)

Le produit **10** est traité par de l'acide fumarique dans une solution EtOH/Et₂O. Le sel de l'acide fumarique **11** cristallise dans le milieu réactionnel. PF = 168-170°C. ¹H RMN (300 MHz, CDCl₃ + 1 goutte DMSO-*d*₆): δ 8,64 (d, 1H, *J* = 3,8 Hz, Hₐᵣₒₘ), 7,93 (d, 2H, *J* = 8,1 Hz, Harom), 7,74-7,65 (m, 2H, Hₐᵣₒₘ), 7,60 (s, 1H, Hₐᵣₒₘ), 7,42 (d, 2H, *J* = 8,1 Hz, Hₐᵣₒₘ), 7,26-7,18 (m, 1H, Hₐᵣₒₘ), 6,76 (s, 2H, =CH), 4,74 (d, 2H, *J* = 5,9 Hz, CH₂), 3,80-3,75 (m, 1H, CH), 3,56-3,45 (m, 4H, 2 CH₂), 2,99 (hept, 1H, *J* = 6,8 Hz, CH), 1,23 (d, 6H, *J* = 6,8 Hz, 2 CH₃).

### Exemple 14 : (2R,3R) 2-(1,3-Dihydroxybut-2-ylamino)-8-isopropyl-4-[4-(pyridin-2-yl)benzylamino]pyrazolo[1,5-a]-1,3,5-triazine (12)

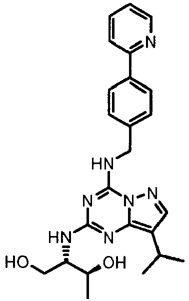

Selon les mêmes conditions conduisant à la préparation du composé **2**, le composé **12** est préparé à partir de **IIa.2** par réaction d'oxydation de l'atome de soufre puis introduction du L-thréoninol. Rdt = 43 %. Huile. ¹H RMN (300 MHz, CDCl₃): δ 8,69 (d, 1H, *J* = 4,7 Hz, Hₐᵣₒₘ), 7,97 (d, 2H, *J* = 8,3 Hz, Hₐᵣₒₘ), 7,78-7,69 (m, 2H, Hₐᵣₒₘ), 7,63 (s, 1H, Hₐᵣₒₘ), 7,45 (d, 2H, *J* = 8,3 Hz, Hₐᵣₒₘ), 7,26-7.21 (m, 1H, Hₐᵣₒₘ), 6,75 (sél, 1H, NH), 5,63 (d, 1H, *J* = 5,8 Hz, NH), 4,77 (d, 2H, *J* = 6,0 Hz, CH₂), 4,22-4,15 (m, 1H, CH), 3,97-3,84 (m, 3H, CH + CH₂), 3,01 (hept, 1H, *J* = 7,0 Hz, CH), 1,29 (d, 3H, *J* = 6,2 Hz, CH₃), 1,28 (d, 6H, *J* = 7,0 Hz, 2 CH₃). SM (ESI): *m*/*z* 448 (MH⁺).

### Exemple 15 : Fumarate du (2R,3R) 2-(1,3-dihydroxybut-2-ylamino)-8-isopropyl-4-[4-(pyridin-2-yl)benzylamino]pyrazolo[1,5-a]-1,3,5-triazine (13)

Le produit **12** est traité par de l'acide fumarique dans une solution EtOH/Et₂O. Le sel de l'acide fumarique **13** cristallise dans le milieu réactionnel. PF = 187-189 °C. ¹H RMN (300 MHz, DMSO-*d*₆): δ 13,12 (sél, 2H, OH), 8,77 (sél, 1H, NH), 8,64 (d, 1H, *J* = 4,5 Hz, Hₐᵣₒₘ), 8,03 (d, 2H, *J* = 8,1 Hz, Hₐᵣₒₘ), 7,94-7,82 (m, 2H, Hₐᵣₒₘ), 7,72 (s, 1H, Hₐᵣₒₘ), 7,49 (sél, 2H, Hₐᵣₒₘ), 7,35-7.31 (m, 1H, Hₐᵣₒₘ), 6,62 (s, 2H, 2 =CH), 6,06 (d, 1H, *J* = 8,7 Hz, NH), 4,73 (sél, 2H, CH₂), 4,00-3,89 (sél, 1H, CH), 3,89-3,76 (sél, 1H, CH), 3,58-3,40 (m, 2H, CH₂), 2,90 (hept, 1H, *J* = 7,0 Hz, CH), 1,22 (d, 6H, *J* = 5,8 Hz, 2 CH₃), 1,04 (sél, 3H, CH₃).

### Exemple 16 : (2S,3S) 2-(1,3-Dihydroxybut-2-ylamino)-8-isopropyl-4-[4-(pyridin-2-yl)benzylamino]pyrazolo[1,5-a]-1,3,5-triazine (14)

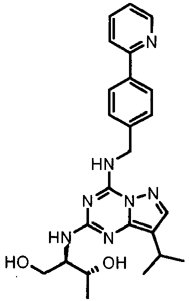

Selon les mêmes conditions conduisant à la préparation du composé **2**, le composé **12** est préparé à partir de **IIa.2** par réaction d'oxydation de l'atome de soufre puis introduction du D-thréoninol. Rdt = 42%. Huile. ¹H RMN (300 MHz, CDCl₃): δ 8,69 (d, 1H, *J* = 4,7 Hz, Hₐᵣₒₘ), 7,97 (d, 2H, *J* = 8,3 Hz, Hₐᵣₒₘ), 7,78-7,69 (m, 2H, Hₐᵣₒₘ), 7,63 (s, 1H, Hₐᵣₒₘ), 7,45 (d, 2H, *J* = 8,3 Hz, Hₐᵣₒₘ), 7,26-7.21 (m, 1H, Hₐᵣₒₘ), 6,71 (sél, 1H, NH), 5,62 (d, 1H, *J* = 6,6 Hz, NH), 4,77 (d, 2H, *J* = 6,0 Hz, CH₂), 4,22-4,15 (m, 1H, CH), 3,97-3,84 (m, 3H, CH + CH₂), 3,01 (hept, 1H, *J* = 7,0 Hz, CH), 1,29 (d, 3H, *J* = 6,2 Hz, CH₃), 1,28 (d, 6H, *J* = 7,0 Hz, 2 CH₃). SM (ESI): *m*/*z* 448 (MH⁺).

### Exemple 17: Fumarate du (2S,3S) 2-(1,3-dihydroxybut-2-ylamino)-8-isopropyl-4-[4-(pyridin-2-yl)benzylamino]pyrazolo[1,5-a]-1,3,5-triazine (15)

Le produit **14** est traité par de l'acide fumarique dans une solution EtOH/Et₂O. Le sel de l'acide fumarique **15** cristallise dans le milieu réactionnel. PF = 187-189 °C. ¹H RMN (300 MHz, DMSO-*d*₆): δ 13,12 (sél, 2H, OH), 8,75 (sél, 1H, NH), 8,64 (d, 1H, *J* = 4,5 Hz, Hₐᵣₒₘ), 8,03 (d, 2H, *J* = 8,1 Hz, Hₐᵣₒₘ), 7,94-7,82 (m, 2H, Hₐᵣₒₘ), 7,72 (s, 1H, Hₐᵣₒₘ), 7,49 (sél, 2H, Hₐᵣₒₘ), 7,35-7.31 (m, 1H, Hₐᵣₒₘ), 6,62 (s, 2H, 2 =CH), 6,05 (d, 1H, *J* = 8,7 Hz, NH), 4,67 (sél, 2H, CH₂), 4,00-3,89 (sél, 1H, CH), 3,89-3,76 (sél, 1H, CH), 3,58-3,40 (m, 2H, CH₂), 2,90 (hept, 1H, *J* = 7,0 Hz, CH), 1,22 (d, 6H, *J* = 5,8 Hz, 2 CH₃), 1,04 (sél, 3H, CH₃).

### Exemple 18: (2R,3R) 2-(1,3-Dihydroxybut-2-ylamino)-8-isopropyl-4-[4-(thiophén-3-yl)benzylamino]pyrazolo[1,5-a]-1,3,5-triazine (16)

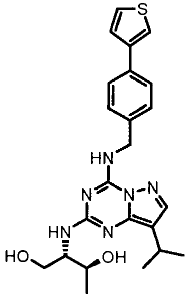

Selon les mêmes conditions conduisant à la préparation du composé **2**, le composé **6** est préparé à partir de **IIa.3** par réaction d'oxydation de l'atome de soufre puis introduction du L-thréoninol commercial. Rdt = 40 %. Huile. ¹H RMN (300 MHz, CDCl₃): δ 7,62 (s, 1H, Hₐᵣₒₘ), 7,56 (d, 2H, *J* = 8,3 Hz, Hₐᵣₒₘ), 7,45-7,43 (m, 1H, Hₐᵣₒₘ), 7,40-7,33 (m, 4H, Hₐᵣₒₘ), 6,83 (sél, 1H, NH), 5,83 (sél, 1H, NH), 4,71 (d, 2H, *J* = 5,7 Hz, CH₂), 4,22-4,15 (m, 1H, CH), 3,97-3,87 (m, 3H, CH + CH₂), 3,00 (hept, 1H, *J* = 6,8 Hz, CH), 1,28 (d, 3H, *J* = 6,2 Hz, CH₃), 1,27 (d, 6H, *J* = 6,8 Hz, 2 CH₃). SM (ESI): *m*/*z* 453 (MH⁺).

### Exemple 19 : 2-(1,3-Dihydroxyprop-2-ylamino)-8-isopropyl-4-[4-(pyridin-2-yl)benzylamino]pyrazolo[1,5-a]-1,3,5-triazine (17)

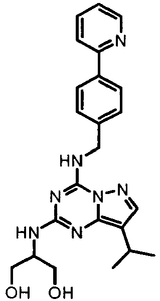

Selon les mêmes conditions conduisant à la préparation du composé **2**, le composé **17** est préparé à partir de **IIa.2** par réaction d'oxydation de l'atome de soufre puis introduction du serinol. Rdt = 30 %. Huile. ¹H RMN (300 MHz, CDCl₃): δ 8,68 (d, 1H, *J* = 4,4 Hz, Hₐᵣₒₘ), 7,96 (d, 2H, *J* = 8,3 Hz, Hₐᵣₒₘ), 7,79-7,69 (m, 2H, Hₐᵣₒₘ), 7,64 (s, 1H, Hₐᵣₒₘ), 7,45 (d, 2H, *J* = 8,3 Hz, Hₐᵣₒₘ), 7,26-7,21 (m, 1H, Hₐᵣₒₘ), 6,80 (sél, 1H, NH), 5,71 (sél, 1H, NH), 4,77 (d, 2H, *J* = 5,9 Hz, CH₂), 4,08-4,95 (m, 1H, CH), 3,92-3,80 (m, 4H, 2 CH₂), 3,02 (hept, 1H, *J* = 7,0 Hz, CH), 1,28 (d, 6H, *J* = 7,0 Hz, 2 CH₃). SM (ESI): *m*/*z* 434 (MH⁺).

### Exemple 20 : Fumarate du 2-(1,3-dihydroxyprop-2-ylamino)-8-isopropyl-4-[4-(pyridin-2-yl)benzylamino]pyrazolo[1,5-a]-1,3,5-triazine (18)

Le produit **17** est traité par de l'acide fumarique dans une solution EtOH/Et₂O. Le sel de l'acide fumarique **18** cristallise dans le milieu réactionnel. PF = 179-181°C. ¹H RMN (300 MHz, DMSO-*d*₆): δ 13,11 (sél, 2H, OH), 8,76 (sél, 1H, NH), 8,64 (d, 1H, *J* = 4,5 Hz, Hₐᵣₒₘ), 8,03 (d, 2H, *J* = 8,1 Hz, Hₐᵣₒₘ), 7,94-7,82 (m, 2H, Hₐᵣₒₘ), 7,72 (s, 1H, Hₐᵣₒₘ), 7,49 (sél, 2H, Hₐᵣₒₘ), 7,35-7.31 (m, 1H, Hₐᵣₒₘ), 6,63 (s, 2H, 2 =CH), 6,33 (d, 1H, *J* = 6,0 Hz, NH), 4,67 (d, 2H, *J* = 6,0 Hz, CH₂), 3,98-3,83 (m, 1H, CH), 3,58-3,42 (m, 4H, 2 CH₂), 2,91 (hept, 1H, *J* = 7,0 Hz, CH), 1,23 (d, 6H, *J* = 7,0 Hz, 2 CH₃).

### Exempte 21 : 2-(1,3-Dihydroxyprop-2-ylamino)-8-isopropyl-4-[4-(pyridin-3-yl)benzylamino]pyrazolo[1,5-a]-1,3,5-triazine (19)

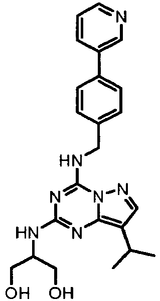

### a. 8-Isopropyl-2-(méthylsulfoxyl)-4-[[4-(pyridin-3-yl)benzylamino]pyrazolo [1,5-a]-1,3,5-triazine (IVa)

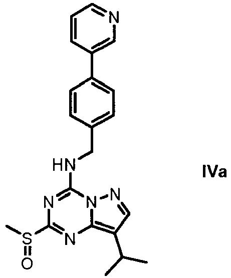

Ce composé est oxydé par l'acide méta-chloroperbenzoïque selon le protocole suivant. Une solution de **IIa.4** (5,22 g, 13,3 mmol) dissout dans 277 mL de dichlorométhane est refroidie à 0 °C puis additionnée de 3,46 g d'acide méta-chloroperbenzoïque à 80 %. Après 1 h d'agitation, le mélange est lavé par une solution de carbonate de sodium. Le produit cristallise par concentration. Le précipité (**IVa**) est lavé par un peu d'éther. Rdt = 84%. ¹H RMN (400 MHz, DMSO-d₆): δ 8,85 (d, 1H, Hₐᵣₒₘ), 8,60 (d, 1H, Hₐᵣₒₘ), 7,95 (s, 1H, Hₐᵣₒₘ), 7,85 (d, 1H, Hₐᵣₒₘ), 7,60 (d, 2H, Hₐᵣₒₘ), 7,50 (d, 2H, Hₐᵣₒₘ), 7,40 (m, 1H, Hₐᵣₒₘ), 7,10 (m, 1H, Hₐᵣₒₘ), 4,98 (d, 2H, CH₂), 3,32 (hept, 1H, CH); 2,95 (s, 3H, CH₃), 1,35 (d, 6H, 2 CH₃).

### b. Obtention du 2-(1,3-dihydroxyprop-2-ylamino)-8-isopropyl-4-[4-(pyridin-3-yl)benzylamino]pyrazolo[1,5-a]-1,3,5-triazine (19)

Le sulfoxyde (**IVa**) à hauteur de 0,2 g est chauffé à 140 °C pendant 4 h en présence de sérinol (2-aminopropan-1,3-diol), dans une teneur de 0,36 g (3,9 mmol) pendant 12 h à 140 °C pour former le produit **19** qui est isolé par cristallisation dans un mélange d'acétate d'éthyle et d'éther éthylique. Rdt = 75%. ¹H RMN (400 MHz, CDCl₃): δ 8,70 (d, 1H, Hₐᵣₒₘ), 8,55 (d, 1H, Hₐᵣₒₘ), 7,82 (d, 1H, Hₐᵣₒₘ), 7,68(s, 1H, Hₐᵣₒₘ), 7,35 (m, 3H, Hₐᵣₒₘ), 7,58 (t, 1H, NH), 7,10 (d, 2H, Hₐᵣₒₘ), 5,80 (d, 1H, NH), 4,35 (d, 2H, CH₂), 3,85 (m, 1H), 3,75 (m, 1H), 2,98 (hept, 1H, CH); 1,28 (d, 6H, 2CH₃). SM (ESI): *m*/*z* 434 (MH⁺).

### Exemple 22 : (S) 2-(-1-Hydroxybut-2-ylamino)-8-isopropyl-4-[4-(pyridin-3-yl)benzylamino]pyrazolo[1,5-a]-1,3,5-triazine (20)

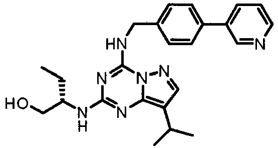

Le produit **20** est préparé dans les mêmes conditions que **19** en chauffant 0,2 g de sulfoxyde **IVa** avec 0,368 mL de (*S*)-(+)-2-aminobutanol commercial. Rdt = 91 %. ¹H RMN (400 MHz, CDCl₃): δ 8,85 (d, 1H, Hₐᵣₒₘ), 8,60 (d, 1H, Hₐᵣₒₘ), 7,85 (dd, 1H, Hₐᵣₒₘ), 7,65 (s, 1H, Hₐᵣₒₘ H), 7,55 (d, 2H, Hₐᵣₒₘ), 7,45 (d, 2H, Hₐᵣₒₘ), 7,40 (m, 1H, Hₐᵣₒₘ), 6,75 (t, 1H, .NH), 5,00 (d, 1H, NH), 4,75 (d, 2H, CH₂), 3,95 (m, 1H), 3,85 (m, 1H), 3,65 (m, 1H), 3,04 (hept, 1H, CH), 1,65 (2H, CH₂), 1,25 (d, 6H, 2 CH₃), 1.03 (t, 3H, CH₃). ). SM (ESI): *m*/*z* 432 (MH⁺).

### Exemple 23: (2S,3S) 2-(1,3-Dihydroxybut-2-ylamino)-8-isopropyl-4-[4-phényl(pyridin-3-yl)méthylamino]pyrazolo[1,5-a]-1,3,5-triazine (21)

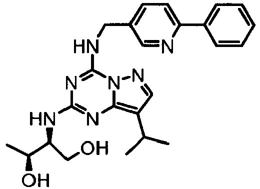

### a) 2-(Méthylsulfoxyl)-8-isopropyl-4-[[4-phényl(pyridin-3-yl)méthylamino]pyrazolo[1,5-a]-1,3,5-triazine (IVb).

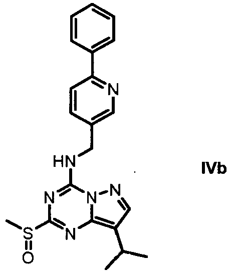

L'oxydation est réalisée comme pour la préparation de **IVa** en agitant à 0 °C 4,76 g de **IIa.5** dans 259,8 mL de CH₂Cl₂ avec 2 g d'acide méta-chloroperbenzoïque. Le sulfoxyde **IVb** est obtenu avec un rendement de 87 %. ¹H RMN (400 MHz, CDCl₃): δ 8,78 (s, 1H, Hₐᵣₒₘ), 8,00 (d, 2H, Hₐᵣₒₘ), 7,95 (s, 1H, Hₐᵣₒₘ), 7,85 (d, 1H, Hₐᵣₒₘ), 7,75 (d, 1H, Hₐᵣₒₘ), 7,45 (m, 3H, Hₐᵣₒₘ), 4,95 (d, 2H, CH₂), 3,42 (hept, 1H, CH), 2,95 (s, 3H, CH₃), 1,3 (d, 6H, 2 CH₃).

### b) Préparation du (2S,3S) 2-(1,3-dihydroxybut-2-ylamino)-8-isopropyl-4-[4-phényl(pyridin-3-yl)méthylamino]pyrazolo[1,5-a]-1,3,5-triazine (21)

Le produit **21** est préparé comme le produit **20** par chauffage à 140° de 0,2 g **IVb** avec 0,413 g de (2S,3S)-thréoninol. Après refroidissement, le produit **21** est extrait par l'acétate d'éthyle et purifié sur une colonne de silice avec l'acétate d'éthyle 100 % comme éluant. Rdt = 82%. ¹H RMN (400 MHz, CDCl₃): δ 8,72 (s, 1H, Hₐᵣₒₘ), 7,95 (d, 2H, Hₐᵣₒₘ), 7,70 (m, 2H), 7,62 (s, 1H, Hₐᵣₒₘ), 7,45 (m, 3H, Hₐᵣₒₘ), 6.85 (sél, 1H, NH), 6,56 (d, 1H), 4,72 (d, 2H, CH₂), 4,20 (m, 1H, CH), 3,80 (m, 3H, CH₂OH) 2,99 (hept, 1H, CH), 1,30 (m, 9H, 3 CH₃). SM (ESI): *m*/*z* 448 (MH⁺).

### Exemple 24 : (S) 2-(-1-Hydroxybut-2-ylamino)-8-isopropyl-4-[4-phényl(pyridin-3-yl)méthylamino]pyrazolo[1,5-a]-1,3,5-triazine (22)

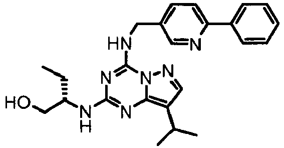

Ce produit est obtenu par chauffage de 0,3 g sulfoxyde **IVb** à 140 °C pendant 4 h de 0,553 mL de (*S*)-(+)-2-aminobutanol commercial. Après 4 h le produit est isolé par extraction avec l'acétate d'éthyle et purifié sur colonne de silice (éluant: AcOEt/CH₂Cl₂ 1:1). Rdt = 80%. ¹H RMN (400 MHz, CDCl₃): δ 8,70 (d, 1H, Hₐᵣₒₘ), 8,00 (d, 2H, Hₐᵣₒₘ), 7,70 (m, 2H, Harom), 7,62 (s, 1H, Hₐᵣₒₘ), 7,50 (m, 3H, Hₐᵣₒₘ), 6,90 (br s, 1H), 5,05 (d, 1H, NH), 4,75 (d, 2H, CH₂), 3,90 (m, 1H, CH), 3,80 (m, 1H, CH₂), 3,60 (m, 1H, CH₂OH), 3,00 (hept, CH), 1,60 (m, 2H, CH₂), 1,25 (d, 6H, 2 CH₃), 1,00 (t, 3H, CH₃). SM (ESI): *m*/*z* 432 (MH⁺).

### Exemple 25 : (2R,3R) 2-(1,3-Dihydroxybut-2-ylamino)-8-isopropyl-4-[4-phényl(pyridin-3-yl)méthylamino]pyrazolo[1,5-a]-1,3,5-triazine (23)

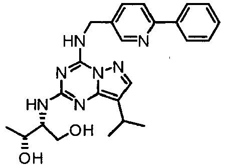

Ce composé est obtenu à partir du sulfoxyde **IVb** en utilisant le (*2R*,*3R*)-thréoninol selon un protocole identique à celui utilisé dans les exemples précédents. Le produit **23** est purifié par chromatographie sur colonne de silice (éluant: AcOEt/CH₂Cl₂ 8:2). Rdt = 91%. ¹H-RMN (400 MHz, CDCl₃) δ 8,72 (s, 1H, Hₐᵣₒₘ), 7,95 (d, 2H, Hₐᵣₒₘ), 7,62 (s, 1H, Hₐᵣₒₘ), 7,70 (m, 2H, Hₐᵣₒₘ), 7,45 (m, 3H, Hₐᵣₒₘ), 6,85 (s large, 1H, NH), 6,56 (d, 1H), 4,72 (d, 2H, CH₂), 4,20 (m, 1H, CH), 3,80 (m, 3H, CH₂), 2,99 (hept, 1H, CH), 1,30 (m, 9H, 3 CH₃). SM (ESI): *m*/*z* 448 (MH⁺).

### Exemple 26 : 2-(1,3-Dihydroxyprop-2-ylamino)-8-isopropyl-4-[4-phényl(pyridin-3-yl)méthylamino]pyrazolo[1,5-a]-1,3,5-triazine (24)

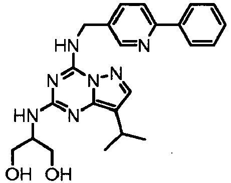

Ce produit est préparé par chauffage du sulfoxyde **IV b** avec le sérinol selon les conditions décrites dans l'exemple 23. Le produit est purifié sur colonne de silice(éluant: AcOEt). Rdt = 78%. ¹H RMN (400 MHz, CDCl₃): δ 8,71 (s, 1H, Hₐᵣₒₘ), 7,95 (d, 2H, Hₐᵣₒₘ), 7,73 (m, 2H, Hₐᵣₒₘ), 7,64 (s, 1H, Hₐᵣₒₘ), 7,47 (m, 3H, Hₐᵣₒₘ), 6,98 (sél, 1H), 5,71 (d,1H, NH), 4,75 (d, 2H, CH₂), 4,06 (m, 1H,CH); 3,84 (m, 4H, 2 CH₂), 2,99 (hept, 1H), 1,26 (d, 6H, 2 CH₃).

### Exemple 27 : (R) 2-[[8-isopropyl-4-[[4-(2-pyridyl)phenyl]methylamino]pyrazolo[1,5-a][1,3,5]triazin-2-yl]amino]-4-methyl-pentan-1-ol ou (R) 2-(1-hydroxy-4-méthylpent-2-ylamino)-8-isopropyl-4-[4-phényl(pyridin-3-yl)méthylamino]pyrazolo[1,5-a]-1,3,5-triazine (25)

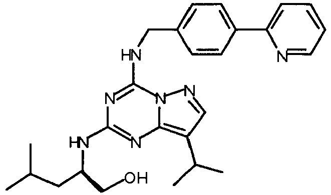

Ce produit est préparé par chauffage du sulfoxyde **IVA** (0,2 g) avec le D-valinol (0,406 g) selon les conditions décrites dans l'exemple 23. Le produit est purifié sur colonne de silice (éluant: AcOEt/cyclohexane 1:1). Rdt = 76%. ¹H NMR (400 MHz, CDCl₃): δ 8,71 (s, 1H, Hₐᵣₒₘ), 7,99 (d, 2H, Hₐᵣₒₘ), 7,75 (m, 2H, Hₐᵣₒₘ), 7,63 (s, 1H, Harom), 7,47 (d, 2H, Hₐᵣₒₘ), 6,72 (sél, 1H), 4,95 (d, 1H, NH), 4,75 (m, 2H, CH₂), 4,10 (m, 1H), 3,80 m, 1H), 3,60(m, 2H), 3,03 (hept, 1H, CH), 1,40-1,80 (m 3H) 1,30 (d 6H, 2 CH₃), 0.93(m, 6H, 2CH₃).

### Exemple 28 : (S) 2-[[8-isopropyl-4-[[4-(2-pyridyl)phenyl] methylamino]pyrazolo[1,5-a][1,3,5]triazin-2-yl]amino]-4-methyl-pentan-1-ol ou (S) 2-(1-hydroxy-4-méthylpent-2-ylamino)-8-isopropyl-4-[4-phényl(pyridin-3-yl)méthylamino]pyrazolo[1,5-a]-1,3,5-triazine (26)

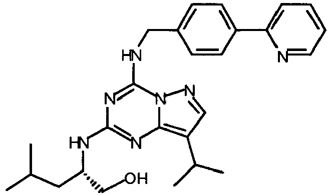

Ce produit est préparé par chauffage du sulfoxyde **IVa** (0,2 g) avec le L-valinol (0,406 g) selon les conditions décrites dans l'exemple 23.. Le produit est purifié sur colonne de silice (éluant: AcOEt/cyclohexane 1:1). Rdt = 76%. ¹H NMR (400 MHz, CDCl₃): δ 8,71 (d, 1H, Hₐᵣₒₘ), 7,99 (d, 2H, Hₐᵣₒₘ), 7,75 (m, 2H, Hₐᵣₒₘ), 7,63 (s, 1H, Hₐᵣₒₘ), 7,47 (d, 2H, Hₐᵣₒₘ), 6,72 (sél, 1H), 4,95 (d, 1H, NH), 4,75 (m, 2H, CH₂), 4,10 (m, 1H), 3,80 (m, 1H), 3,60 (m, 2H), 3,03 (hept, 1H, CH), 1,40-1,80 (m 3H) 1,31 (d 6H, 2 CH₃), .0.93(m, 6H, 2CH₃).

### Exempte 29 : (S) 2-(1-Hydroxy-3,3-diméthylbut-2-ylamino)-8-isopropyl-4-[4-(pyridin-2-yl)benzylamino]pyrazolo[1,5-a]-1,3,5-triazine (27)

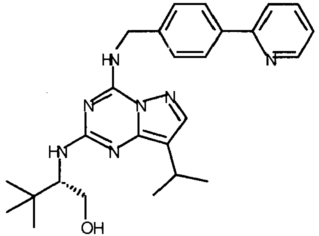

### a) 2-(Méthylsulfoxyl)-8-isopropyl-4-[[4-(pyridin-2-yl)benzylamino]pyrazolo[1,5-a]-1,3,5-triazine (IVc)

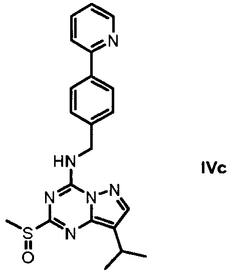

L'oxydation est réalisée comme pour la préparation **IVb** en agitant à 0 °C 4,76 g de **IIa2** dans 259,8 mL de CH₂Cl₂ avec 2 g d'acide méta-chloroperbenzoïque. Rdt = 90%. ¹H NMR (400 MHz, CDCl₃): δ 8.70 (d, 1H, Hₐᵣₒₘ), 8,05 (d, 2H, Hₐᵣₒₘ), 7,95 (s, 1H, Hₐᵣₒₘ), 7,75 (m, 2H, Hₐᵣₒₘ), 7,50 (d, 2H, Hₐᵣₒₘ), 7,22 (m, 1H, Hₐᵣₒₘ), 7,10 (t, 1H, NH), 4,92 (d, 2H, CH₂), 3,32 (hept, 1H, CH), 2,92 (s, 3H, CH₃), 1,3 (d, 6H, 2 CH₃).

### b) 2-(1-Hydroxy-3,3-diméthylbut-2-ylamino)-8-isopropyl-4-[4-(pyridin-2-yl)benzylamino]pyrazolo[1,5-a]-1,3,5-triazine (27).

Le dérivé **27** est préparé par chauffage du produit **IVc** 0,2 g avec le 0,512 g de *(S)*-tert leucinol après 4 h de chauffage à 140 °C. Après extraction par l'AcOEt et lavage par l'eau, le produit formé cristallise par concentration du solvant. ¹H NMR (400 MHz, CDCl₃): δ 8,00 (d, 2H, Hₐᵣₒₘ), 7,75 (m, 2H, Hₐᵣₒₘ), 7,62 (s, 1H, Hₐᵣₒₘ), 7,45 (d, 2H, Hₐᵣₒₘ), 7,25 (1H, Hₐᵣₒₘ), 6,75 (s, 1H), 5,10 (m, 1H), 4,70 (m, 2H), 4,04 (m, 1 H), 3,92 (m, 1H), 3,62 (m, 1H), 3,03 (hept, CH), 1,30 (d, 6H, 2 CH₃), 1,00 (s, 9H, 3 CH₃).

### Exempte 30 : (S) 2-(1-Hydroxy-3-méthylbut-2-ylamino)-8-isopropyl-4-[4-(pyridin-2-yl)benzylamino]pyrazolo[1,5-a]-1,3,5-triazine (28)

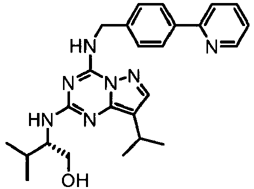

Le dérivé **28** est préparé comme dans l'exemple **29** par chauffage du produit **IVc** avec le (*S*)-(*L*)-valinol. Rdt = 72%. ¹H NMR (400 MHz, CDCl₃): δ 8,70 (d, 1H, Hₐᵣₒₘ), 7,98 (d, 2H, Hₐᵣₒₘ), 7,75 (m, 2H, Hₐᵣₒₘ), 7,62 (s, 1H, Hₐᵣₒₘ), 7,45 (d, 2H, Hₐᵣₒₘ), 7,25 (m, 1H, Hₐᵣₒₘ), 6,80 (s, 1H, NH), 5,10 (d, 1H, NH), 4,80 (d, 2H, CH₂), 3,80 (m, 2H, CH₂), 3,0X (CH, 1H), 1,80 (m, 1H, CH), 1,25 (d, 6H, 2 CH₃), 1,0X (d, 6H, 2 CH₃),

### Exemple 31 : 2-[4-[1-[8-isopropyl-4-[[4-(pyridin-2-yl)phényl]méthylamino] pyrazolo[1,5-a]-1,3,5-triazin-2-yl]pipéridin-4-yl]pipéridin-1-yl]éthanol (29)

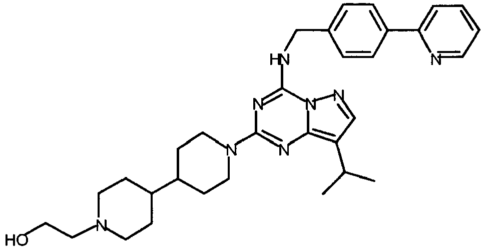

Le dérivé **29** est préparé comme dans l'exemple 29 par chauffage du produit **IVc** avec la bispipéridine-éthanol décrite par P. Leon, C. Garbay-Jaureguiberry, B. Lambert, J. B. Le Pecq, B. P. Roques. Asymmetrical bisintercalators as potential antitumor agents J. Med. Chem., 1988, 31 (5), pp 1021-1026

Rdt = 78%. ¹H NMR (400 MHz, CDCl₃): δ 8,68 (d, 1H, Hₐᵣₒₘ), 7,98 (d, 2H Hₐᵣₒₘ), 7,75 (m, 2H, Hₐᵣₒₘ), 7,60 (s, 1H, Hₐᵣₒₘ), 7,48 (d, 2H, Hₐᵣₒₘ), 7,22 (m, 1H, Hₐᵣₒₘ), 6,64 (t, 1H, NH), 4,85 (s, 1H), 4,8 (s, 1H), 4,70 (d, 2H, CH₂), 3,80 (m, 2H, CH₂), 3,60 (m, 2H, CH₂), 3,00 (d, 2H, CH₂), 2,74 (t, 2H, CH₂), 2,55 (m, 1H), 2 (t, 2H, CH₂), 1,70 (m, 4H, CH₂), 1,40 (m, 2H, CH₂), 1,30 (d, 6H, 2 CH₃), 1,15 (m, 2H).

### Exemple 32: 2-[4-[8-isopropyl-4-[[4-(pyridin-2-yl)phényl]méthylamino] pyrazolo[1,5-a]-1,3,5-triazin-2-yl]pipérazin-1-yl]éthanol (30)

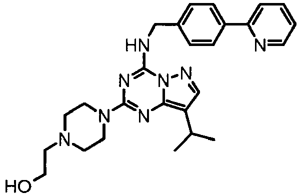

Le produit **30** est préparé comme dans l'exemple **29** par chauffage du produit **IVc** avec la 2-pipérazinyléthanol. Rdt = 65%. ¹H NMR (400 MHz, CDCl₃): δ 8,70 (d, 1H, Hₐᵣₒₘ), 7,98 (d, 2H, Hₐᵣₒₘ), 7,72 (m, 2H, Hₐᵣₒₘ), 7,65 (s, 1H, Hₐᵣₒₘ), 7,48 (d, 2H, Hₐᵣₒₘ), 7,25 (m, 1H, Hₐᵣₒₘ), 6,70 (t, 1H, NH), 4,78 (d, 2H, CH₂), 3,83 (m, 4H, CH₂), 3,65 (m, 2H, CH₂), 3,05 (hept, 1H, CH), 2,52 (m, 2H, CH₂) 2,48 (m, 4H, CH₂), 1,30 (d, 6H, 2 CH₃).

### Exemple 33: (R) 2-(1,2-Dihydroxypropan-3-ylamino)-8-éthyl-4-[4-(pyridin-2-yl)benzylamino]pyrazolo[1,5-a]-1,3,5-triazine (31)

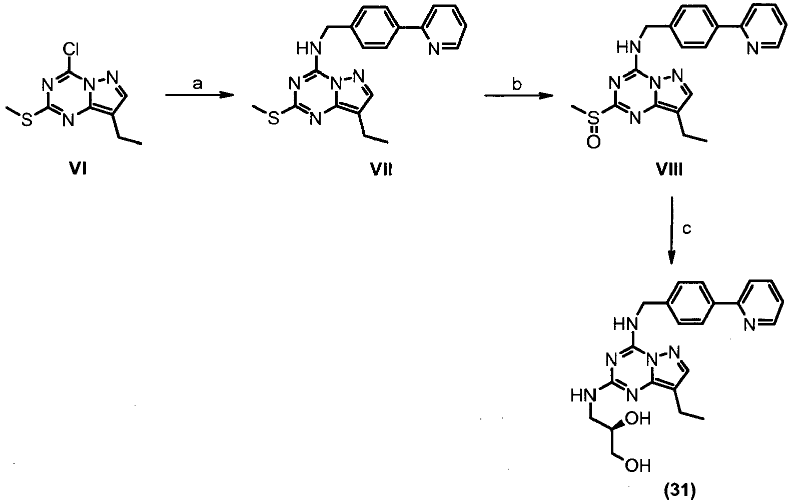

Le produit **31** est préparé à partir de l'intermédiaire **VI** obtenu comme décrit par Z Nie, CPerretta, Ph Erickson, S Margosiak, Ji Lu, A Averill, Rt Almassy, Shaosong hu . Structure-based design and synthesis of novel macrocyclic pyrazolo[1,5-a] [1,3,5]triazine compounds as potent inhibitors of protein kinase CK2 and their anticancer activities Bioorganic & Medicinal Chemistry Letters, Volume 18, Issue 2, 15 January 2008, Pages 619-623*.*

L'intermédiaire **VIII** est caractérisé par RMN. ¹H RMN (400 MHz, CDCl₃): δ 8,70 (d, 1H, Hₐᵣₒₘ), 8.00 (d, 2H, Hₐᵣₒₘ), 7,75 (m, 2H, Hₐᵣₒₘ), 7,50 (d, 2H, Hₐᵣₒₘ), 7,26 (s, 1H, Hₐᵣₒₘ), 6,80 (t, 1H, NH), 4,85 (d, 2H, CH₂), 2,68(q, 2H, CH₂), 2,55 (s, 3H, CH₃), 1,25 (t, 3H, CH₃).

Le produit **31** est préparé par chauffage à 140 °C de l'intermédiaire **VIII**, dans les conditions de l'exemple 23, avec le (*R*)-3-amino-1,2-propanediol commercial. Rdt = 76 %. ¹H NMR (400 MHz, CDCl₃): δ 8,70 (d, 1H, Hₐᵣₒₘ), 8,00 (d, 2H, Hₐᵣₒₘ), 7,75 (m, 2H, Hₐᵣₒₘ), 7,66 (s, 1H, Hₐᵣₒₘ), 7,48 (d, 2H, Hₐᵣₒₘ), 7,82 (sél, 1H, NH), 7,25 (m, 1H), 5,32 (sél, 1H), 4,75 (d, 2H, CH₂), 3,80 (m, 1H, CH), 3,60 (m, 4H, 2 CH₂), 2,55 (q, 2H, CH₂), 1,21 (t, 3 H, CH₃).

### Exemple 34 : préparation de l'ester 33 à partir de produit 31

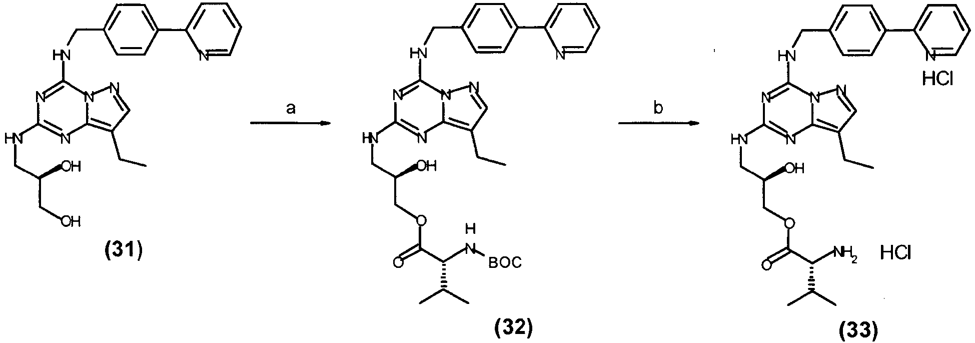

### a) Estérification par la D-BocValine.

Une solution de 0,7 g de D-BocValine et de 0,49 g d'hydroxybenzotriazole (HOBt) dans 10 mL d'AcOEt est refroidie sous agitation à 0 °C. A cette solution est ajoutée 0,763 g, de dicyclohexylcarbodiimide (DCC). Le mélange est alors agité 2 h à température ambiante puis filtré. Le précipité (dicyclohexylurée) est rincé par 3 mL d'AcOEt. Le filtrat est immédiatement ajouté à une solution de 0,73 g de **31** et de triéthylamine (1,5 mL) dans le tétrahydrofuranne (THF, 15 mL). Le mélange est agité 24 h à température ambiante puis concentré sous vide, repris par de l'AcOEt. La phase organique est lavée par une solution d'acide citrique puis par une solution de carbonate de sodium et par de l'eau. La phase organique est séchée puis évaporée sous vide. Le résidu contient l'ester pratiquement pur qui peut être purifié sur colonne de silice (éluant: AcOEt/cyclohexane/THF/Et₃N 45:50:4:1). L'ester **32** est obtenu avec un rendement de 75%. Dans cette réaction d'estérification, bien que les alcools secondaires puissent également être estérifiés dans des conditions similaires, on constate de manière inattendue, la formation exclusive de l'ester de l'alcool primaire.

### b) Obtention du sel 33.

A l'ester **32** en solution dans de l'éther éthylique, on ajoute une solution d'acide chlorhydrique anhydre (2 M) dans l'éther éthylique. Après 30 min à température ambiante, le précipité (**33**) est filtré, lavé à l'éther et séché sous vide. Rdt = 90%. ¹H NMR (400 MHz, DMSO-*d₆*): δ 10,05 (t, 1H), 8,60 (d, 1H), 8,55 (sél, 1H), 8,20 (m, 2H, Hₐᵣₒₘ), 8,05 (d, 1H, Hₐᵣₒₘ), 7,85 (m, 2H, Hₐᵣₒₘ), 7,60 (t, 1H, Hₐᵣₒₘ), 7,40 (m, 3H, Hₐᵣₒₘ), 4,50 (d, 2H), 3,95 (m, 1H), 3,80 (m, 1H), 3,70 (m, 1H), 3,40 (m, 1H), 2,00 (m, 2H), 0,90 (t, 3H, CH₃), 0,65 et 0.70 (2 d, 6H, 2 CH₃).

### Exemple 35: (S) 2-(1,2-Dihydroxypropan-3-ylamino)-8-éthyl-4-[4-(pyridin-2-yl)benzylamino]pyrazolo[1,5-a]-1,3,5-triazine (34).

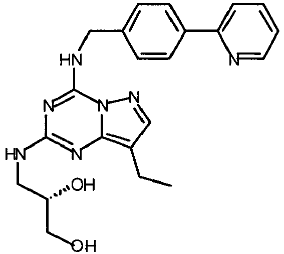

Ce produit est préparé comme son énantiomère **31** (exemple 34). Dans les tests sur les 4 kinases essayées le dérivé **34** se montre plus actif que le dérivé **31.**

### Exemple 36 : (S) 8-Ethyl-2-(-1-hydroxybut-2-ylamino)-4-[4-(pyridin-2-yl)benzylamino]pyrazolo[1,5-a]-1,3,5-triazine (35)

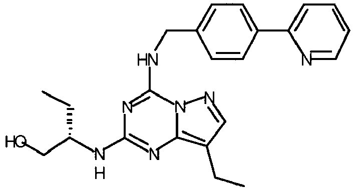

Le produit **31** est préparé par chauffage à 140°C de l'intermédiaire **VIII*,*** dans les conditions de l'exemple 23, avec le (*S*)-(+)-2-aminobutanol commercial.

¹H NMR (400 MHz, CDCl₃): δ 8.70 (d, 1H) ; 8.00(d, 2H) ; 7.75(m, 2H) ; 7,64(s, 1H); 7.48(m, 2H); 7,25(m, 1H); 4.80(m, 2H); 3,95(m, 1H); 3,80(m, 1H); 3,63(m, 1H); 1,60(m, 1H); 1.22(m, 5H).

### Exemple 37 : (R) 8-Ethyl-2-(-1-hydroxybut-2-ylamino)-4-[4-(pyridin-2-yl)benzylamino]pyrazolo[1,5-a]-1,3,5-triazine (36)

Le produit **36** est préparé par chauffage à 140 °C de l'intermédiaire **VIII**, dans les conditions de l'exemple 23, avec le (*R*)-(-)-2-aminobutanol commercial.

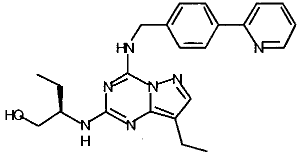

¹H NMR (400 MHz, CDCl₃): δ: 8.70 (d, 1H) ; 8.00(d, 2H) ; 7.75(m, 2H) ; 7,64(s, 1H); 7.48(m, 2H); 7,25(m, 1H); 4.80(m, 2H); 3,95(m, 1H); 3,80(m, 1H); 3,63(m, 1H); 1,60(m, 1H); 1.22(m, 5H).

### Exemple 38 : 2-(1,3-Dihydroxyprop-2-ylamino)-8-éthyl-4-[4-(pyridin-2-yl)benzylamino]pyrazolo[1,5-a]-1,3,5-triazine (37)

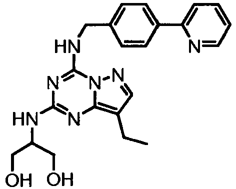

Le produit **37** est préparé par chauffage à 140°C de l'intermédiaire **VIII**, dans les conditions de l'exemple 23, avec le sérinol.

### Exemple 39 : 2-[(2S)-1-[8-éthyl-4-[[4-(pyridin-2-yl)phényl]méthylamino] pyrazolo[1,5-a]-1,3,5-triazin-2-yl]pipéridin-2-yl]éthanol (38).

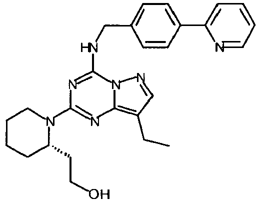

Le produit **38** est préparé par chauffage à 140 °C de l'intermédiaire **VIII**, dans les conditions de l'exemple 23, avec le (*S*) 2-(pipéridin-2yl)éthanol.. Rdt = 75%. ¹H NMR (400 MHz, CDCl₃): 8,64 (d, 1H, Haro), 7.92 (d, 2H, Hₐᵣₒₘ), 7,70 (m, 2H, Hₐᵣₒₘ), 7,54 (s, 1H, Hₐᵣₒₘ), 7,42 (d, 2H, Hₐᵣₒₘ), 4,95(m, 1H), 4,79 (m, 1H), 4,75 (d, 2H), 3,55 (d, 1H), 3,28 (t, 1H), 2,74 (t, 1H), 2,47 (q, 2H), 2,03(t, 1H); 1,40-1,80(m, piperidine) 1,14 (t, 3H, CH₃).

### Exemple 40 : 2-[[8-ethyl-4-[[4-(2-pyridyl)phenyl]methylamino]-pyrazolo[1,5-a][1,3,5]triazin-2-yl]-(2-hydroxyethyl)amino]ethanol (39).

Le produit **39** est préparé par chauffage à 140 °C de l'intermédiaire, **VIII**, dans les conditions de l'exemple 23, avec la diéthanolamine.

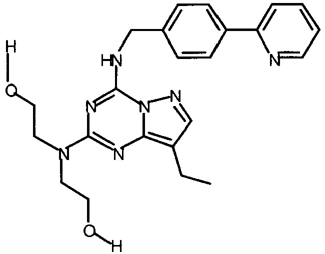

Rdt = 78 %. ¹H NMR (400 MHz, CDCl₃): δ 8,68 (d, 1H, Hₐᵣₒₘ), 7,96 (d, 2H Hₐᵣₒₘ), 7,74 (m, 2H, Harom), 7,65 (s, 1H, 7-H), 7,43 (d, 2H, Hₐᵣₒₘ), 7,26 (m, 1H, Hₐᵣₒₘ), 6,97(t, 1H, NH), 4,74 (d, 2H, CH₂N), 3,77 (m, 4H, 2 CH₂OH), 2,56 (q, 2H, CH₂CH₃), 1,22(t, 3H, CH₃).

### Exemple 41 : (2 R,3R)-2-[[8-ethyl-4-[[4-(2-pyridyl)phenyl]methylamino]pyrazolo[1,5-a][1,3,5]triazin-2-yl]amino]butane-1,3-diol(40).

Le produit **40** est préparé par chauffage à 140 °C de l'intermédiaire, **VIII**, dans les conditions de l'exemple 23, avec le L-thréoninol (2*R*,3*R*-thréoninol).

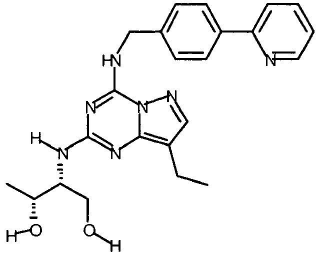

Rdt = 78 %. ¹H NMR (400 MHz, CDCl₃): δ 8,68 (d, 1H, Hₐᵣₒₘ), 7,96 (d, 2H Hₐᵣₒₘ), 7,74 (m, 2H, Harom), 7,65 (s, 1H, 7-H), 7,43 (d, 2H, Hₐᵣₒₘ), 7,26 (m, 1H, Hₐᵣₒₘ), 6,97(t, 1H, NH), 4,74 (d, 2H, CH₂N), 3,77 (m, 4H, 2 CH₂OH), 2,56 (q, 2H, CH₂CH₃), 1,22(t, 3H, CH₃).

### Exemple 42 : (2S,3S)-2-[[8-ethyl-4-[[4-(2-pyridyl)phenyl]methylamino]pyrazolo[1,5-a][1,3,5]triazin-2-yl]amino]butane-1,3-diol(41).

Le produit **41** est préparé par chauffage à 140 °C de l'intermédiaire, **VIII,** dans les conditions de l'exemple 23, avec le *D*-thréoninol (2*S*,3*S*-thréoninol).

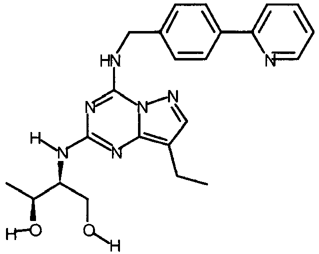

Rdt = 75 %. ¹H NMR (400 MHz, CDCl₃): δ 8,68 (d, 1H, Hₐᵣₒₘ), 7,97 (d, 2H Hₐᵣₒₘ), 7,75 (m, 2H, Harom), 7,63 (s, 1H, 7-H), 7,45 (d, 2H, Hₐᵣₒₘ), 7,24 (m, 1H, Hₐᵣₒₘ), 6,75(t, 1H, NH), 4,77 (d, 2H, CH₂N), 4.18 (m, 1H), 3.90(m, 3H), 2,55 (q, 2H, CH₂CH₃), 1,30(d, 3H, CHCH₃); 1,42(t, 3H, CH₂CH₃).

Le tableau II ci-après illustre les structures chimiques et les propriétés physiques de quelques exemples de composés de formule (I) selon l'invention. Dans ce tableau :
- PF = Point de Fusion, en degré Celsius (°C)
- « Rdt » désigne le rendement (%),
- MH⁺ = spectrométrie de masse (m/z),
- isoPr représente un groupe isopropyle,
- Et représente un groupe éthyle,
- dans la colonne « sels », « - » représente un composé sous forme de base libre, alors que « fumarate » représente un composé sous sa forme de fumarate,
- (+) indique que le composé est un énantiomère dextrogyre, (-) un énantiomère lévogyre :

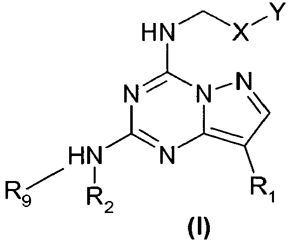

**Tableau II**

| **N^{o} compos é** | **R₁** | **R₂ ou NR₂R₉** | **R₉** | **X** | **Y** | **sel** | **PF (°C) et/ou masse (m/z)** |
|---|---|---|---|---|---|---|---|
| **1** | isoPr | 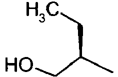 | H | 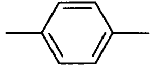 | 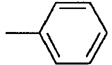 | - | MH⁺ = 431 |
| **2** | isoPr | 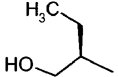 | H | 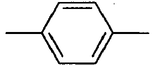 | 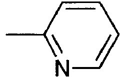 | - | MH⁺= 432 |
| **3** | isoPr | 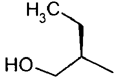 | H | 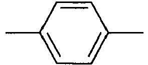 | 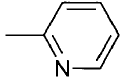 | fu | PF = 175-177 |
| **4** | isoPr | 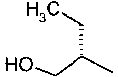 | H | 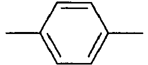 | 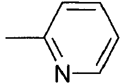 | - | MH⁺ = 432 |
| **5** | isoPr | 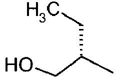 | H | 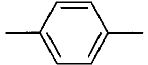 | 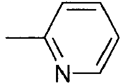 | fu | PF = 175-177 |
| **6** | isoPr | 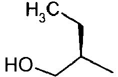 | H | 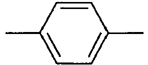 | 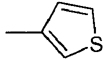 | - | MH⁺ = 437 |
| **7** | isoPr | 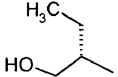 | H | 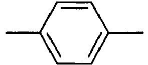 | 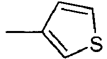 | - | MH⁺ = 437 |
| **8** | isoPr | 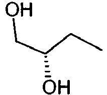 | H | 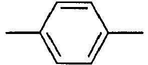 | 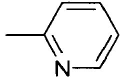 | - | MH⁺ = 434 |
| **9** | isoPr | 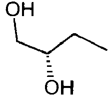 | H | 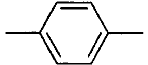 | 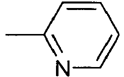 | fu | PF= 168-170 |
| **10** | isoPr | 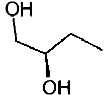 | H | 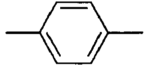 | 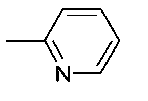 | - | MH⁺ = 434 |
| **11** | isoPr | 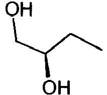 | H | 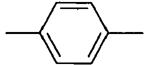 | 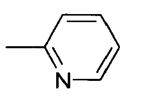 | fu | PF = 168-170 |
| **12** | isoPr | 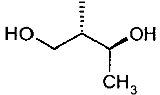 | H | 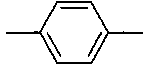 | 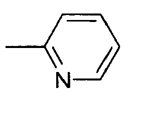 | - | MH⁺ = 448 |
| **13** | isoPr | 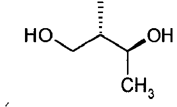 | H | 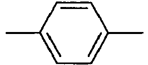 | 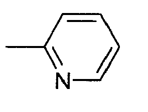 | fu | PF = 187-189 |
| **14** | isoPr | 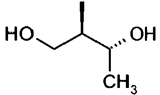 | H | 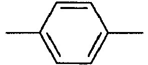 | 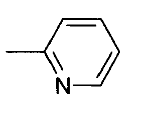 | - | MH⁺ = 448 |
| **15** | isoPr | 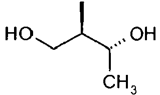 | H | 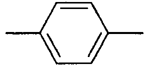 | 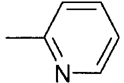 | fu | PF = 187-189 |
| **16** | isoPr | 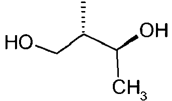 | H | 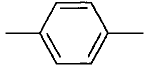 | 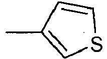 | - | MH⁺ = 453 |
| **17** | isoPr | 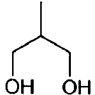 | H | 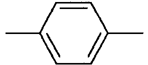 | 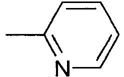 | - | MH⁺ = 434 |
| **18** | isoPr | 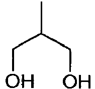 | H | 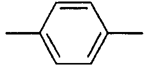 | 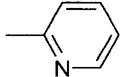 | fu | PF = 179-181 |
| **19** | isoPr | 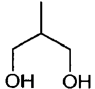 | H | 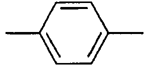 | 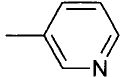 | - | MH⁺= 434 |
| **20** | isoPr | 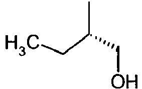 | H | 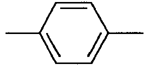 | 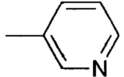 | - | MH⁺= 432 |
| **21** | isoPr | 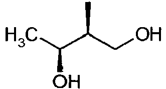 | H | 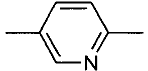 | 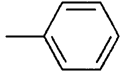 | - | MH⁺= 448 |
| **22** | isoPr | 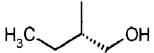 | H | 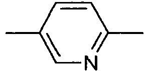 | 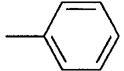 | - | MH⁺= 432 |
| **23** | isoPr | 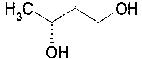 | H | 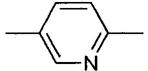 | 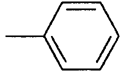 | - | MH⁺= 448 |
| **24** | isoPr | 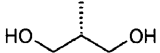 | H | 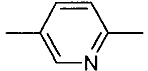 | 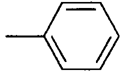 | - | MH⁺= 448 |
| **25** | isoPr | 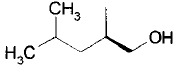 | H | 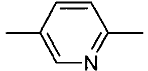 | 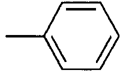 | - | MH⁺= 460 |
| **26** | isoPr | 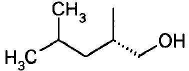 | H | 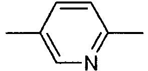 | 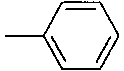 | - | MH⁺= 460 |
| **27** | isoPr | 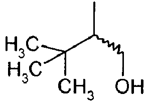 | H | 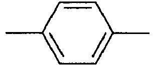 | 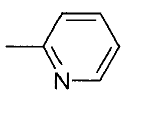 | - | MH⁺= 460 |
| **28** | isoPr | 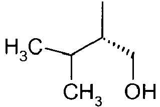 | H | 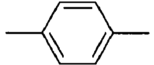 | 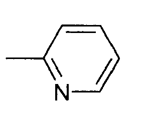 | - | MH⁺= 446 |
| **29** | isoPr | 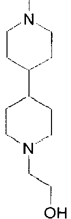 | | 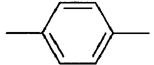 | 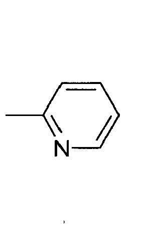 | - | |
| **30** | isoPr | 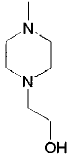 | | 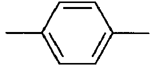 | 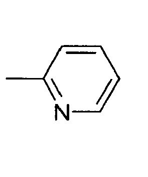 | - | |
| **31** | Et | 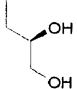 | H | 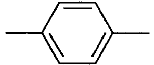 | 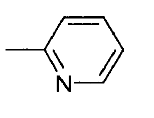 | - | MH⁺= 420 |
| **32** | Et | 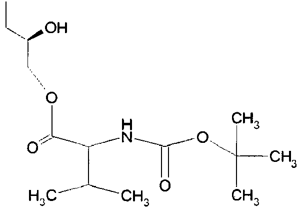 | H | 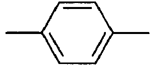 | 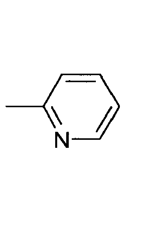 | - | MH⁺= 618 |
| **33** | Et | 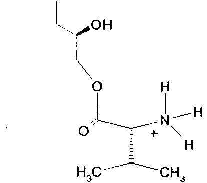 | H | 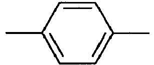 | 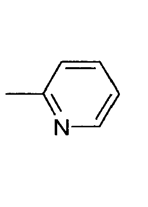 | H | - |
| **34** | Et | 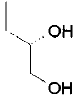 | H | 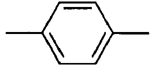 | 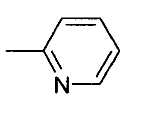 | - | MH⁺= 420 |
| **35** | Et | 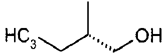 | H | 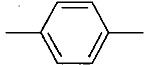 | 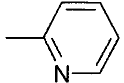 | - | MH⁺= 418 |
| **36** | Et | 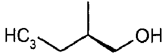 | H | 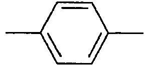 | 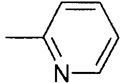 | - | MH⁺= 418 |
| **37** | Et | 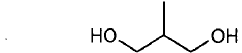 | H | 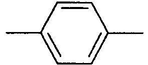 | 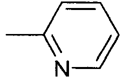 | - | MH⁺=420 |
| **38** | Et | 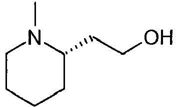 | | 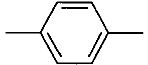 | 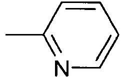 | - | MH⁺= 458 |
| **39** | Et | 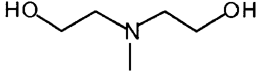 | | 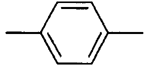 | 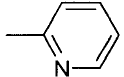 | - | MH⁺= 434 |
| **40** | Et | 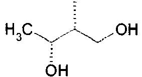 | H | 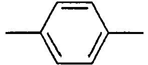 | 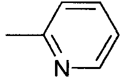 | | MH⁺= 434 |
| **41** | Et | 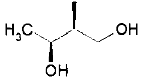 | H | 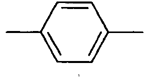 | 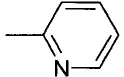 | | MH⁺= 434 |

Les composés selon l'invention ont fait l'objet d'essais pharmacologiques permettant de déterminer leur effet inhibiteur de l'activité des protéines kinases.

Selon une première série d'essais pharmacologiques, les composés ont été testés sur 7 protéines kinases selon la méthodologie décrite ci-après.

On constate en particulier que l'activité inhibitrice des composés selon l'invention sur CDK1, CDK2, CDK5 et CDK9 présente généralement une CI₅₀ autour de 0,050 µM. (CDK1 : 0,015-0,070 µM ; CDK2 : 0,012-0,060 µM ; CDK5 : 0,018-0,064 µM; CDK9 : 0,030-0,091 µM). Sur CK1, les composés de l'invention montrent une CI₅₀ entre 0,06 et 0,2 µM, alors que sur DYRK1A et GSK-3, les CI₅₀ sont autour de 0,5-2,0 µM et > 2,8 µM, respectivement.

Le tableau III rassemble les résultats sur les CI₅₀ rapportés en µM.

### MATERIEL ET METHODES

### Tampons

*Tampons d'homogénéisation:* 60 mM β-glycérophosphate, 15 mM p-nitrophénylphosphate, 25 mM Mops (pH 7.2), 15 mM EGTA, 15 mM MgCl₂, 1 mM DTT, 1 mM sodium vanadate, 1 mM NaF, 1 mM phénylphosphate, 10 µg leupeptine/ml, 10 µg aprotinine/ml, 10 µg d'inhibiteur de trypsine de soja /ml et 100 µM benzamidine.

*Tampon A*: 10 mM MgCl₂, 1 mM EGTA, 1 mM DTT, 25 mM Tris-HCl pH 7,5, 50 µg héparine/ml.

*Tampon C*: 60 mM β-glycérophosphate, 15 mM p-nitrophénylphosphate, 25 mM Mops (pH 7.2), 5 mM EGTA, 15 mM MgCl₂, 1 mM DTT, 1 mM sodium vanadate, 1 mM phénylphosphate, 10 µg leupeptine/ml, 10 µg aprotinine/ml et 100 µM benzamidine.

### Préparation et dosage des kinases

Les kinases ont été dosées dans le tampon A ou le tampon C, à 30 °C, à une concentration finale en ATP de 15 µM. Les valeurs de blanc ont été soustraites et les activités calculées en tant que µmoles de phosphate incorporé pour 10 minutes d'incubation. Les activités sont habituellement exprimées en % (pourcentage) de l'activité maximale, c'est-à-dire en l'absence d'inhibiteurs. Des contrôles ont été effectués avec des dilutions appropriées de diméthylsulfoxide.

*CDK1*/*cycline B* : a été extrait dans un tampon d'homogénisation à partir d'oocytes d'étoiles de mer de phase M (*Marthasterias glacialis*), purifié par chromatographie d'affinité sur des billes de sépharose marquées à p9^{CKShs1} dont il a été élué par p9^{CKShs1} libre comme antérieurement décrit dans Meijer et al., (1997) « Biochemical and cellular effects of roscovitine, a potent and selective inhibitor of the cyclin-dependent kinases cdc2, cdk2 and cdk5 », Eur. J. Biochem. 1997, 243, 527-536. L'activité kinase a été dosée dans le tampon C, avec 1 mg d'histone Hl/ml, en présence de 15 µM de [γ-³³P] ATP (3 000 Ci/mmol; 10 mCi/ml) dans un volume final de 30 µl. Après 30 minutes d'incubation à 30 °C, des aliquotes de 25 µl du surnageant ont été mises en tâches sur des filtres en papier de phosphocellulose Whatman P81, et, 20 secondes plus tard, les filtres ont été lavés cinq fois (pendant au moins cinq minutes, chaque fois) dans une solution de 10 ml d'acide phosphorique/litre d'eau. Les filtres humides ont été soumis à un comptage en présence d'un fluide de scintillation ACS d'Amersham.

*CDK2*/*cycline A* (humain, recombinant, exprimé dans des cellules d'insecte) a été dosé comme décrit pour CDK1/cycline B.

*CDK5*/*p25* a été reconstitué en mélangeant des quantités égales de CDK5 mammalien recombinant et p25 exprimé dans *E. coli* en tant que protéine de fusion GST (glutathione-S-transférase) et purifié par chomatographie d'affinité sur glutathione-agarose (p25 est une version tronquée de p35, l'activateur de CDK5 de 35 kDa). Son activité a été dosée avec de l'histone H1 dans le tampon C comme décrit pour CDK1/cycline B.

*CDK9*/*cycline T* (humain, recombinant, exprimé dans des cellules d'insecte) a été dosé comme décrit pour CDK1/cycline B, mais en utilisant un fragment pRB (a.a.773-928) (3.5µg/dosage) en tant que substrat.

*GSK-3α*/*β* (cerveau de porc, native, purifiée par affinité) est dosée comme décrit pour la CDK1 mais dans le tampon A et avec un substrat spécifique de GSK-3 (GS-1: YRRAAVPPSPSLSRHSSPHQSpEDEEE) (Sp : serine phosphorylée) (Bach S. et al. J Biol Chem 2005; 280:31208-19).

*CK1δ*/*ε* (cerveau de porc, native, purifiée par affinité) est dosée comme décrit pour la CDK1 avec un substrat spécifique de CK1, RRKHAAIGSpAYSITA (Reinhardt J. et al. Protein Expr & Purif 2007; 54:101-9).

*DYRK1A* (humaine, recombinante, exprimée en *E. coli* comme protéine de fusion GST) a été purifiée sur glutathion-agarose dosée comme décrit pour la CDK1/cyclin B avec la myelin basic protein (1 mg/ml) comme substrat.

**Tableau III**

| Kinase | **CDK1/ cyclin B** | **CDK2/ cyclin A** | **CDK5/ p25** | **CDK9/ cyclin T** | **GSK-3α/β** | **CK1** | **DYRK1A** |
|---|---|---|---|---|---|---|---|
| **Roscovitine** | 0,33 | 0,21 | 0,28 | 0,23 | 60 | 4,0 | 3,0 |
| **N-&-N1** | 0,09 | 0,04 | 0,07 | 0,043 | 11,0 | 1,2 | 1,3 |
| **1** | 0,18 | 0,22 | 0,18 | 0,093 | > 10 | 0,21 | 2,2 |
| **2** | 0,023 | 0,028 | 0,040 | 0,073 | 4,0 | 0,21 | 1,8 |
| **3** | 0,016 | 0,021 | 0,018 | 0,031 | 3,1 | 0,13 | 2,2 |
| **5** | 0,023 | 0,016 | 0,031 | 0,030 | 3,5 | 0,21 | 0,5 |
| **17** | 0,070 | 0,062 | 0,053 | 0,068 | 4,4 | 0,092 | 0,92 |
| **12** | 0,029 | 0,022 | 0,029 | 0,060 | 3,0 | 0,20 | 0,47 |
| **9** | 0,019 | 0,012 | 0,014 | 0,038 | 1,8 | 0,061 | 0,26 |
| **13** | 0,031 | 0,020 | 0,021 | 0,049 | 2,3 | 0,18 | 0,30 |
| **16** | 0,059 | 0,034 | 0,050 | 0,091 | 4,2 | 0,19 | 1,80 |
| **14** | 0,042 | 0,042 | 0,044 | 0,053 | 2,5 | 0,12 | 0,58 |
| **15** | 0,042 | 0,031 | 0,045 | 0,060 | 2,2 | 0,12 | 0,53 |
| **18** | 0,051 | 0,049 | 0,064 | 0,061 | 2,8 | 0,12 | 0,61 |
| **25** | NT | NT | 0,11 | NT | >10 | 0,23 | 1,4 |
| **34** | NT | NT | 0,010 | NT | 0,53 | 0.10 | 0,42 |
| **35** | NT | NT | 0,034 | NT | 1,4 | 0,93 | 0,82 |
| **36** | NT | NT | 0,022 | NT | 1,6 | 0,58 | 1,6 |
| **31** | NT | NT | 0,025 | NT | 1,0 | 0,36 | 0,71 |
| **23** | NT | NT | <0,03 | NT | 1,2 | 0,13 | 1,3 |
| **21** | NT | NT | 0,043 | NT | 1,2 | 0,11 | 1,2 |
| **28** | NT | NT | 0,12 | NT | 7,0 | 0,41 | 0,58 |
| **30** | NT | NT | 0,38 | NT | 2,1 | 0,56 | 1,4 |

| | | | | | | | |
|---|---|---|---|---|---|---|---|
| « *NT* » : *Non testé* | | | | | | | |

Selon une deuxième série d'essais pharmacologiques, des tests sur cellules ont été menés, plus particulièrement sur la lignée SH-SY5Y et CLL selon la méthodologie décrite ci-après. Le tableau IV rassemble des exemples de résultats en valeurs de CI₅₀ en µM.

### MATERIEL ET METHODES

### A. Cellules de Neuroblastome humain SH-SY5Y

### Réactifs chimiques

Un kit Cell Titer 96^{®} contenant le réactif MTS a été acheté à Promega (Madison, WI, USA). Le cocktail inhibiteur de protéase provenait de Roche et le sérum de veau foetal (FCS) provenait d'Invitrogen. Les réactifs non listés provenaient de Sigma, sauf s'il en est indiqué autrement.

### Lignée cellulaire et conditions de culture

La lignée de cellules du neuroblastome humain SH-SY5Y a été mise à pousser dans un milieu DMEM avec de la L-glutamine provenant d'Invitrogen (Cergy Pontoise, France), des antibiotiques et 10 % en volume de FCS provenant d'Invitrogen. Les conditions générales de culture étaient une atmosphère de 5 % en CO₂ et une température de 37°C. Les plaques de culture et autres outils en plastique jetables ont été fournis par Corning (Coming, NY, USA). Les traitements par les composés de l'invention ont été effectués sur des cultures en croissance exponentielle au temps, et concentrations indiquées. Les expériences de contrôles ont été mises en oeuvre en utilisant également des dilutions appropriées de DMSO.

### Mise en évidence de la viabilité des cellules

La viabilité des cellules a été déterminée en mesurant la réduction de 3-(4,5-diméthylthiazol-2-yl)-5-(3-carboxyméthoxyphényl)-2-(4-sulfophényl)-2*H*-tétrazolium (MTS). La procédure était telle que décrite en détails dans Ribas J, et al., 2004, « Cell differentiation, caspase inhibition, and macromolecular synthesis blockage, but not BCL-2 or BCL-XL proteins, protect SH-SYS5 cells from apoptosis triggered by two CDK inhibitory drugs », Exp. Cell Res, 2004, 195, 9-24.

### B. Lymphocyte humains de Leucémie Lymphoïde Chronique (B-LLC)

### Patients et purification des cellules

Les cellules LLC ont été isolées à partir de sang héparinisé de patients non traités après consentement éclairé.

Les cellules mononucleaires ont été isolées par centrifugation sur gradient de densité Lymphosep (Biowest). Tous les échantillons de LLC-B avaient un score de Matutes de 4 ou 5. Le pourcentage de lymphocytes B LLC a été évalué par cytométrie de flux après marquage au CD19-PE (clone J4,119) et CD5-PC5 (clone BL1a) et les cellules analysées par FACS (EPICS XL, Beckman Coulter, France). Lorsque le pourcentage de cellules CD19+/CD5+ était inferieur à 90%, les lymphocytes B ont été enrichies en utilisant le kit II d'enrichissement en cellules B par déplétion immuno-magnétique des monocytes, NK, granulocytes et lymphocytes T (Miltenyi Biotech).

### Apoptose

Les cellules B-LLC ont été cultivées (300 000 cellules /puits) à 37 °C dans le milieu RPMI 1640 (Lonza) contenant 10% de FCS (InVitrogen) avec diverses concentrations des composés de l'invention. Après incubation, les cellules B-LLC ont été récoltées, lavées en PBS et remises en suspension dans 100µL de « binding buffer 1x » contenant l'annexine V conjugee au FITC et de l'iodure de Propidium (Beckman Coulter Apoptosis Detection Kit). Après 10 min d'incubation sur la glace les cellules ont été analysées par cytométrie de flux et le pourcentage de cellules en apoptose a été déterminé.

Les composés de l'invention présentent généralement une CI₅₀ inférieure à 1 µm pour les deux lignées cellulaires considérées.

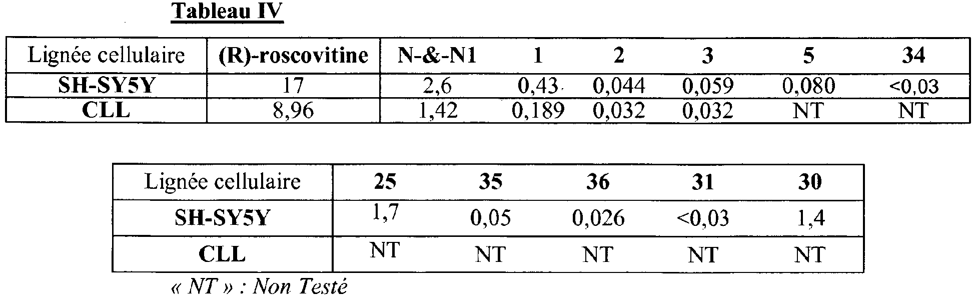

Les composés selon l'invention peuvent donc être utilisés pour la préparation de médicaments, en particulier de médicaments présentant une activité inhibitrice des protéines kinases et en particulier des kinases cycline-dépendantes (CDKs), des Caseine kinase 1 (CK1s) et des DYRKs.

Ainsi, selon un autre de ses aspects, la présente invention a pour objet des médicaments qui comprennent un composé de formule (**I**), ou un sel d'addition de ce dernier à un acide pharmaceutiquement acceptable, ou encore un hydrate ou un solvat.

Ces médicaments trouvent leur emploi en thérapeutique, notamment dans la prévention et le traitement de différentes pathologies telles que les cancers, maladies neurodégénératives chroniques telles que maladies d'Alzheimer, de Parkinson, maladies neurodégénératives aigües telles que traumatisme cérébral, accident vasculaire cérébral, épilepsie, inflammations pulmonaires, arthrite, infections virales (SIDA, Herpes), traitement de la douleur, diabète, en particulier de type 2, maladies rénales telle que la polykystose rénale ou les glomérulonéphrites, leucémies telles que la leucémie lymphoïde chronique, en particulier de type B, parasites tels que *Plasmodium* et *Leishmania.*

Les composés de l'invention sont tout particulièrement utiles pour prévenir et/ou traiter les maladies suivantes : cancers, leucémie lymphoïde chronique, maladie d'Alzheimer, maladie de Parkinson, accident vasculaire cérébral, inflammations pulmonaires, SIDA, polykystose rénale, glomérulonéphrites.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intraveineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux êtres humains pour la prophylaxie ou le traitement des troubles ou des maladies cités ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants.

Par voie orale, la dose de principe actif administrée par jour peut atteindre 1 à 500 mg/kg, en une ou plusieurs prises,

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

La présente invention, selon un de ses aspects, concerne l'utilisation d'un composé de formule (**I**), (**Ia**), (**Ib**), (**Ic**), (**Id**), (**Ie**), (**If**) ou (**1**) à (**38**) ou l'un de ses sels pharmaceutiquement acceptable, pour la préparation d'un médicament destiné à traiter l'une au moins des maladies citées ci-dessus et en particulier le cancer.

Selon un autre de ses aspects, la présente invention concerne un composé de formule (**I**), (**Ia**), (**Ib**), (**Ic**), (**Id**), (**Ie**), (**If**) ou (**1**) à (**38**) ou l'un de ses sels pharmaceutiquement acceptable pour son utilisation à titre de médicament destiné au traitement et à la prévention d'au moins une des maladies citées ci-dessus et en particulier à titre d'agent anticancéreux.

La présente invention, selon encore un autre de ses aspects, concerne également une méthode de prévention et/ou de traitement des pathologies indiquées ci-dessus qui comprend l'administration, à un patient, d'une dose efficace d'un composé selon l'invention, ou un de ses sels pharmaceutiquement acceptables ou hydrates ou solvats.

Les compositions pharmacetuiques selon l'invention peuvent également trouver une utilisation dans le domaine vétérinaire et aussi être administrées à des animaux tels que chiens et chats pour la prophylaxie ou le traitement des troubles ou des maladies cités ci-dessus.

## Revendications

1. Composé de formule (I) suivante : 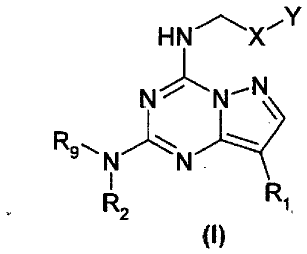 dans laquelle:
R₁ représente un groupe (C₁-C₆)alkyle ou un groupe (C₃-C₆)cycloalkyle,
R₂ représente un groupe (C₁-C₆)alkyle, un groupe (C₃-C₆)cycloalkyle, un groupe (C₁-C₆)alkényle, un groupe (C₁-C₆)fluoroalkyle, un groupe (C₁-C₃)fluoroalcoxy ou un groupe (C₁-C₆)alcoxy(C₁-C₆)alkyle, substitué (i) par un à trois groupes hydroxy, ou (ii) par un groupe NRₐR_{b}, où Rₐ et R_{b} représentent indépendamment un atome d'hydrogène ou un groupe (C₁-C₃)alkyle,
ou bien R₂ représente un groupe pyrrolidinylméthyle substitué par un à trois groupes hydroxy,
ledit groupe R₂ étant éventuellement substitué par un groupe -OCOR₃, dans lequel R₃ représente un dérivé d'acide aminé naturel ou non naturel ou un groupe
pipéridyle de formule (**B**) 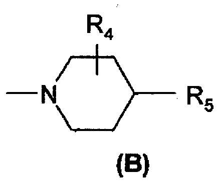 dans laquelle :
R₄ représente un hydrogène, un atome d'halogène, un groupe (C₁-C₃)alkyle, un groupe hydroxy(C₁-C₃)alkyle ou un groupe -NRₐR_{b}, où Rₐ et R_{b} sont tels que définis précédemment et R₅ représente un hydrogène, un groupe (C₁-C₃)alkyle, un groupe -N(Me)₂, un groupe pipéridyle ou un groupe morpholinyle,
R₉ prend la même signification que R₂ et peut également représenter un atome d'hydrogène,
alternativement R₂ et R₉ forment ensemble, avec l'atome d'azote qui les porte, un hétérocycle choisi parmi les groupes pyrrolidinyle, pipéridinyle, pipérazinyle et pipéridinylpipéridinyle, ces hétérocycles pouvant être substitués par un à trois groupes choisis parmi hydroxy, (C₁-C₆)alkyle ou (C₁-C₆)alcoxy(C₁-C₆)alkyle, ces deux derniers groupes étant substitués (i) par un à trois groupes hydroxy, ou (ii) par un groupe NRₐR_{b}, ou Rₐ et R_{b} représentent indépendamment un atome d'hydrogène ou un groupe (C₁-C₃)alkyle,
X et Y représentent indépendamment un groupe phényle ou un groupe hétéroaryle, lesdits groupes hétéroaryle et phényle pouvant être substitués par un ou deux groupes indépendamment choisis parmi un groupe (C₁-C₂)alkyle, un groupe (C₁-C₂)alcoxy, un atome d'halogène, un groupe (C₁-C₂)fluoroalkyle, un groupe (C₁-C₂)fluoroalcoxy, un groupe hydroxy, un groupe -COOH, un groupe -CONHR₆ et un groupe -NRₐR_{b}, où Rₐ et R_{b} sont tels que définis précédemment,
R₆ est un hydrogène ou un groupe (C₁-C₃)alkyle,
ledit groupe hétéroaryle étant choisi parmi un groupe thiényle, un groupe pyridyle, un groupe pyrimidinyle, un groupe thiazolyle, un groupe pyrrolyle et un groupe furanyle,
ainsi que ses sels pharmaceutiquement acceptables.

2. Composé selon la revendication 1, **caractérisé en ce que** R₃ représente une des formules (**a-1**) à (**a-5**) suivantes : 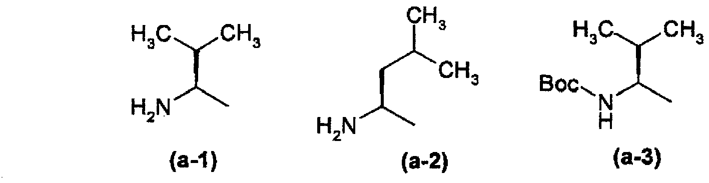 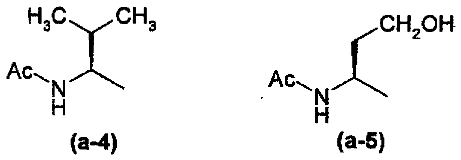 où Boc représente un groupe tert-butoxycarbonyle et Ac un groupe acétyle, ainsi que leurs énantiomères.

3. Composé selon la revendication 1 ou 2, **caractérisé en ce que** l'un au moins des groupes X et Y est substitué et comporte un ou deux groupes de substitution, ledit groupe de substitution étant choisi parmi un groupe (C₁-C₂)alkyle, un groupe (C₁-C₂)fluoroalkyle, un groupe (C₁-C₂)alcoxy, un groupe (C₁-C₂)fluoroalcoxy, un atome d'halogène, un groupe hydroxy et un groupe -COOH.

4. Composé selon l'une quelconque des revendications précédentes, **caractérisé en ce que** R₂ représente l'une des formules suivantes : 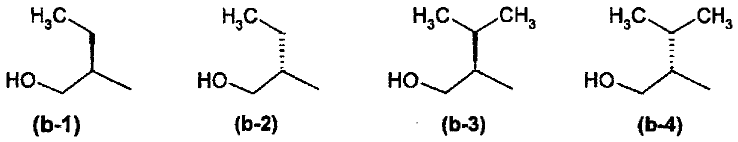 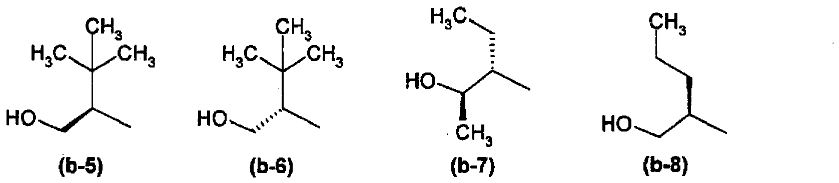 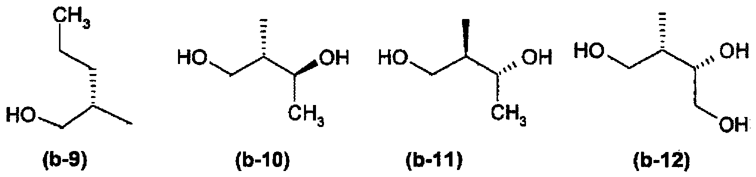 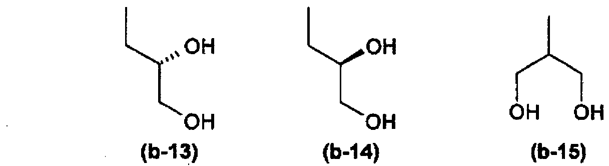 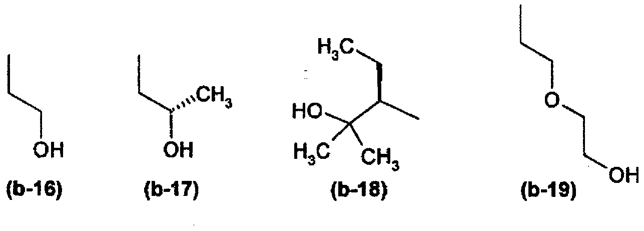 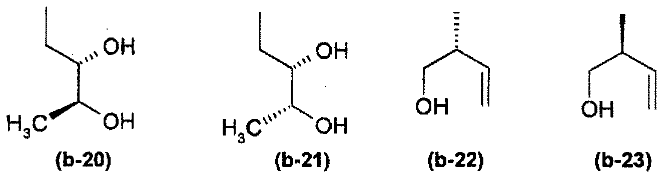 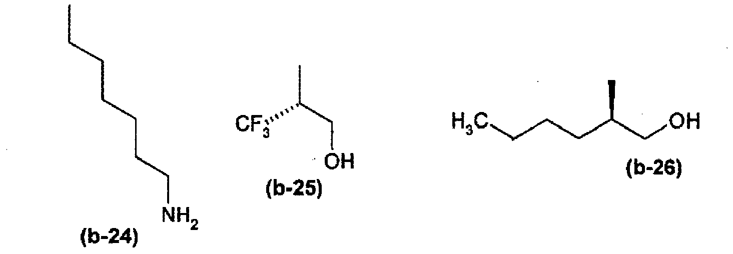 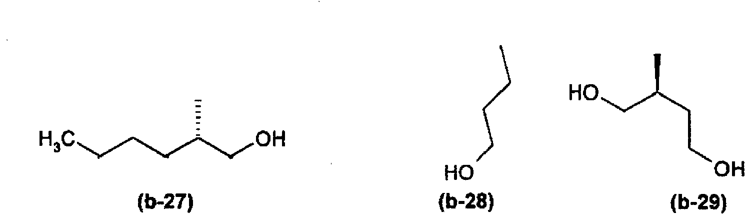 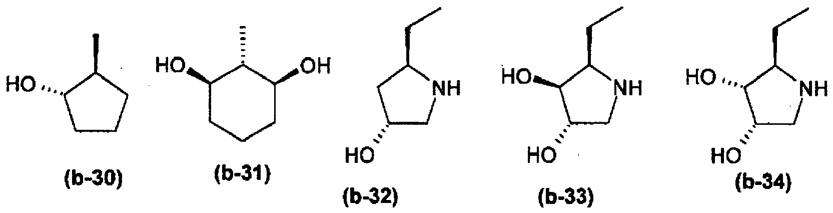 et en particulier l'une des formules (**b-1**), (**b-2**), (**b-10**), (**b-11**), (**b-13**), (**b-14**) ou (**b-15**), ou bien R₂ et R₉ forment ensemble, avec l'atome d'azote qui les porte, l'une des formules suivantes : 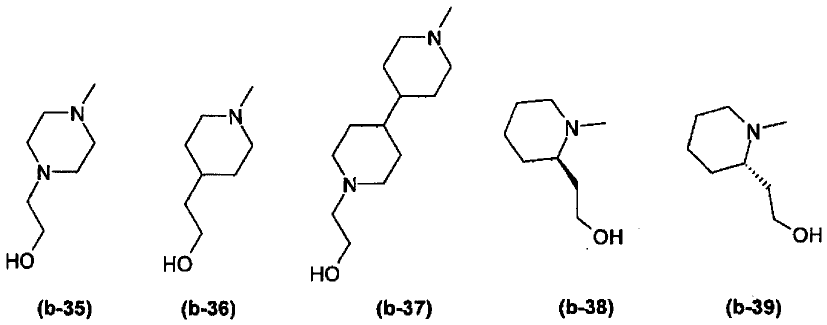 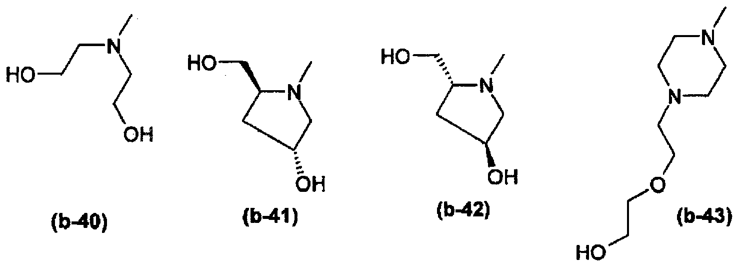 et, en particulier, l'une des formules (**b-37**), (**b-38**), (**b-39**) ou (**b-40**).

5. Composé selon l'une quelconque des revendications précédentes, **caractérisé en ce qu'**il présente une formule (**Ia**) : 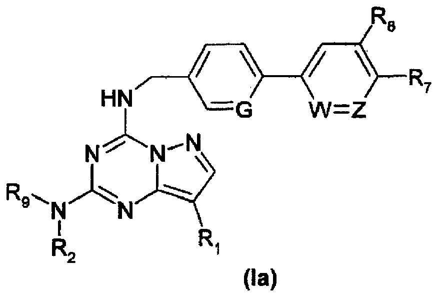 dans laquelle :
R₁ est tel que défini en revendication 1 et est avantageusement un groupe isopropyle,
R₂ et R₉ représentent indépendamment un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy avec R₉ pouvant représenter un atome d'hydrogène et R₂ représente avantageusement l'une des formules (**b-1**), (**b-2**), (**b-10**), (**b-11**), (**b-13**), (**b-14**) ou (**b-15**) telles que définies ci-dessus, tandis que R₉ est un atome d'hydrogène, ou bien,
R₂ et R₉ forment ensemble, avec l'atome d'azote qui les porte, un groupe pipéridin-1-yle ou pipéridin-4-ylpipéridin-1-yle, ledit groupe étant substitué par un à trois groupes (C₁-C₆)alkyle substitué par un groupe hydroxy et R₂ et R₉ pris ensemble représentent avantageusement l'une des formules (**b-37**), (**b-38**), (**b-39**) ou (**b-40**) telles que définies ci-dessus,
R₇ et R₈ représentent indépendamment un atome d'hydrogène, un groupe (C₁-C₂)alkyle, un groupe (C₁-C₂)fluoroalkyle, un groupe (C₁-C₂)alcoxy, un groupe (C₁-C₂)fluoroalcoxy, un atome d'halogène, un groupe hydroxy ou un groupe -COOH,
G représente -CH=, ou -N=, et
lorsque G représente -CH=, les groupes W et Z représentent soit simultanément -CH=, soit l'un représente -N= et l'autre représente -CH=,
lorsque G représente -N=, alors W et Z représentent -CH=,
ou un de ses sels pharmaceutiquement acceptables.

6. Composé selon l'une quelconque des revendications 1 à 4, **caractérisé en ce qu'**il présente une formule (**Ib**) : 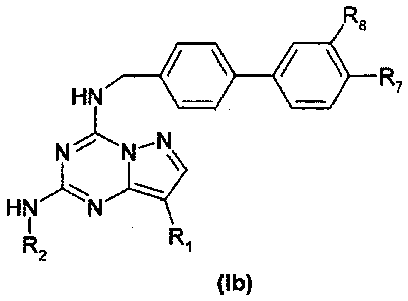 dans laquelle :
R₁ est tel que défini en revendication 1 et est avantageusement un groupe isopropyle,
R₂, R₇ et R₈ sont tels que définis dans la revendication précédente,
ou un de ses sels pharmaceutiquement acceptables.

7. Composé selon l'une quelconque des revendications 1 à 4, **caractérisé en ce qu'**il présente une formule (**Ic**) : 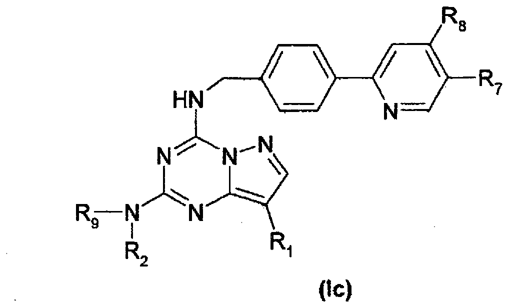 dans laquelle :
R₁ est tel que défini en revendication 1 et est avantageusement un groupe éthyle ou isopropyle,
soit R₉ est un atome d'hydrogène et R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy, et avantageusement représente l'une des formules (**b-1**), (**b-2**), (**b-10**), (**b-11**), (**b-13**), (**b-14**) ou (**b-15**) telles que définies ci-dessus ou alternativement,
soit R₂ et R₉ forment ensemble, avec l'atome d'azote qui les porte, un groupe pipéridin-1-yle ou pipéridin-4-ylpipéridin-1-yle, ledit groupe étant substitué par un à trois groupes (C₁-C₆)alkyle substitué par un groupe hydroxy et R₂ et R₉ pris ensemble représentent avantageusement l'une des formules (**b-37**), (**b-38**), (**b-39**) ou (**b-40**) telles que définies ci-dessus, et
R₇ et R₈ sont tels que définis dans la revendication 5,
ou un de ses sels pharmaceutiquement acceptables.

8. Composé selon l'une quelconque des revendications 1 à 4, **caractérisé en ce qu'**il présente une formule (**Id**) :
dans laquelle : 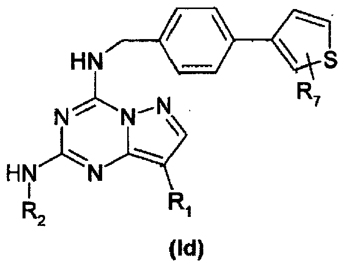 R₁ est tel que défini en revendication 1 et est avantageusement un groupe isopropyle,
R₂ et R₇ sont tels que définis dans la revendication 5,
ou un de ses sels pharmaceutiquement acceptables.

9. Composé selon l'une quelconque des revendications 1 à 4, **caractérisé en ce qu'**il présente une formule (**Ic**) : 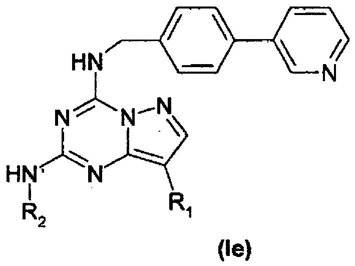 dans laquelle :
R₁ est tel que défini en revendication 1 et est avantageusement un groupe isopropyle,
R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy, et avantageusement représente l'une des formules (**b-1**), (**b-2**), (**b-10**), (**b-11**), (**b-13**), (**b-14**) ou (**b-15**) telles que définies ci-dessus,
ou encore une formule (If) : 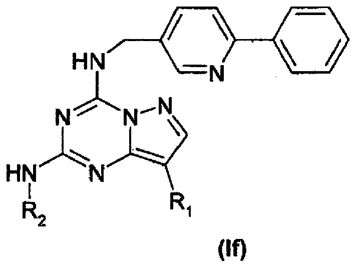
R₁ est tel que défini en revendication 1 et est avantageusement un groupe isopropyle,
R₂ représente un groupe (C₁-C₆)alkyle, un groupe (C₁-C₆)fluoroalkyle, un groupe (C₁-C₃)fluoroalcoxy ou un groupe (C₁-C₆)alcoxy(C₁-C₆)alkyle, ledit groupe étant substitué par un à trois groupes hydroxy, et avantageusement représente l'une des formules (**b-1**), (**b-2**), (**b-10**), (**b-11**), (**b-13**), (**b-14**) ou (**b-15**) telles que définies ci-dessus.

10. Composé selon l'une quelconque des revendications précédentes, **caractérisé en ce qu'**il est choisi parmi l'un des composés suivants :
- (*R*) 2-(1-hydroxybut-2-ylamino)-8-isopropyl-4-(4-phénylbenzylamino)pyrazolo[1,5-*a*]-1,3,5-triazine (**1**)
- (*R*) 2-(1-hydroxybut-2-ylamino)-8-isopropyl-4-[4-(pyridin-2-yl)benzylamino]pyrazolo[1,5-*a*]-1,3,5-triazine (**2**)
- Sel de fumarate du (*R*) 2-(1-hydroxybut-2-ylamino)-8-isopropyl-4-[4-(pyridin-2-yl)benzylamino]pyrazolo[1,5-*a*]-1,3,5-triazine (**3**)
- (*S*) 2-(1-hydroxybut-2-ylamino)-8-isopropyl-4-[4-(pyridin-2-yl)benzylamino]pyrazolo[1,5-*a*]-1,3,5-triazine (**4**)
- Sel de fumarate du (*S*) 2-(1-hydroxybut-2-ylamino)-8-isopropyl-4-[4-(pyridin-2-yl)benzylamino]pyrazolo[1,5-*a*]-1,3,5-triazine (**5**)
- (*R*) 2-(1-hydroxybut-2-ylamino)-8-isopropyl-4-[4-(thiophén-3-yl)benzylamino]pyrazolo[1,5-*a*]-1,3,5-triazine (**6**)
- (*S*) 2-(1-hydroxybut-2-ylamino)-8-isopropyl-4-[4-(thiophén-3-yl)benzylamino]pyrazolo[1,5-*a*]-1,3,5-triazine (**7**)
- (*S*) 2-(1,2-dihydroxypropan-3-ylamino)-8-isopropyl-4-[4-(pyridin-2-yl)benzylamino]pyrazolo[1,5-*a*]-1,3,5-triazine (**8**)
- Sel de fumarate du (*S*) 2-(1,2-dihydroxypropan-3-ylamino)-8-isopropyl-4-[4-(pyridin-2-yl)benzylamino]pyrazolo[1,5-*a*]-1,3,5-triazine (**9**)
- (*R*) 2-(1,2-dihydroxypropan-3-ylamino)-8-isopropyl-4-[4-(pyridin-2-yl)benzylamino]pyrazolo[1,5-*a*]-1,3,5-triazine (**10**)
- Sel de fumarate du (*R*) 2-(1,2-dihydroxypropan-3-ylamino)-8-isopropyl-4-[4-(pyridin-2-yl)benzylamino]pyrazolo[1,5-*a*]-1,3,5-triazine (**11**)
- (*2R*,*3R*) 2-(1,3-dihydroxybut-2-ylamino)-8-isopropyl-4-[4-(pyridin-2-yl)benzylamino]pyrazolo[1,5-*a*]-1,3,5-triazine (**12**)
- sel de fumarate du (*2R*,*3R*) 2-(1,3-dihydroxybut-2-ylamino)-8-isopropyl-4-[4-(pyridin-2-yl)benzylamino]pyrazolo[1,5-*a*]-1,3,5-triazine (**13**)
- (*2S*,*3S*) 2-(1,3-dihydroxybut-2-ylamino)-8-isopropyl-4-[4-(pyridin-2-yl)benzylamino]pyrazolo[1,5-*a*]-1,3,5-triazine (**14**)
- Sel de fumarate du (*2S*,*3S*) 2-(1,3-dihydroxybut-2-ylamino)-8-isopropyl-4-[4-(pyridin-2-yl)benzylamino]pyrazolo [1,5-*a*]-1,3,5-triazine (**15**)
- (*2R,3R*) 2-(1,3-dihydroxybut-2-ylamino)-8-isopropyl-4-[4-(thiophén-3-yl)benzylamino]pyrazolo[1,5-*a*]-1,3,5-triazine (**16**)
- 2-(1,3-dihydroxyprop-2-ylamino)-8-isopropyl-4-[4-(pyridin-2-yl)benzylamino]pyrazolo[1,5-*a*]-1,3,5-triazine (**17**)
- Sel de fumarate du 2-(1,3-dihydroxyprop-2-ylamino)-8-isopropyl-4-[4-(pyridin-2-yl)benzylamino]pyrazolo[1,5-*a*]-1,3,5-triazine (**18**),
- 2-(1,3-dihydroxyprop-2-ylamino)-8-isopropyl-4-[4-(pyridin-3-yl)benzylamino]pyrazolo[1,5-*a*]-1,3,5-triazine (**19**)
- (*S*) 2-(-1-hydroxybut-2-ylamino)-8-isopropyl-4-[4-(pyridin-3-yl)benzylamino]pyrazolo[1,5-*a*]-1,3,5-triazine (**20**)
- (*2S,3S*) 2-(1,3-dihydroxybut-2-ylamino)-8-isopropyl-4-[4-phényl(pyridin-3-yl)méthylamino]pyraxolo[1,5-*a*]-1,3,5-triazine (**21**)
- (*S*) 2-(-1-hydroxybut-2-ylamino)-8-isopropyl-4-[4-phényl(pyridin-3-yl)méthylamino]pyrazolo[1,5-*a*]-1,3,5-triazine (**22**)
- (*2R*,*3R*) 2-{1,3-dihydroxybut-2-ylamino)-8-isopropyl-4-[4-phényl(pyridin-3-yl)méthylamino]pyrazolo[1,5-*a*]-1,3,5-triazine (**23**)
- 2-(1,3-dihydroxyprop-2-ylamino)-8-isopropyl-4-[4-phényl(pyridin-3-yl)méthylamino]pyrazolo[1,5-*a*]-1,3,5-triazine (**24**)
- (*R*) 2-(1-hydroxy-4-méthylpent-2-ylamino)-8-isopropyl-4-[4-phényl(pyridin-3-yl)méthylamino]pyrazolo[1,5-*a*]-1,3,5-triazine (**25**)
- (*S*) 2-(1-hydroxy-4-méthylpent-2-ylamino)-8-isopropyl-4-[4-phényl(pyridin-3-yl)méthylamino]pyrazolo[1,5-*a*]-1,3,5-triazine (**26**)
- (*S*) 2-(1-hydroxy-3,3-diméthylbut-2-ylamino)-8-isopropyl-4-[4-(pyridin-2-yl)benzylamino]pyrazolo[1,5-*a*]-1,3,5-triazine (**27**)
- (*S*) 2-(1-hydroxy-3-méthylbut-2-ylamino)-8-isopropyl-4-[4-(pyridin-2-yl)benzylamino]pyrazolo[1,5-*a*]-1,3,5-triazine (**28**)
- 2-[4-[1-[8-isopropyl-4-[[4-(pyridin-2-yl)phényl]méthylamino]pyrazolo[1,5-a]-1,3,5-triazin-2-yl]pipéridin-4-yl]pipéridin-1-yl]éthanol (**29**)
- 2-[4-[8-isopropyl-4-[[4-(pyridin-2-yl)phényl]méthylamino]pyrazolo[1,5-*a*]-1,3,5-triazin-2-yl]pipérazin-1-yl]éthanol (**30**)
- (*R*) 2-(1,2-dihydroxypropan-3-ylamino)-8-éthyl-4-[4-(pyridin-2-yl)benzylamino]pyrazolo[1,5-*a*]-1,3,5-triazine (**31**)
- [(2R)-3-[[8-ethyl-4-[[4-(2-pyridyl)phenyl]methylamino]pyrazolo[1,5-a][1,3,5]triazin-2-yl]amino]-2-hydroxy-propyl] (2R)-2-Bocamino-3-methyl-butanoate (**32**)
- [(2R)-3-[[8-ethyl-4-[[4-(2-pyridyl)phenyl]methylamino]pyrazolo[1,5-a][1,3,5]triazin-2-yl]amino]-2-hydroxy-propyl] (2R)-2-amino-3-methyl-butanoate (**33**) à l'état de base ou de sel notamment de dichlorhydrate
- (*S*) 2-(1,2-dihydroxypropan-3-ylamino)-8-éthyl-4-[4-(pyridin-2-yl)benzylamino]pyrazolo[1,5-*a*]-1,3,5-triazine (**34**)
- (*S*) 8-ethyl-2-(-1-hydroxybut-2-ylamino)-4-[4-(pyridin-2-yl)benzylamino]pyrazolo[1,5-*a*]-1,3,5-triazine (**35**)
- (*R*) 8-ethyl-2-(-1-hydroxybut-2-ylamino)-4-[4-(pyridin-2-yl)benzylamino]pyrazolo[1,5-*a*]-1,3,5-triazine (**36**)
- 2-(1,3-dihydroxyprop-2-ylamino)-8-éthyl-4-[4-(pyridin-2-yl)benzylamino]pyrazolo[1,5-*a*]-1,3,5-triazine (**37**)
- 2-[(2*S*)-1-[8-éthyl-4-[[4-(2-pyridyl)phényl]methylamino]pyrazolo[1,5-a][1,3,5]triazin-2-yl]-2-pipéridyl]éthanol (**38**)
- 2-[[8-éthyl-4-[[4-(2-pyridyl)phényl]méthylamino]-pyrazolo[1,5-a][1,3,5]triazin-2-yl]-(2-hydroxyéthyl)amino]éthanol (**39**)
- (2*R*,3*R*)-2-[[8-éthyl-4-[[4-(2-pyridyl)phényl]méthylamino]pyrazolo[1,5-a][1,3,5]triazin-2-yl]amino]butane-1,3-diol (**40**)
- (2*S*,3*S*)-2-[[8-ethyl-4-[[4-(2-pyridyl)phényl]méthylamino]pyrazolo[1,5-a][1,3,5]triazin-2-yl]amino]butane-1,3-diol (**41**)
ainsi que leurs éventuels sels d'acides pharmaceutiquement acceptables,

11. Procédé de préparation d'un composé de formule (**I**) selon l'une quelconque des revendications précédentes, **caractérisé en ce que** l'on fait réagir un composé de formule (**IIa**) qui suit : 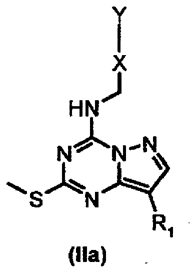 dans laquelle X, Y et R₁ sont tels que définis en revendication 1, avec de l'acide métachloroperbenzoïque dans une réaction d'oxydation et **en ce qu'**on engage la sulfone obtenue directement dans une réaction de substitution nucléophile en présence d'une amine primaire de formule NH-R₂R₉, où R₂ et R₉ sont tels que définis en revendication 1, à une température variant de 100 à 180°C, pour conduire à un composé de formule (**I**),
ou bien **en ce que** l'on fait réagir un composé de formule (**IIb**) 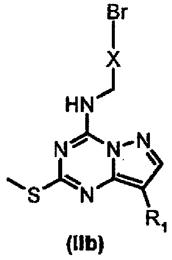 dans laquelle X et R₁ sont tels que définis en revendication 1, avec un métal comme par exemple le palladium ou le nickel, pour donner un composé de formule (**IIa**) tel que défini ci-dessus, ou alternativement, **en ce que** l'on fait réagir un composé de formule (**IIb**) tel que défini ci-dessus avec de l'acide méta-chloroperbenzoïque dans une réaction d'oxydation, la sulfone obtenue étant directement engagée dans une réaction de substitution nucléophile en présence d'une amine primaire de formule NH-R₂R₉, où R₂ et R₉ sont tels que définis dans la revendication 1, pour donner un composé de formule (**III**) : 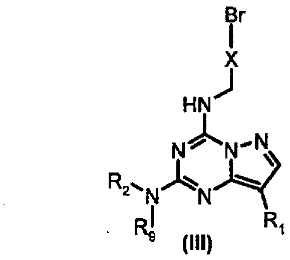 dans laquelle X, R₁ et R₂ sont tels que définis dans la revendication 1, que l'on soumet à une réaction de couplage catalysée par un métal comme par exemple le palladium ou le nickel, pour conduire à un composé de formule (**I**).

12. Médicament, **caractérisé en ce qu'**il comprend un composé de formule (I) selon l'une quelconque des revendications 1 à 10.

13. Composition pharmaceutique, **caractérisée en ce qu'**elle comprend un composé de formule (I) selon l'une quelconque des revendications 1 à 10, ainsi qu'au moins un excipent pharmaceutiquement acceptable.

14. Composé de formule (**I**) selon l'une quelconque des revendications 1 à 10, pour son utilisation à titre de médicament destiné au traitement et à la prévention des maladies suivantes : cancers, maladies neurodégénératives chroniques telles que maladies d'Alzheimer, de Parkinson, maladies neurodégénératives aigües telles que traumatisme cérébral, accident vasculaire cérébral, épilepsie, inflammations pulmonaires, arthrite, infections virales (SIDA, Herpes), traitement de la douleur, diabète, en particulier de type 2, maladies rénales telle que la polykystose rénale ou les glomérulonéphrites, leucémies telles que la leucémie lymphoïde chronique, en particulier de type B, parasites tels que *Plasmodium* et *Leishmania.*

## Patentansprüche

1. Verbindung der folgenden Formel (**I**): 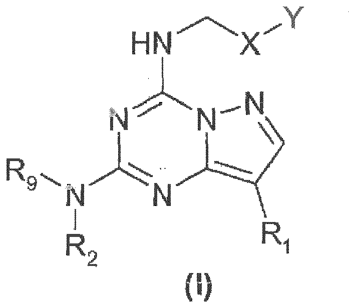 worin:
R₁ eine (C₁-C₆)-Alkylgruppe oder eine (C₃-C₆)-Cycloalkylgruppe darstellt,
R₂ eine (C₁-C₆)-Alkylgruppe, eine (C₃-C₆)-Cycloalkylgruppe, eine (C₁-C₆)-Alkenylgruppe, eine (C₁-C₆)-Fluoralkylgruppe, eine (C₁-C₃)-Fluoralkoxygruppe oder eine (C₁-C₆)-Alkoxy-(C₁-C₆)-alkylgruppe darstellt, die (i) mit ein bis drei Hydroxygruppen substituiert ist, oder (ii) mit einer Gruppe NRₐR_{b} substituiert ist, worin Rₐ und R_{b} unabhängig ein Wasserstoffatom oder eine (C₁-C₃)-Alkylgruppe darstellen,
oder R₂ eine Pyrrolidinylmethylgruppe darstellt, die mit ein bis drei Hydroxygruppen substituiert ist,
worin die Gruppe R₂ gegebenenfalls mit einer Gruppe -OCOR₃ substituiert ist, worin R₃ ein natürliches oder nicht natürliches Aminosäurederivat oder eine
Piperidylgruppe der Formel (B) darstellt 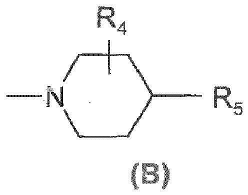 worin:
R₄ ein Wasserstoff, ein Halogenatom, eine (C₁-C₃)-Alkylgruppe, eine Hydroxy-(C₁-C₃)-alkylgruppe oder eine Gruppe -NRₐR_{b} darstellt, worin Rₐ und R_{b} wie vorstehend definiert sind, und R₅ ein Wasserstoff, eine (C₁-C₃)-Alkylgruppe, eine Gruppe -N(Me)₂, eine Piperidylgruppe oder eine Morpholinylgruppe darstellt,
wobei R₉ die gleiche Bedeutung wie R₂ hat und auch ein Wasserstoffatom darstellen kann,
alternativ R₂ und R₉ gemeinsam mit dem das sie tragende Stickstoffatom einen Heterozyklus bilden können, ausgewählt aus den Gruppen: Pyrrolidinyl, Piperidinyl, Piperazinyl und Piperidinylpiperidinyl, wobei diese Heterozyklen mit ein bis drei Gruppen substituiert sein können, ausgewählt aus Hydroxy, (C₁-C₆)-Alkyl oder (C₁-C₆-Alkoxy-(C₁-C₆)-alkyl, wobei die zwei letztgenannten Gruppen substituiert sind mit: (i) ein bis drei Hydroxygruppen oder (ii) einer Gruppe NRₐR_{b}, worin Rₐ und R_{b} unabhängig ein Wasserstoffatom oder eine (C₁-C₃)-Alkylgruppe darstellen,
X und Y unabhängig eine Phenylgruppe oder eine Heteroarylgruppe darstellen, wobei die Heteroarylgruppen und Phenylgruppe mit ein oder zwei Gruppen substituiert sein können, die unabhängig ausgewählt sind aus einer (C₁-C₂)-Alkylgruppe, einer (C₁-C₂)-Alkoxygruppe, einem Halogenatom, einer (C₁-C₂)-Fluoralkylgruppe, einer (C₁-C₂)-Fluoralkoxygruppe, einer Hydroxygruppe, einer -COOH-Gruppe, einer Gruppe -CONHR₆ und einer Gruppe -NRₐR_{b}, wobei Rₐ und R_{b} wie vorstehend definiert sind,
R₆ ein Wasserstoff oder eine (C₁-C₃)-Alkylgruppe ist,
worin die Heteroarylgruppe aus einer Thienylgruppe, einer Pyridylgruppe, einer Pyrimidinylgruppe, einer Thiazolylgruppe, einer Pyrrolylgruppe und einer Furanylgruppe ausgewählt ist,
sowie pharmazeutisch akzeptable Salze davon.

2. Verbindung nach Anspruch 1, **dadurch gekennzeichnet, dass** R₃ eine der folgenden Formeln (**a-1**) bis (**a-5**) darstellt: 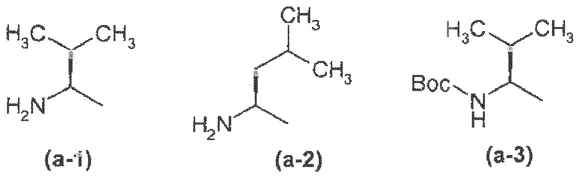 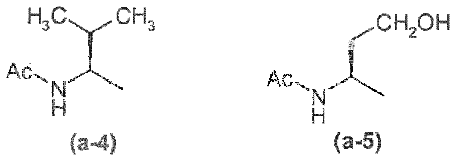 worin Boc eine tert-Butoxycarbonylgruppe darstellt und Ac eine Acetylgruppe darstellt, sowie die Enantiomere davon.

3. Verbindung nach Anspruch 1 oder 2, **dadurch gekennzeichnet, dass** mindestens eine der Gruppen X und Y substituiert ist und ein oder zwei Substitutions-gruppen trägt, wobei diese Substitutionsgruppe ausgewählt ist aus einer (C₁-C₂)-Alkylgruppe, einer (C₁-C₂-Fluoralkylgruppe, einer (C₁-C₂)-Alkoxygruppe, einer (C₁-C₂)-Fluoralkoxygruppe, einem Halogenatom, einer Hydroxygruppe und einer Gruppe -COOH:

4. Verbindung nach einem der vorhergehenden Ansprüche, **dadurch gekennzeichnet, dass** R₂ eine der folgenden Formeln darstellt: 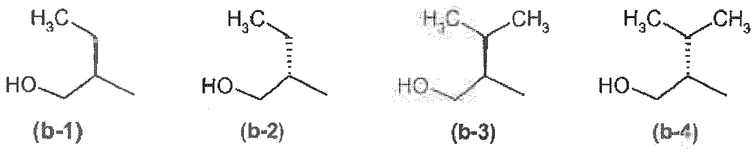 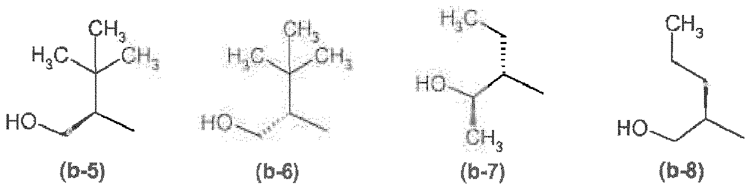 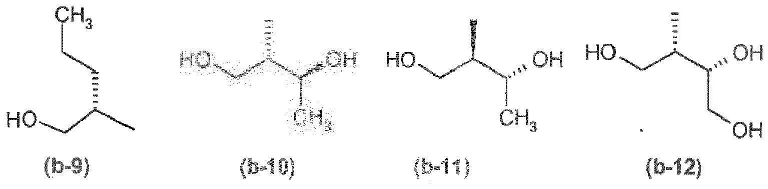 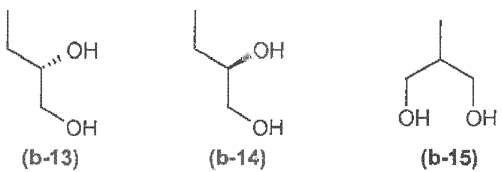 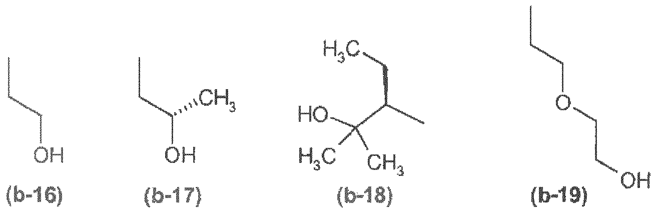 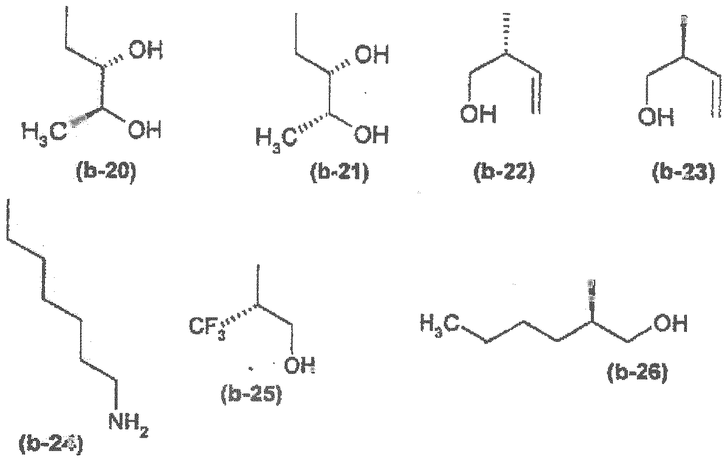 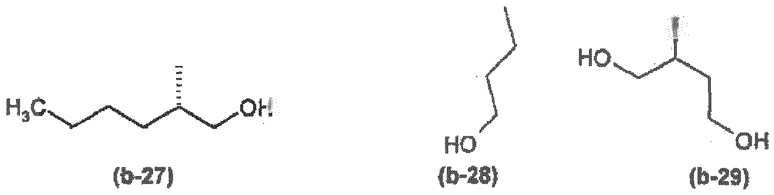 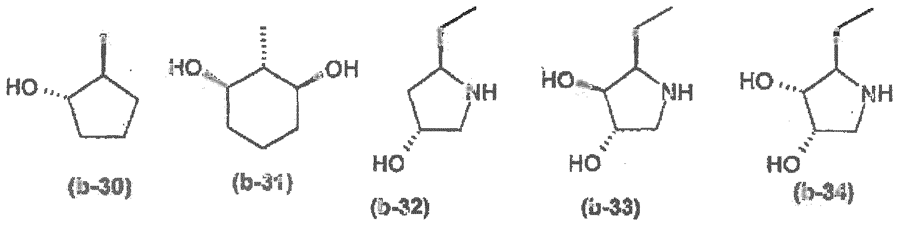 und insbesondere eine der Formeln (**b-1**), (**b-2**), (**b-10**), (**b-11**), (**b-13**), (**b-14**) oder (**b-15**), oder R₂ und R₉ zusammen mit dem sie tragenden Stickstoffatom eine der folgenden Formeln bilden: 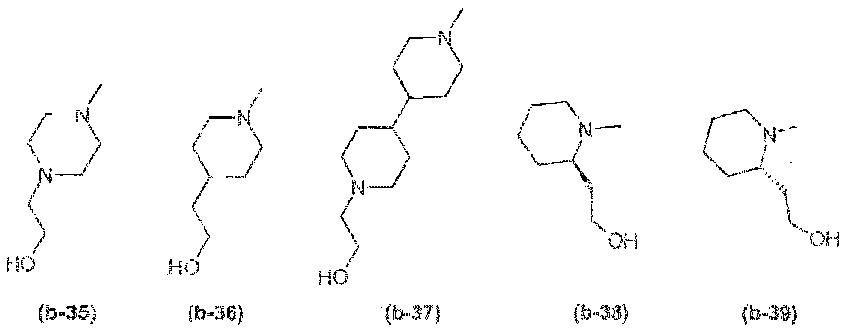 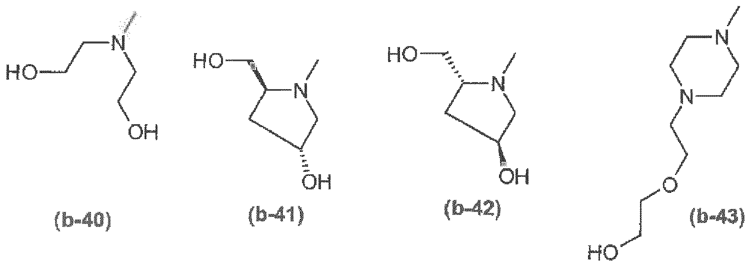 und insbesondere eine der Formeln (**b-37**), (**b-38**) (**b-39**) oder (**b-40**).

5. Verbindung nach einem der vorhergehenden Ansprüche, **dadurch gekennzeichnet, dass** sie eine Formel (**Ia**) aufweist: 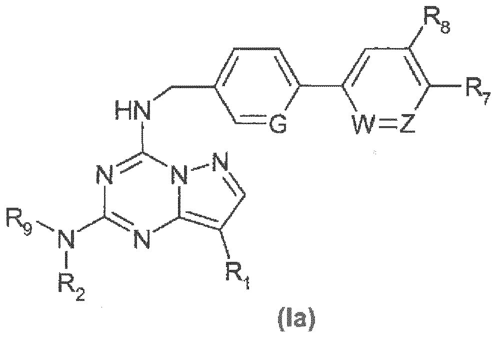 worin:
R₁ so wie für Anspruch 1 definiert ist und bevorzugt eine Isopropylgruppe ist,
R₂ und R₉ unabhängig eine (C₁-C₆)-Alkylgruppe, eine (C₁-C₆)-Fluoralkylgruppe, eine (C₁-C₃)-Fluoralkoxygruppe oder eine (C₁-C₆)-Alkoxy-(C₁-C₆)-alkylgruppe darstellen, wobei die Gruppe mit ein bis drei Hydroxygruppen substituiert ist, wobei R₉ ein Wasserstoffatom darstellen kann, und R₂ bevorzugt eine der Formeln (**b-1**), (**b-2**), (**b-10**), (**b-11**), (**b-13**), (**b-14**) oder (**b-15**) wie oben definiert darstellt, während R₉ ein Wasserstoffatom ist, oder,
R₂ und R₉ zusammen mit dem sie tragenden Stickstoffatom eine Piperidin-1-yl-Gruppe oder Piperidin-4-ylpiperidin-1-yl-Gruppe darstellen, wobei die Gruppe mit ein bis drei (C₁-C₆)-Alkylgruppen, die mit einer Hydroxygruppe substituiert sind, substituiert ist, und R₂ und R₉ zusammen genommen bevorzugt eine der Formeln (**b-37**), (**b-38**), (**b-39**) oder (**b-40**) wie oben definiert darstellen,
R₇ und R₈ unabhängig ein Wasserstoffatom, eine (C₁-C₂)-Alkylgruppe, eine (C₁-C₂)-Fluoralkylgruppe, eine (C₁-C₂)-Alkoxygruppe, eine (C₁-C₂)-Fluoralkoxygruppe, ein Halogenatom, eine Hydroxygruppe oder eine Gruppe -COOH darstellen,
G -CH= oder -N= bedeutet, und
wenn G -CH= bedeutet, die Gruppen W und Z gleichzeitig -CH= darstellen oder eines -N= darstellt und das andere -CH= darstellt,
wenn G -N= darstellt, W und Z -CH= darstellen,
oder pharmazeutisch akzeptable Salze davon.

6. Verbindung nach einem der Ansprüche 1 bis 4, **dadurch gekennzeichnet, dass** sie eine Formel (**Ib**) aufweist: 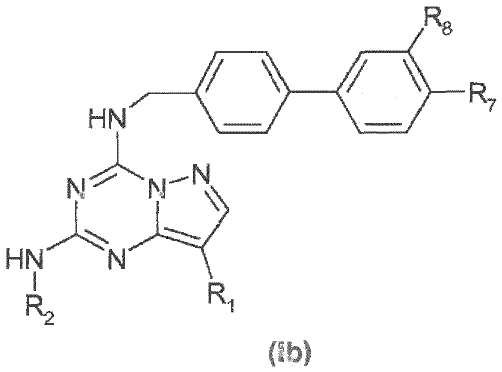 worin:
R₁ wie in Anspruch 1 definiert ist und bevorzugt eine Isopropylgruppe ist, R₂, R₇ und R₈ wie im vorhergehenden Anspruch definiert sind,
oder pharmazeutisch akzeptable Salze davon.

7. Verbindung nach einem der Ansprüche 1 bis 4, **dadurch gekennzeichnet, dass** sie eine Formel (**Ic**) aufweist: 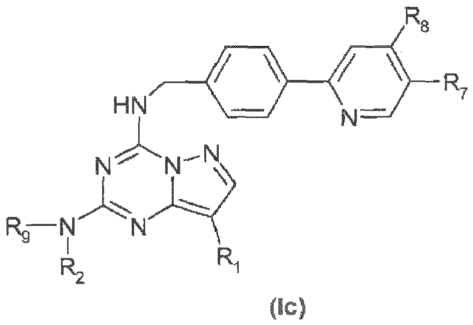 worin:
R₁ wie in Anspruch 1 definiert ist und bevorzugt eine Ethyl- oder Isopropylgruppe ist,
R₉ ein Wasserstoffatom ist und R₂ eine (C₁-C₆)-Alkylgruppe, eine (C₁-C₆)-Fluoralkylgruppe, eine (C₁-C₃)-Fluoralkoxygruppe oder eine (C₁-C₆)-Alkoxy-(C₁-C₆)-alkylgruppe ist, wobei die Gruppe mit ein bis drei Hydroxygruppen substituiert ist und bevorzugt eine der wie oben definierten Formeln (**b-1**), (**b-2**), (**b-10**), (**b-11**), (**b-13**), (**b-14**) oder (**b-15**) darstellt, oder
R₂ und R₉ zusammen mit dem sie tragenden Stickstoffatom eine Piperidin-1-yl-Gruppe oder Piperidin-4-yl-piperidin-1-yl-Gruppe darstellen, wobei die Gruppe mit ein bis drei (C₁-C₆)-Alkylgruppen, die mit einer Hydroxygruppe substituiert sind, substituiert ist, und R₂ und R₉ zusammen genomen bevorzugt eine der Formeln (**b-37**)**,** (**b-38**), (**b-39**) oder (**b-40**) wie oben definiert darstellen, und
R₇ und R₈ wie in Anspruch 5 definiert sind,
oder pharmazeutisch akzeptable Salze davon.

8. Verbindung nach einem der Ansprüche 1 bis 4, **dadurch gekennzeichnet, dass** sie die Formel (**Id**) aufweist: 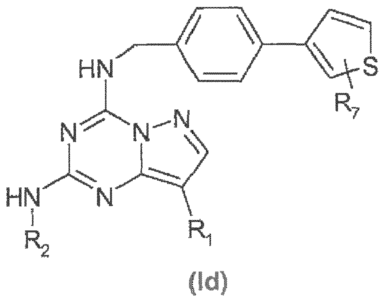 worin:
R₁ wie in Anspruch 1 definiert ist und bevorzugt eine Isopropylgruppe ist,
R₂ und R₇ wie in Anspruch 5 definiert sind,
oder pharmazeutisch akzeptable Salze davon.

9. Verbindung nach einem der Ansprüche 1 bis 4, **dadurch gekennzeichnet, dass** sie eine Formel (Ie) aufweist: 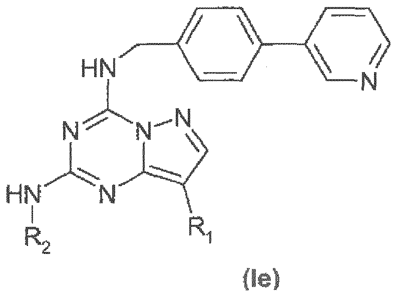 worin:
R₁ wie für Anspruch 1 definiert ist und bevorzugt eine Isopropylgruppe ist,
R₂ eine (C₁-C₆)-Alkylgruppe, eine (C₁-C₆)-Fluoralkylgruppe, eine (C₁-C₃)-Fluor-alkoxygruppe oder eine (C₁-C₆)-Alkoxy-(C₁-C₆)-alkylgruppe ist, wobei die Gruppe mit ein bis drei Hydroxygruppen substituiert ist und bevorzugt eine der Formeln (**b-1**), (**b-2**), (**b-10**), (**b-11**), (**b-13**), (**b-14**) oder (**b-15**) wie oben definiert darstellt, oder eine Formel (**If**): 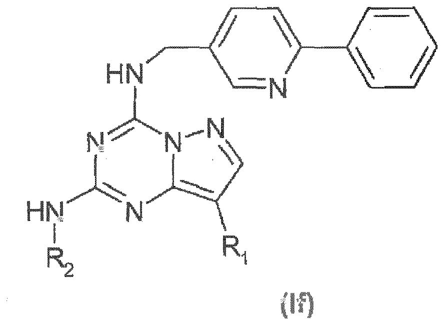
worin
R₁ wie für Anspruch 1 definiert ist und bevorzugt eine Isopropylgruppe ist, und
R₂ eine (C₁-C₆)-Alkylgruppe, eine (C₁-C₆)-Fluoralkylgruppe, eine (C₁-C₃)-Fluor-alkoxygruppe oder eine (C₁-C₆)-Alkoxy-(C₁-C₆)-alkylgruppe ist, wobei die Gruppe mit ein bis drei Hydroxygruppen substituiert ist und bevorzugt eine der Formeln (**b-1**), (**b-2**), (**b-10**), (**b-11**), (**b-13**), (**b-14**) oder (**b-15**) wie oben definiert darstellt.

10. Verbindung nach einem der vorhergehenden Ansprüche, **dadurch gekennzeichnet, dass** sie aus den folgenden Verbindungen ausgewählt ist:
- (*R*)-2-(1-Hydroxybut-2-ylamino)-8-isopropyl-4-(4-phenylbenzylamino)-pyrazolo[1,5-*a*]-1,3,5-triazin (**1**)
- (*R*)-2-(1-Hydroxybut-2-ylamino)-8-isopropyl-4-(4-(pyridin-2-yl)benzylamino]-pyrazolo[1,5-*a*]-1,3,5-triazin (**2**)
- Fumaratsalz von (*R*)-2-(1-Hydroxybut-2-ylamino)-8-isopropyl-4-[4-(pyridin-2-yl)benzylamino]pyrazolo[1,5-*a*]-1,3,5-triazin (**3**)
- (*S*)-2-(1-Hydroxybut-2-ylamino)-8-isopropyl-4-[4-(pyridin-2-yl)benzylamino]-pyrazolo[1,5-*a*]-1,3,5-triazin (**4**)
- Fumaratsalz von (*S*)-2-(1-Hydroxybut-2-ylamino)-8-isopropyl-4-[4-(pyridin-2-yl)benzylamino]pyrazolo[1,5-*a*]-1,3,5-triazin (**5**)
- (*R*)-2-(1-Hydroxybut-2-ylamino)-8-isopropyl-4-[4-(thiophen-3-yl)benzylamino]-pyrazolo[1,5-*a*]-1,3,5-triazin (**6**)
- (*S*)-2-(1-Hydroxybut-2-ylamino)-8-isopropyl-4-[4-(thiophen-3-yl)benzylamino]-pyrazolo [1,5-*a*]-1,3,5-triazin (**7**)
- (*S*)-2-(1,2-Dihydroxypropan-3-ylamino)-8-isopropyl-4-[4-(pyridin-2-yl)benzyl-amino]pyrazolo[1,5-*a*]-1,3,5-triazin (**8**)
- Fumaratsalz von (*S*)-2-(1,2-Dihydroxypropan-3-ylamino)-8-isopropyl-4-[4-(pyridin-2-yl)benzylamino]pyrazolo[1,5-*a*]-1,3,5-triazin (**9**)
- (*R*)-2-(1,2-Dihydroxypropan-3-ylamino)-8-isopropyl-4-[4-(pyridin-2-yl)benzyl-amino]pyrazolo[1,5-*a*]-1,3,5-triazin (**10**)
- Fumaratsalz von (*R*)-2-(1,2-Dihydroxypropan-3-ylamino)-8-isopropyl-4-[4-(pyridin-2-yl)benzylamino]pyrazolo[1,5-*a*]-1,3,5-triazin (**11**)
- (2*R*,3*R*)-2-(1,3-Dihydroxybut-2-ylamino)-8-isopropyl-4-[4-(pyridin-2-yl)benzyl-amino]pyrazolo[1,5-*a*]-1,3,5-triazin (**12**)
- Fumaratsalz von (2*R*,3*R*)-2-(1,3-Dihydroxybut-2-ylamino)-8-isopropyl-4-[4-(pyridin-2-yl)benzylamino]pyrazolo[1,5-*a*]-1,3,5-triazin (**13**)
- (2*S*,3*S*)-2-(1,3-Dihydroxybut-2-ylamino)-8-isopropyl-4-[4-(pyridin-2-yl)benzyl-amino]pyrazolo[1,5-*a*]-1,3,5-triazin (**14**)
- Fumaratsalz von (2*S*,3*S*)-2-(1,3-Dihydroxybut-2-ylamino)-8-isopropyl-4-[4-(pyridin-2-yl)benzylamino]pyrazolo[1,5-*a*]-1,3,5-triazin (**15**)
- (2*R*,3*R*)-2-(1,3-Dihydroxybut-2-ylamino)-8-isopropyl-4-[4-(thiophen-3-yl)benzylamino]pyrazolo[1,5-*a*]-1,3,5-triazin (**16**)
- 2-(1,3-Dihydroxyprop-2-ylamino)-8-isopropyl-4-[4-(pyridin-2-yl)benzylamino]-pyrazolo[1,5-*a*]-1,3,5-triazin (**17**)
- Fumaratsalz von 2-(1,3-Dihydroxyprop-2-ylamino)-8-isopropyl-4-[4-(pyridin-2-yl)benzylamino]pyrazolo[1,5*-a*]-1,3,5-triazin (**18**)
- 2-(1,3-Dihydroxyprop-2-ylamino)-8-isopropyl-4-[4-(pyridin-3-yl)benzylamino]-pyrazolo[1,5-*a*]-1,3,5-triazin (**19**)
- (*S*)-2-(1-Hydroxybut-2-ylamino)-8-isopropyl-4-[4-(pyridin-3-yl)benzylamino]-pyrazolo[1,5-*a*]-1,3,5-triazin (**20**)
- (2*S*, 3*S*)-2-(1,3-Dihydroxybut-2-ylamino)-8-isopropyl-4-[4-phenyl(pyridin-3-yl)methylamino]-pyrazolo[1,5-*a*]-1,3,5-triazin (**21**)
- (*S*)-2-(1-Hydroxybut-2-ylamino)-8-isopropyl-4-[4-phenyl(pyridin-3-yl)methyl-amino]pyrazolo[1,5-*a*]-1,3,5-triazin (**22**)
- (*2R*,*3R*)-2-(1,3-Dihydroxybut-2-ylamino)-8-isopropyl-4-[4-phenyl(pyridin-3-yl)methylamino]pyrazolo[1,5-*a*]-1,3,5-triazin (**23**)
- 2-(1,3-Dihydroxyprop-2-ylamino)-8-isopropyl-4-[4-phenyl(pyridin-3-yl)methyl-amino]pyrazolo[1,5-*a*]-1,3,5-triazin (**24**)
- (*R*)-2-(1-Hydroxy-4-methylpent-2-ylamino)-8-isopropyl-4-[4-phenyl(pyridin-3-yl)methylamino]pyrazolo[1,5-*a*]-1,3,5-triazin (**25**)
- (*S*)-2-(1-Hydroxy-4-methylpent-2-ylamino)-8-isopropyl-4-[4-phenyl(pyridin-3-yl)methylamino]pyrazolo[1,5-*a*]-1,3,5-triazin (**26**)
- (*S*)-2-(1-Hydroxy-3,3-dimethylbut-2-ylamino)-8-isopropyl-4-[4-(pyridin-2-yl)benzylamino]-pyrazolo[1,5-*a*]-1,3,5-triazin (**27**)
- (*S*)-2-(1-Hydroxy-3-methylbut-2-ylamino)-8-isopropyl-4-[4-(pyridin-2-yl)benzyl-amino]pyrazolo[1,5-*a*]-1,3,5-triazin (**28**)
- 2-[4-[1-[8-Isopropyl-4-[[4-(pyridin-2-yl)phenyl] methylamino]pyrazolo[1,5-*a*]-1,3,5-triazin-2-yl]piperidin-4-yl] piperidin-1-yl]ethanol (**29**)
- 2-[4-[8-Isopropyl-4-[[4-(pyridin-2-yl)phenyl]methylamino]-pyrazolo[1,5-*a*]-1,3,5-triazin-2-yl]piperazin-1-yl]ethanol (**30**)
- (*R*)-2-(1,2-Dihydroxypropan-3-ylamino)-8-ethyl-4-[4-(pyridin-2-yl)benzylamino]-pyrazolo[1,5-*a*]-1,3,5-triazin (**31**)
- [(2*R*)-3-[[8-ethyl-4-[[4-(2-pyridyl)phenyl]methylamino]pyrazolo[1,5-*a*][1,3,5]-triazin-2-yl]amino]-2-hydroxypropyl]-(2R)-2-Bocamino-3-methyl-3-butanoat (**32**)
- [(2*R*)-3-[[8-Ethyl-4-[[4-(2-pyridyl)phenyl]methylamino]pyrazolo[1,5-*a*]-[1,3,5]triazin-2-yl]amino]-2-hydroxy-propyl]-(2R)-2-amino-3-methyl-butanoat (**33**) in Form einer Base oder eines Salzes, insbesondere Dichlorhydrat,
- (*S*)-2-(1,2-Dihydroxypropan-3-ylamino)-8-ethyl-4-[4-(pyridin-2-yl)benzylamino]-pyrazolo[1,5-*a*]-1,3,5-triazin (**34**)
- (*S*)-8-Ethyl-2-(1-hydroxybut-2-ylamino)-4-[4-(pyridin-2-yl)benzylamino]-pyrazolo[1,5- *a*]-1,3,5-triazin (**35**)
- (*R*)-8-Ethyl-2-(1-hydroxybut-2-ylamino)-4-[4-(pyridin-2-yl)benzylamino]-pyrazolo[1,5-*a*]-1,3,5-triazin (**36**)
- 2-(1,3-Dihydroxyprop-2-ylamino)-8-ethyl-4-[4-(pyridin-2-yl)benzylamino]-pyrazolo[1,5-*a*]-1,3,5-triazin (**37**)
- 2-[(2*S*)-1-[8-Ethyl-4-[[4-(2-pyridyl)phenyl]methylamino]pyrazolo[1,5-*a*][1,3,5]-triazin-2-yl]-2-piperidyl]ethanol (**38**)
- 2-[[8-Ethyl-4-[[4-(2-pyridyl)phenyl]methylamino]pyrazolo[1,5*-a*][1,3,5]-triazin-2-yl]-(2-hydroxyethyl)amino]ethanol (**39**)
- (2*R*, 3*R*)-2-[[8-Ethyl-4-[[4-(2-pyridyl)phenyl]methylamino]pyrazolo[1,5-*a*][1,3,5]-triazin-2-yl]amino]butan-1,3-diol (**40**)
- (2*S*, 3*S*)-2-[[8-Ethyl-4-[[4-(2-pyridyl)phenyl]methylamino]pyrazolo[1,5-*a*][1,3,5]-triazin-2-yl]amino]butan-1,3-diol (**41**)
und deren optionale, pharmazeutisch akzeptable Säuresalze.

11. Verfahren zur Herstellung einer Verbindung der Formel (I) nach einem der vorhergehenden Ansprüche, **dadurch gekennzeichnet, dass** man eine Verbindung der folgenden Formel (IIa): 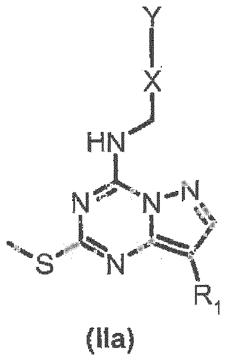 worin X, Y und R wie in Anspruch 1 definiert sind, mit Metachlorperbenzoesäure in einer Oxidationsreaktion umsetzt, und man das erhaltene Sulfon direkt bei einer Temperatur im Bereich von 100 bis 180°C einer nukleophilen Substitutionsreaktion in Gegenwart eines primären Amins der Formel NH-R₂R₉ unterwirft, worin R₂ und R₉ wie in Anspruch 1 definiert sind, um eine Verbindung der Formel (**I**) zu erhalten,
oder man eine Verbindung der Formel (**IIb**) 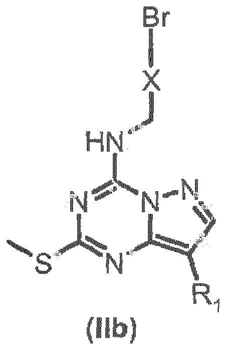 worin X und R₁ wie in Anspruch 1 definiert sind, mit einem Metall umsetzt, wie zum Beispiel Palladium oder Nickel, um eine Verbindung der Formel (**IIa**) wie oben definiert zu erhalten, oder man alternativ eine Verbindung der Formel (**IIb**) wie oben definiert mit Metachlorperbenzoesäure in einer Oxidationsreaktion umsetzt, und man das erhaltene Sulfon direkt in einer nukleophilen Substitutionsreaktion in Gegenwart eines primären Amins der Formel NH-R₂R₉ umsetzt, worin R₂ und R₉ wie in Anspruch 1 definiert sind, um eine Verbindung der Formel (**III**) zu erhalten: 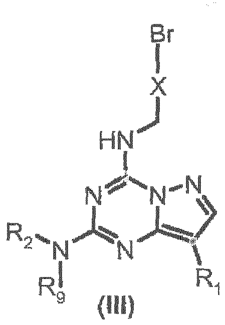 worin X, R₁ und R₂ wie in Anspruch 1 definiert wird, welche man einer katalysierten Kupplungsreaktion mit einem Metall wie beispielsweise Palladium oder Nickel unterzieht, um eine Verbindung der Formel (I) zu erhalten.

12. Medikament, **dadurch gekennzeichnet, dass** es eine Verbindung der Formel (**I**) nach einem der Ansprüche 1 bis 10 enthält.

13. Pharmazeutische Zusammensetzung, **dadurch gekennzeichnet, dass** sie eine Verbindung der Formel (**I**) nach einem der Ansprüche 1 bis 10 sowie mindestens einen pharmazeutisch akzeptablen Exzipienten enthält.

14. Verbindung der Formel (I) nach einem der Ansprüche 1 bis 10 zur Verwendung als Medikament für die Behandlung und Vorbeugung der folgenden Erkrankungen: Krebserkrankungen, chronische neurodegenerative Erkrankungen, wie Alzheimer, Parkinson, akute neurodegenerative Erkrankungen, wie zerebrales Trauma, Schlaganfall, Epilepsie, Lungenentzündungen, Arthritis, Virusinfektionen (AIDS, Herpes), zur Schmerzbehandlung, Diabetes, insbesondere Typ 2, Nierenerkrankungen, wie polyzystische Nieren oder Glomerulonephritis, Leukämien, wie chronische lymphatische Leukämie, insbesondere Typ B, Parasiten, wie *Plasmodium* und *Leishmania.*

## Claims

1. A compound of formula (I) below: 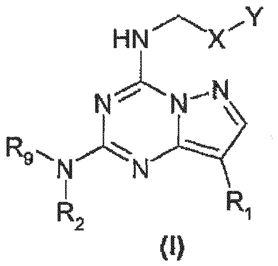 in which:
R₁ is a (C₁-C₆) alkyl group or a (C₃-C₆) cycloalkyl group,
R₂ is a (C₁-C₆)alkyl group, a (C₃-C₆) cycloalkyl group, a (C₁-C₆) alkenyl group, a (C₁-C₆)fluoroalkyl group, a (C₁-C₃) fluoroalkoxy group or a (C₁-C₆)alkoxy(C₁-C₆)alkyl group, substituted (i) with one to three hydroxyl groups, or (ii) with an NRₐR_{b} group, where Rₐ and R_{b} are independently a hydrogen atom or a (C₁-C₃)alkyl group,
or else R₂ is a pyrrolidinylmethyl group substituted with one to three hydroxyl groups,
said R₂ group being optionally substituted with an -OCOR₃ group, in which R₃ is a natural or unnatural amino acid derivative or a piperidyl group of formula (B) 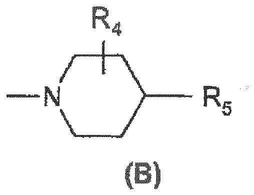 in which:
R₄ is a hydrogen, a halogen atom, a (C₁-C₃) alkyl group, a hydroxy (C₁-C₃) alkyl group or an -NRₐR_{b} group, where Rₐ and R_{b} are as defined above, and R₅ is a hydrogen, a (C₁-C₃) alkyl group, an -N(Me)₂ group, a piperidyl group or a morpholinyl group,
R₉ has the same meaning as R₂ and may also be a hydrogen atom,
alternatively, R₂ and R₉ together form, with the nitrogen atom which bears them, a heterocycle chosen from pyrrolidinyl, piperidinyl, piperazinyl and piperidinylpiperidinyl groups, it being possible for these heterocycles to be substituted with one to three groups chosen from hydroxyl, (C₁-C₆) alkyl or (C₁-C₆)alkoxy(C₁-C₆)alkyl, these last two groups being substituted (i) with one to three hydroxyl groups, or (ii) with an NRₐR_{b} group, where Rₐ and R_{b} are independently a hydrogen atom or a (C₁-C₃) alkyl group,
X and Y are independently a phenyl group or a heteroaryl group, it being possible for said heteroaryl and phenyl groups to be substituted with one or two groups independently chosen from a (C₁-C₂)alkyl group, a (C₁-C₂)alkoxy group, a halogen atom, a (C₁-C₂)fluoroalkyl group, a (C₁-C₂)fluoroalkoxy group, a hydroxyl group, a -COOH group, a -CONHR₆ group and an -NRₐR_{b} group, where Rₐ and R_{b} are as defined above,
R₆ is a hydrogen or a (C₁-C₃)alkyl group,
said heteroaryl group being chosen from a thienyl group, a pyridyl group, a pyrimidinyl group, a thiazolyl group, a pyrrolyl group and a furanyl group,
and also the pharmaceutically acceptable salts thereof.

2. The compound as claimed in claim 1, **characterized in that** R₃ is one of the following formulae (a-1) to (a-5) : 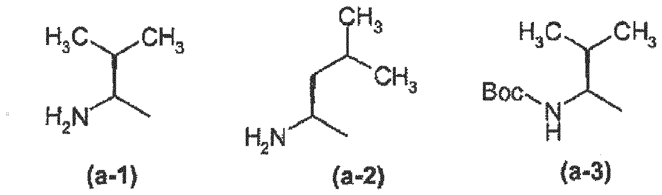 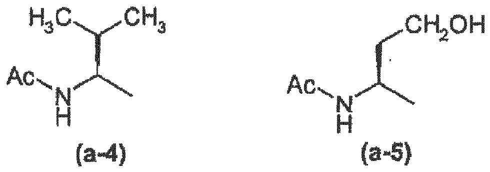 where Boc is a tert-butoxycarbonyl group and Ac an acetyl group, and also the enantiomers thereof.

3. The compound as claimed in claim 1 or 2, **characterized in that** at least one of the X and Y groups is substituted and comprises one or two substitution groups, said substitution group being chosen from a (C₁-C₂) alkyl group, a (C₁-C₂)fluoroalkyl group, a (C₁-C₂)alkoxy group, a (C₁-C₂)fluoroalkoxy group, a halogen atom, a hydroxyl group and a -COOH group.

4. The compound as claimed in any one of the preceding claims, **characterized in that** R₂ is one of the following formulae: 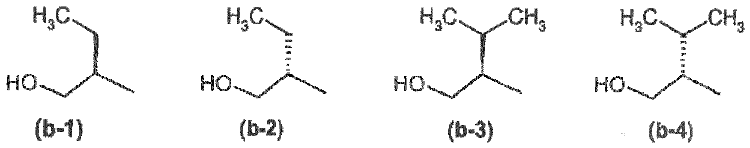 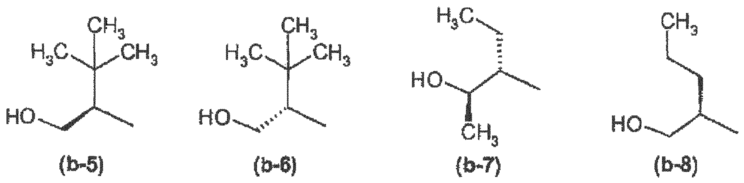 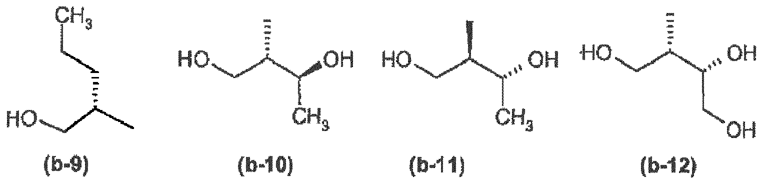 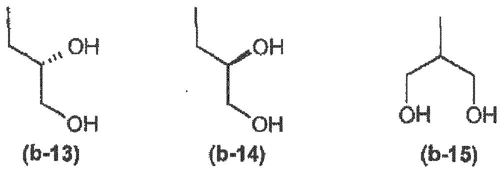 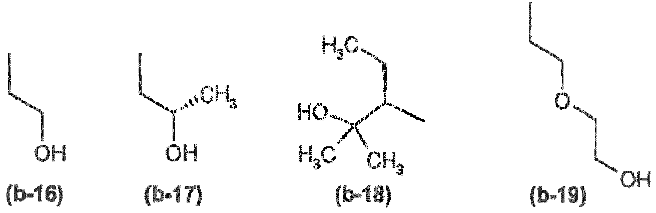 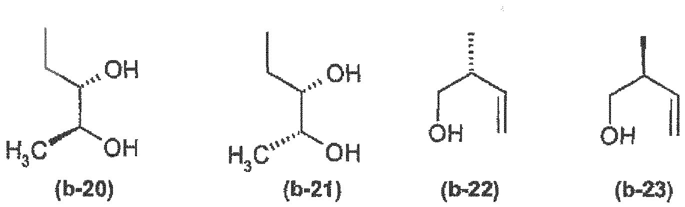 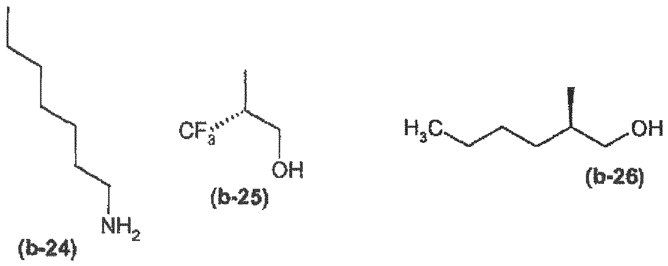 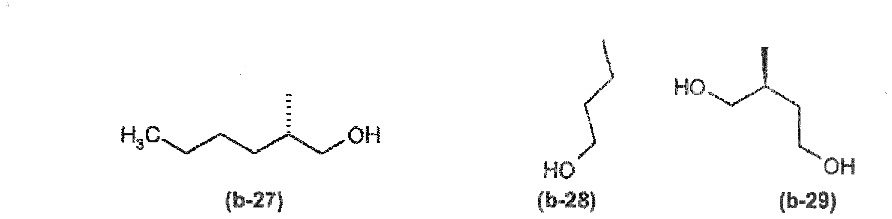 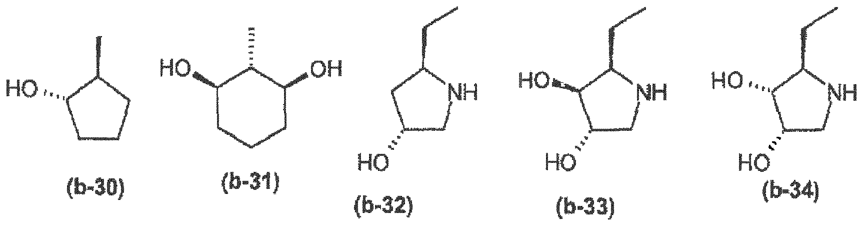 and in particular one of the formulae (**b-1**), (**b-2**), (**b-10**), (**b-11**), (**b-13**), (**b-14**) or (**b-15**), or else R₂ and R₉ together form, with the nitrogen atom which bears them, one of the following formulae: 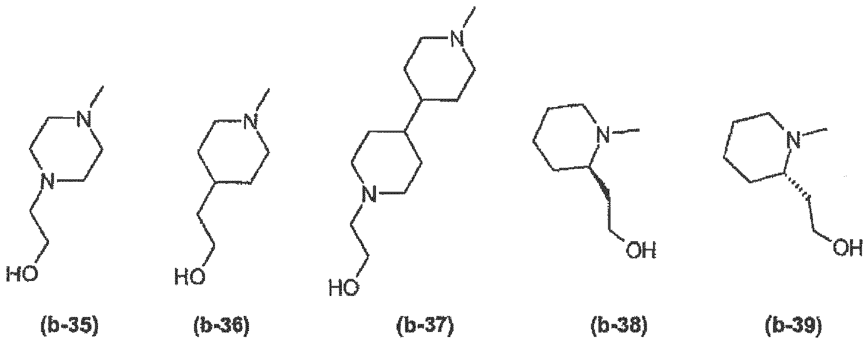 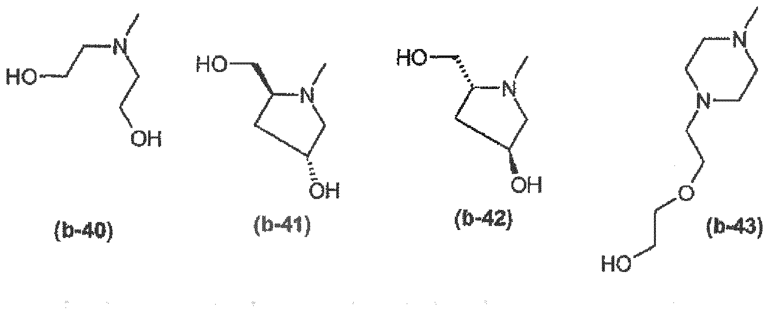 and, in particular, one of the formulae (**b-37**), (**b-38**), (**b-39**) or (**b-40**).

5. The compound as claimed in any one of the preceding claims, **characterized in that** it has a formula (Ia): 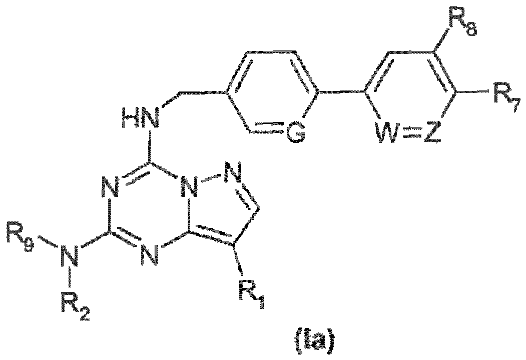 in which:
R₁ is as defined in claim 1 and is advantageously an isopropyl group,
R₂ and R₉ are independently a (C₁-C₆)alkyl group, a (C₁-C₆)fluoroalkyl group, a (C₁-C₃)fluoroalkoxy group or a (C₁-C₆)alkoxy(C₁-C₆)alkyl group, said group being substituted with one to three hydroxyl groups, with it being possible for R₉ to be a hydrogen atom, and R₂ advantageously is one of the formulae (**b-1**), (**b-2**), (**b-10**), (**b-11**), (**b-13**), (**b-14**) or (**b-15**) as defined above, while R₉ is a hydrogen atom,
or else R₂ and R₉ together form, with the nitrogen atom which bears them, a piperidin-1-yl or
piperidin-4-ylpiperidin-1-yl group, said group being substituted with one to three (C₁-C₆) alkyl groups substituted with a hydroxyl group, and R₂ and R₉ taken together are advantageously one of the formulae (**b-37**), (**b-38**), (**b-39**) or (**b-40**) as defined above,
R₇ and R₈ are independently a hydrogen atom, a (C₁-C₂)alkyl group, a (C₁-C₂) fluoroalkyl group, a (C₁-C₂) alkoxy group, a (C₁-C₂)fluoroalkoxy group, a halogen atom, a hydroxyl group or a -COOH group,
G is -CH=, or -N=, and
when G is -CH=, the W and Z groups are either simultaneously -CH=, or one is -N= and the other is -CH=,
when G is -N=, then W and Z are -CH=,
or a pharmaceutically acceptable salt thereof.

6. The compound as claimed in any one of claims 1 to 4, **characterized in that** it has a formula (Ib): 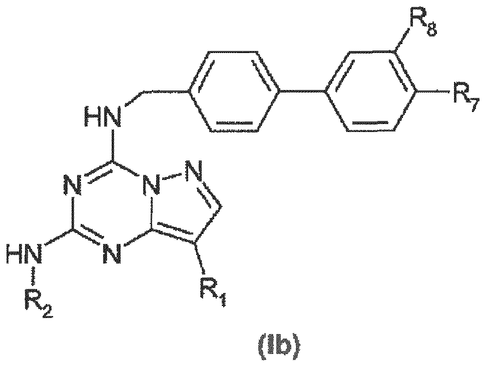 in which:
R₁ is as defined in claim 1 and is advantageously an isopropyl group,
R₂, R₇ and R₈ are as defined in the preceding claim,
or a pharmaceutically acceptable salt thereof.

7. The compound as claimed in any one of claims 1 to 4, **characterized in that** it has a formula (Ic): 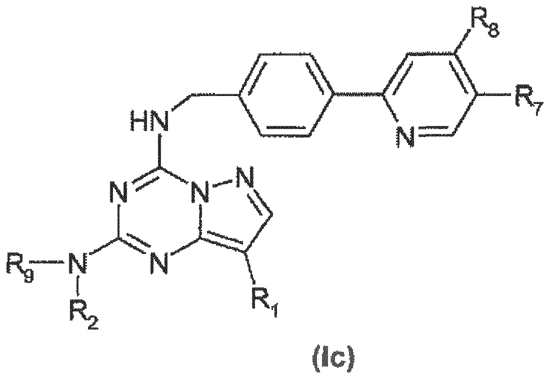 in which:
R₁ is as defined in claim 1 and is advantageously an ethyl or isopropyl group,
either R₉ is a hydrogen atom and R₂ is a (C₁-C₆) alkyl group, a (C₁-C₆) fluoroalkyl group, a (C₁-C₃) fluoroalkoxy group or a (C₁-C₆) alkoxy (C₁-C₆) alkyl group, said group being substituted with one to three hydroxyl groups, and advantageously is one of the formulae (**b-1**), (**b-2**), (**b-10**), (**b-11**), (**b-13**), (**b-14**) or (**b-15**) as defined above,
or alternatively R₂ and R₉ together form, with the nitrogen atom which bears them, a piperidin-1-yl or piperidin-4-ylpiperidin-1-yl group, said group being substituted with one to three (C₁-C₆)alkyl groups substituted with a hydroxyl group, and R₂ and R₉ taken together are advantageously one of the formulae (**b-37**), (**b-38**), (**b-39**) or (**b-40**) as defined above, and
R₇ and R₈ are as defined in claim 5,
or a pharmaceutically acceptable salt thereof.

8. The compound as claimed in any one of claims 1 to 4, **characterized in that** it has a formula (**Id**): 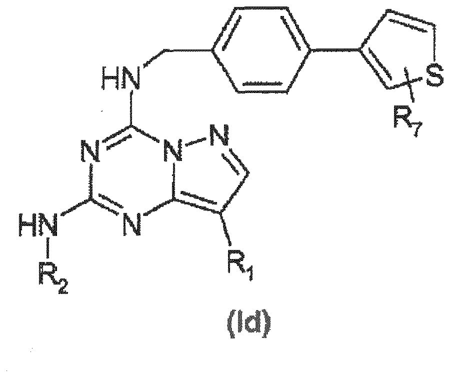 in which:
R₁ is as defined in claim 1 and is advantageously an isopropyl group,
R₂ and R₇ are as defined in claim 5,
or a pharmaceutically acceptable salt thereof.

9. The compound as claimed in any one of claims 1 to 4, **characterized in that** it has a formula (Ie): 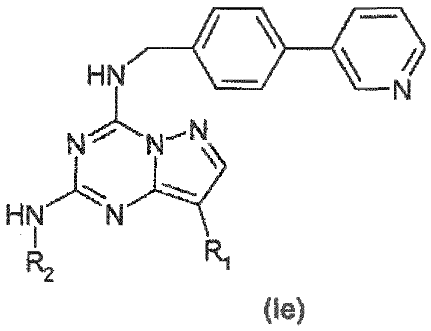 in which:
R₁ is as defined in claim 1 and is advantageously an isopropyl group,
R₂ is a (C₁-C₆) alkyl group, a (C₁-C₆)fluoroalkyl group, a (C₁-C₃) fluoroalkoxy group or a (C₁-C₆)alkoxy(C₁-C₆)alkyl group, said group being substituted with one to three hydroxyl groups, and advantageously is one of the formulae (**b-1**), (**b-2**), (**b-10**), (**b-11**), (**b-13**), (**b-14**) or (**b-15**) as defined above,
or else a formula (**If**): 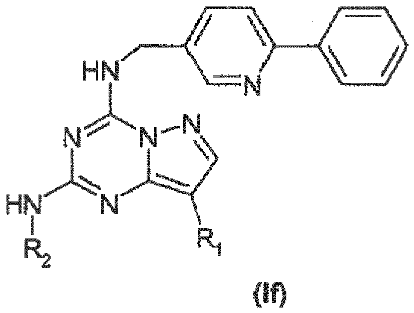
in which:
R₁ is as defined in claim 1 and is advantageously an isopropyl group,
R₂ is a (C₁-C₆)alkyl group, a (C₁-C₆)fluoroalkyl group, a (C₁-C₃) fluoroalkoxy group or a (C₁-C₆)alkoxy(C₁-C₆)alkyl group, said group being substituted with one to three hydroxyl groups, and advantageously is one of the formulae (**b-1**), (**b-2**), (**b-10**), (**b-11**), (**b-13**), (**b-14**) or (**b-15**) as defined above.

10. The compound as claimed in any one of the preceding claims, **characterized in that** it is chosen from one of the following compounds:
- (*R*)-2-(1-hydroxybut-2-ylamino)-8-isopropyl-4-(4-phenylbenzylamino)pyrazolo[1,5-*a*]-1,3,5-triazine (1)
- (*R*)-2-(1-hydroxybut-2-ylamino)-8-isopropyl-4-[4-(pyridin-2-yl)benzylamino]pyrazolo[1,5-*a*]-1,3,5-triazine (**2**)
- Fumarate salt of (*R*)-2-(1-hydroxybut-2-ylamino)-8-isopropyl-4-[4-(pyridin-2-yl)benzylamino]pyrazolo[1,5-*a*]-1,3,5-triazine (3)
- (*S*)-2-(1-hydroxybut-2-ylamino)-8-isopropyl-4-[4-(pyridin-2-yl)benzylamino]pyrazolo[1,5-*a*]-1,3,5-triazine (**4**)
- Fumarate salt of (*S*)-2-(1-hydroxybut-2-ylamino)-8-isopropyl-4-[4-(pyridin-2-yl)benzylamino]pyrazolo[1,5-*a*]-1,3,5-triazine (**5**)
- (*R*)-2-(1-hydroxybut-2-ylamino)-8-isopropyl-4-[4-(thiophen-3-yl)benzylamino]pyrazolo[1,5-*a*]-1,3,5-triazine (**6**)
- (*S*)-2-(1-hydroxybut-2-ylamino)-8-isopropyl-4-[4-(thiophen-3-yl)benzylamino]pyrazolo[1,5-*a*]-1,3,5-triazine (**7**)
- (*S*)-2-(1,2-dihydroxypropan-3-ylamino)-8-isopropyl-4-[4-(pyridin-2-yl)benzylamino]pyrazolo[1,5-*a*]-1,3,5-triazine (**8**)
- Fumarate salt of (*S*)-2-(1,2-dihydroxypropan-3-ylamino)-8-isopropyl-4-[4-(pyridin-2-yl)benzylamino]pyrazolo[1,5-*a*]-1,3,5-triazine (**9**)
- (*R*)-2-(1,2-dihydroxypropan-3-ylamino)-8-isopropyl-4-[4-(pyridin-2-yl)benzylamino]pyrazolo[1,5-*a*]-1,3,5-triazine (**10**)
- Fumarate salt of (*R*)-2-(1,2-dihydroxypropan-3-ylamino)-8-isopropyl-4-[4-(pyridin-2-yl)benzylamino]pyrazolo[1,5-*a*]-1,3,5-triazine (**11**)
- (2*R*,3*R*)-2-(1,3-dihydroxybut-2-ylamino)-8-isopropyl-4-[4-(pyridin-2-yl)benzylamino]pyrazolo[1,5-*a*]-1,3,5-triazine (**12**)
- Fumarate salt of (2*R*,3*R*)-2-(1,3-dihydroxybut-2-ylamino)-8-isopropyl-4-[4-(pyridin-2-yl)benzylamino]pyrazolo[1,5-*a*]-1,3,5-triazine (**13**)
- (*2S*,*3S*)-2-(1,3-dihydroxybut-2-ylamino)-8-isopropyl-4-[4-(pyridin-2-yl)benzylamino]pyrazolo[1,5-*a*]-1,3,5-triazine (**14**)
- Fumarate salt of (*2S*,*3S*)-2-(1,3-dihydroxybut-2-ylamino)-8-isopropyl-4-[4-(pyridin-2-yl)benzylamino]pyrazolo[1,5-*a*]-1,3,5-triazine (**15**)
- (*2R*,*3R*)-2-(1,3-dihydroxybut-2-ylamino)-8-isopropyl-4-[4-(thiophen-3-yl)benzylamino]pyrazolo[1,5-*a*]-1,3,5-triazine (**16**)
- 2-(1,3-dihydroxyprop-2-ylamino)-8-isopropyl-4-[4-(pyridin-2-yl)benzylamino]pyrazolo[1,5-*a*]-1,3,5-triazine (**17**)
- Fumarate salt of 2-(1,3-dihydroxyprop-2-ylamino)-8-isopropyl-4-[4-(pyridin-2-yl)benzylamino]pyrazolo[1,5-*a*]-1,3,5-triazine (**18**)
- 2-(1,3-dihydroxyprop-2-ylamino)-8-isopropyl-4-[4-(pyridin-3-yl)benzylamino]pyrazolo[1,5-*a*]-1,3,5-triazine (**19**)
- (*S*)-2-(1-hydroxybut-2-ylamino)-8-isopropyl-4-[4-(pyridin-3-yl)benzylamino]pyrazolo[1,5-*a*]-1,3,5-triazine (**20**)
- (*2S*,*3S*)-2-(1,3-dihydroxybut-2-ylamino)-8-isopropyl-4-[4-phenyl(pyridin-3-yl)methylamino]pyrazolo[1,5-*a*]-1,3,5-triazine (**21**)
- (*S*)-2-(1-hydroxybut-2-ylamino)-8-isopropyl-4-[4-phenyl(pyridin-3-yl)methylamino]pyrazolo[1,5-*a*]-1,3,5-triazine (**22**)
- (*2R,3R*)-2-(1,3-dihydroxybut-2-ylamino)-8-isopropyl-4-[4-phenyl(pyridin-3-yl)methylamino]pyrazolo[1,5-*a*]-1,3,5-triazine (**23**)
- 2-(1,3-dihydroxyprop-2-ylamino)-8-isopropyl-4-[4-phenyl(pyridin-3-yl)methylamino]pyrazolo[1,5-*a*]-1,3,5-triazine (**24**)
- (*R*)-2-(1-hydroxy-4-methylpent-2-ylamino)-8-isopropyl-4-[4-phenyl(pyridin-3-yl)methylamino]pyrazolo[1,5-*a*]-1,3,5-triazine (**25**)
- (*S*)-2-(1-hydroxy-4-methylpent-2-ylamino)-8-isopropyl-4-[4-phenyl(pyridin-3-yl)methylamino]pyrazolo[1,5-*a*]-1,3,5-triazine (**26**)
- (*S*)-2-(1-hydroxy-3,3-dimethylbut-2-ylamino)-8-isopropyl-4-[4-(pyridin-2-yl)benzylamino]pyrazolo[1,5-*a*]-1,3,5-triazine (**27**)
- (*S*)-2-(1-hydroxy-3-methylbut-2-ylamino)-8-isopropyl-4-[4-(pyridin-2-yl)benzylamino]pyrazolo[1,5-*a*]-1,3,5-triazine (**28**)
- 2-[4-[1-[8-isopropyl-4-[[4-(pyridin-2-yl)phenyl]methylamino]pyrazolo[1,5-*a*]-1,3,5-triazin-2-yl]piperidin-4-yl]piperidin-1-yl]ethanol (**29**)
- 2-[4-[8-isopropyl-4-[[4-(pyridin-2-yl)phenyl]methylamino]pyrazolo[1,5-*a*]-1,3,5-triazin-2-yl]piperazin-1-yl]ethanol (**30**)
- (*R*)-2-(1,2-dihydroxypropan-3-ylamino)-8-ethyl-4-[4-(pyridin-2-yl)benzylamino]pyrazolo[1,5-*a*]-1,3,5-triazine (**31**)
- [(2R)-3-[[8-ethyl-4-[[4-(2-pyridyl)phenyl]methylamino[pyrazolo[1,5-*a*][1,3,5]triazin-2-yl]amino]-2-hydroxypropyl] (2R)-2-Bocamino-3-methylbutanoate (**32**)
- [(2R)-3-[[8-ethyl-4-[[4-(2-pyridyl)phenyl]methylamino]pyrazolo[1,5-a] [1,3,5]triazin-2-yl]amino]-2-hydroxypropyl] (2R)-2-amino-3-methylbutanoate (**33**) in the form of a base or of a salt, in particular dihydrochloride
- (*S*)-2-(1,2-dihydroxypropan-3-ylamino)-8-ethyl-4-[4-(pyridin-2-yl)benzylamino]pyrazolo[1,5-*a*]-1,3,5-triazine (**34**)
- (*S*)-8-ethyl-2-(1-hydroxybut-2-ylamino)-4-[4-(pyridin-2-yl)benzylamino]pyrazolo[1,5-*a*]-1,3,5-triazine (**35**)
- (*R*)-8-ethyl-2-(1-hydroxybut-2-ylamino)-4-[4-(pyridin-2-yl)benzylamino]pyrazolo[1,5-*a*]-1,3,5-triazine (**36**)
- 2-(1,3-dihydroxyprop-2-ylamino)-8-ethyl-4-[4-(pyridin-2-yl)benzylamino]pyrazolo[1,5-*a*]-1,3,5-triazine (**37**)
- 2-[(*2S*)-1-[8-ethyl-4-[[4-(2-pyridyl)phenyl]methylamino]pyrazolo[1,5-*a*][1,3,5]triazin-2-yl]-2-piperidyl]ethanol (**38**)
- 2-[[8-ethyl-4-[[4-(2-pyridyl)phenyl]methylamino]pyrazolo[1,5-a][1,3,5]triazin-2-yl]-(2-hydroxyethyl)amino]ethanol (**39**)
- (*2R*,*3R*)-2-[[8-ethyl-4-[[4-(2-pyridyl)phenyl]methylamino]pyrazolo[1,5-*a*][1,3,5]triazin-2-yl]amino]butane-1,3-diol (**40**)
- (2*S*,3*S*)-2-[[8-ethyl-4-[[4-(2-pyridyl)phenyl]methylamino]pyrazolo[1,5-a] [1,3,5]triazin-2-yl]amino]butane-1,3-diol (**41**)
and also their optional salts of pharmaceutically acceptable acids.

11. A process for preparing a compound of formula (**I**) as claimed in any one of the preceding claims, **characterized in that** a compound of formula (**IIa**) which follows: 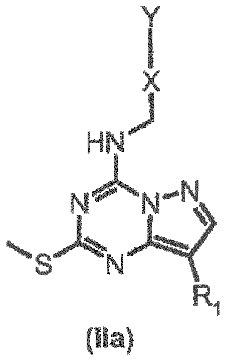 in which X, Y and R₁ are as defined in claim 1, is reacted with meta-chloroperbenzoic acid in an oxidation reaction, and **in that** the sulfone obtained is used directly in a nucleophilic substitution reaction in the presence of a primary amine of formula NH-R₂R₉, where R₂ and R₉ are as defined in claim 1, at a temperature ranging from 100 to 180°C, so as to produce a compound of formula (**I**),
or else **in that** a compound of formula (**IIb**) 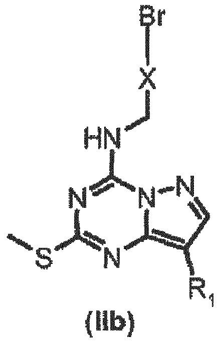 in which X and R₁ are as defined in claim 1, is reacted with a metal, for instance palladium or nickel, so as to give a compound of formula (IIa) as defined above, or alternatively **in that** a compound of formula (**IIb**) as defined above is reacted with meta-chloroperbenzoic acid in an oxidation reaction, the sulfone obtained being directly used in a nucleophilic substitution reaction in the presence of a primary amine of formula NH-R₂R₉, where R₂ and R₉ are as defined in claim 1, so as to give a compound of formula (**III**): 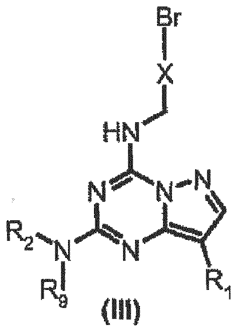 in which X₁, R₁ and R₂ are as defined in claim 1, which is subjected to a coupling reaction catalyzed by a metal, for instance palladium or nickel, so as to produce a compound of formula (I).

12. A medicament, **characterized in that** it comprises a compound of formula (**I**) as claimed in any one of claims 1 to 10.

13. A pharmaceutical composition, **characterized in that** it comprises a compound of formula (I) as claimed in any one of claims 1 to 10, and also at least one pharmaceutically acceptable excipient.

14. The compound of formula (**I**) as claimed in any one of claims 1 to 10, for use thereof as a medicament intended for treating and preventing the following diseases: cancers, chronic neurodegenerative diseases such as Alzheimer's disease or Parkinson's disease, acute neurodegenerative diseases such as cerebral trauma, stroke, epilepsy, pulmonary inflammations, arthritis, viral infections (AIDS, Herpes), pain treatment, diabetes, in particular type 2 diabetes, kidney diseases such as renal polycystosis or glomerulonephritis, leukemias such as chronic lymphoid leukemia, in particular of B-cell type, or parasites such as *Plasmodium* and *Leishmania*
